# EUROPEAN PATENT SPECIFICATION

(11) **EP 2 250 287 B1**
(45) Date of publication and mention of the grant of the patent: **18.09.2013**
(21) Application number: 09712527.2
(22) Date of filing: 19.02.2009
(51) Int. Cl.: C12Q 1/68

(54) **DETECTION AND PROGNOSIS OF LUNG CANCER**
NACHWEIS UND PROGNOSE VON LUNGENKREBS
DÉTECTION ET PRONOSTIC DE CANCER DU POUMON

(30) Priority: 19.02.2008 US 29693 P
(43) Date of publication of application: 17.11.2010
(60) Divisional application: 13177701.3
(73) Proprietor: MDxHealth SA, 4000 Sart-Tilman (Liege) (BE); The Johns Hopkins University, Baltimore MD 21218 (US)
(72) Inventor: VAN CRIEKINGE, Wim, B-2550 Waarloos (BE); STRAUB, Josef, 64342 Seeheim-Jugenheim (DE); TROOSKENS, Geert, B-9000 Gent (BE); BAYLIN, Stephen, Baltimore Maryland 21230 (US); HERMAN, James, Baltimore Maryland 21093 (US); SCHUEBEL, Kornel, Baltimore Maryland 21228 (US); COPE, Leslie, Baltimore Maryland 21205 (US); VAN NESTE, Leander, B-3320 Hoegaarden (BE)
(74) Representative: Tombling, Adrian George
(86) International application number: PCT/US2009/034531
(87) International publication number: WO 2009/105549

(56) References cited:
- WO-A2-2006/113671
- WO-A2-2007/009755
- US-A1- 2005 064 401
- US-A1- 2007 044 141
- US-A1- 2007 044 171
- US-A1- 2007 050 146
- KIM HOJOONG ET AL: "Tumor-specific methylation in bronchial lavage for the early detection of non-small-cell lung cancer.", JOURNAL OF CLINICAL ONCOLOGY : OFFICIAL JOURNAL OF THE AMERICAN SOCIETY OF CLINICAL ONCOLOGY 15 JUN 2004 LNKD- PUBMED:15197197, vol. 22, no. 12, 15 June 2004 (2004-06-15) , pages 2363-2370, XP002641112, ISSN: 0732-183X
- HSU HAN-SHUI ET AL: "Characterization of a multiple epigenetic marker panel for lung cancer detection and risk assessment in plasma", CANCER, vol. 110, no. 9, November 2007 (2007-11), pages 2019-2026, XP002641113, ISSN: 0008-543X
- HSU H-S ET AL: "Multiple genetic and epigenetic biomarkers for lung cancer detection in cytologically negative sputum and a nested case-control study for risk assessment", JOURNAL OF PATHOLOGY, vol. 213, no. 4, December 2007 (2007-12), pages 412-419, XP002641114, ISSN: 0022-3417
- CORTESE R ET AL: "Correlative gene expression and DNA methylation profiling in lung development nominate new biomarkers in lung cancer", INTERNATIONAL JOURNAL OF BIOCHEMISTRY AND CELL BIOLOGY, EXETER, GB, vol. 40, no. 8, 1 January 2008 (2008-01-01), pages 1494-1508, XP022655992, ISSN: 1357-2725, DOI: DOI:10.1016/J.BIOCEL.2007.11.018 [retrieved on 2007-12-27]
- COOPER W N ET AL: "Epigenetic regulation of the ras effector/tumour suppressor RASSF2 in breast and lung cancer", ONCOGENE, vol. 27, no. 12, 24 September 2007 (2007-09-24), pages 1805-1811, XP002641115, ISSN: 0950-9232
- LUXEN SYLVIA ET AL: "Silencing of DUOX NADPH oxidases by promoter hypermethylation in lung cancer", CANCER RESEARCH, vol. 68, no. 4, February 2008 (2008-02), pages 1037-1045, XP002641116, ISSN: 0008-5472
- HONNORAT J ET AL: "ULIP/CRMP PROTEINS ARE RECOGNIZED BY AUTOANTIBODIES IN PARANEOPLASTIC NEUROLOGICAL SYNDROMES", EUROPEAN JOURNAL OF NEUROSCIENCE, OXFORD UNIVERSITY PRESS, GB, vol. 11, no. 11, 1 January 1999 (1999-01-01), pages 4226-4232, XP001075062, ISSN: 0953-816X, DOI: DOI:10.1046/J.1460-9568.1999.00864.X
- KIM ET AL.: 'Tumor-Specific Methylation in Bronchial Lavage for the Early Detection of Non-Small Cell Lung Cancer.' J. CLIN ONCOL vol. 22, no. 12, 15 June 2004, pages 2363 - 2370
- REIN ET AL.: 'Identifying 5-methylcytosine and related modifications in DNA genomes.' NUCL ACIDS RES. vol. 26, no. 10, 15 May 1998, pages 2255 - 2264
- TESSEMA ET AL.: 'Concomitant promoter methylation of multiple genes in lung adenocarcinomas from current, former and never smokers.' CARCINOGENESIS vol. 30, no. 7, 12 May 2009, pages 1132 - 1138
- TESSEMA ET AL.: 'Mining the epigenome for methylated genes in lung cancer.' PROC AM THORAC SOC vol. 5, no. 8, 01 December 2008, pages 806 - 810

## Description

### TECHNICAL FIELD OF THE INVENTION

The present invention relates to the area of cancer diagnostics and therapeutics. In particular, it relates to methods and kits for identifying, diagnosing, prognosing and monitoring lung cancer. These methods include determining the methylation status or the expression levels of particular genes, or a combination thereof. In particular, the lung cancer relates to non-small cell lung cancer.

### BACKGROUND OF THE INVENTION

Lung cancer is the most common cause of cancer-related death and causes over one million deaths worldwide each year (Greenlee et al, 2001). Lung cancer is clinically subdivided into small cell lung cancer (SCLC; comprise about 20% of lung cancers), the most aggressive form of lung cancer, and non-small cell lung cancer (NSCLC, the most common lung cancer accounting for about 80%), consisting of adenocarcinoma, squamous cell carcinoma, large cell carcinoma, and miscellaneous other types such as carcinoids, pleomorphic and mixed carcinomas and a range of neuroendocrine cancers (Travis, 2002).

The first signs of cancer usually come from one or more of the following sources: presentation of symptoms, visual detection or direct palpation, histopathological analysis of a biopsy specimen, remote imaging or the detection of a cancer biomarker in a tissue or bodily fluid specimen. The rather late appearance of symptomatology associated with lung cancer, and the poor accessibility to the lung tissue thwart the timely detection of malignancy, contributing to high mortality rates (Ganti *et al.*, 2006; Greenberg *et al*., 2007). Therefore, remote imaging and the development of cancer biomarkers offers the best hope for early detection of lung cancer.

Cancer biomarkers have been described in literature. One can distinguish between immunological markers and genetic markers. Genetic markers are based on detection of mutation in distinct genes in particular in tumor suppressor genes. More recently, DNA methylation markers are evaluated as potential genetic markers for detection of cancer because they offer certain advantages when compared to mutation markers. One of the most important features is that they occur at the early stages of cancer development and in many cases are tissue- and tumor-type specific (Esteller et al. 2001). A further advantage, methylation profile is preserved in purified isolated DNA and methylation changes appear to precede apparent malignancy in many cases. In addition, methylation markers may serve for predictive purposes as they often reflect the sensitivity to therapy or duration of patient survival.

DNA methylation is a chemical modification of DNA performed by enzymes called methyltransferases, in which a methyl group (m) is added to certain cytosines (C) of DNA. This non-mutational (epigenetic) process (mC) is a critical factor in gene expression regulation. See, J.G. Herman, Seminars in Cancer Biology, 9: 359-67, 1999. By turning genes off that are not needed, DNA methylation is an essential control mechanism for the normal development and functioning of organisms. Alternatively, abnormal DNA methylation is one of the mechanisms underlying the changes observed with aging and development of many cancers.

Although the phenomenon of gene methylation has attracted the attention of cancer researchers for some time, its true role in the progression of human cancers is just now being recognized. In normal cells, methylation occurs predominantly in regions of DNA that have few CG base repeats, while CpG islands, regions of DNA that have long repeats of CG bases, remain non-methylated. Gene promoter regions that control protein expression are often CpG island-rich. Aberrant methylation of these normally non-methylated CpG islands in the promoter region causes transcriptional inactivation or silencing of certain tumor suppressor expression in human cancers.

Genes that are hypermethylated in tumor cells are strongly specific to the tissue of origin of the tumor. Molecular signatures of cancers of all types can be used to improve cancer detection, the assessment of cancer risk and response to therapy. Promoter hypermethylation events provide some of the most promising markers for such purposes.

An early diagnosis is critical for the successful treatment of many types of cancer, including lung cancer. If the exact methylation profiles of lung tumors are available and drugs targeting the specific genes are obtainable, then the treatment of lung cancer could be more focused and rational. Therefore, the detection and mapping of novel methylation markers is an essential step towards improvement of lung cancer prevention, screening and treatment.

There is a continuing need in the art to identify methylation markers that can be used for improved assessment of lung cancer.

### SUMMARY OF THE INVENTION

According to one embodiment of the invention a method is provided for identifying lung cancer or its precursor, or predisposition to lung cancer as defined in the appended claims 1 to 8.

According to another embodiment of the invention a kit is provided as defined in appended claims 9 to 17

An additional aspect of the invention provides an isolated polynucleotide as defined in appended claim 18.

These and other embodiments which will be apparent to those of skill in the art upon reading the specification provide the art with reagents and methods for detecting lung cancer, early lung cancer, or predisposition to lung cancer.

### BRIEF DESCRIPTION OF THE DRAWINGS

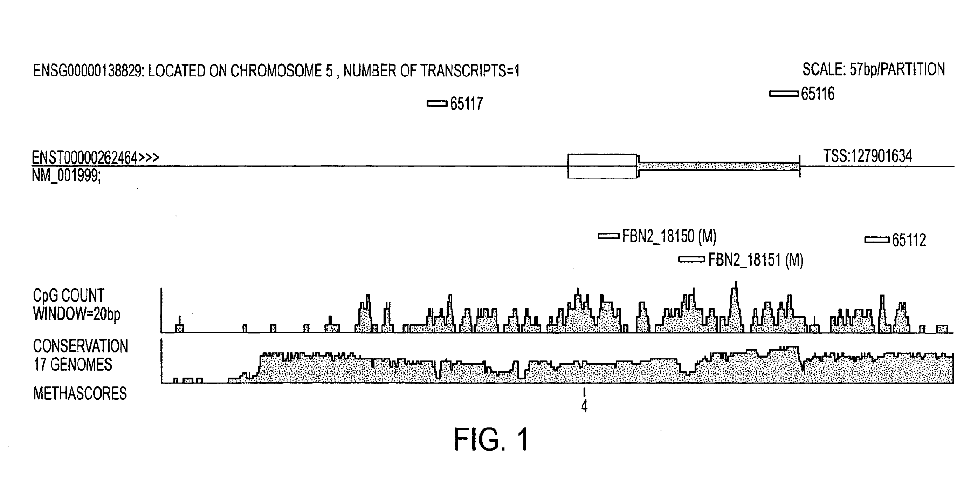

Figure 1: Position of the different primers relative to the TSS (transcription start site). Multiple primer designs are displayed by blue boxes and red boxes (=final primer pairs retained for the assays). The exon of FBN2 is indicated in green. The number of CpG count is spotted in blue over a region of 20 kb.

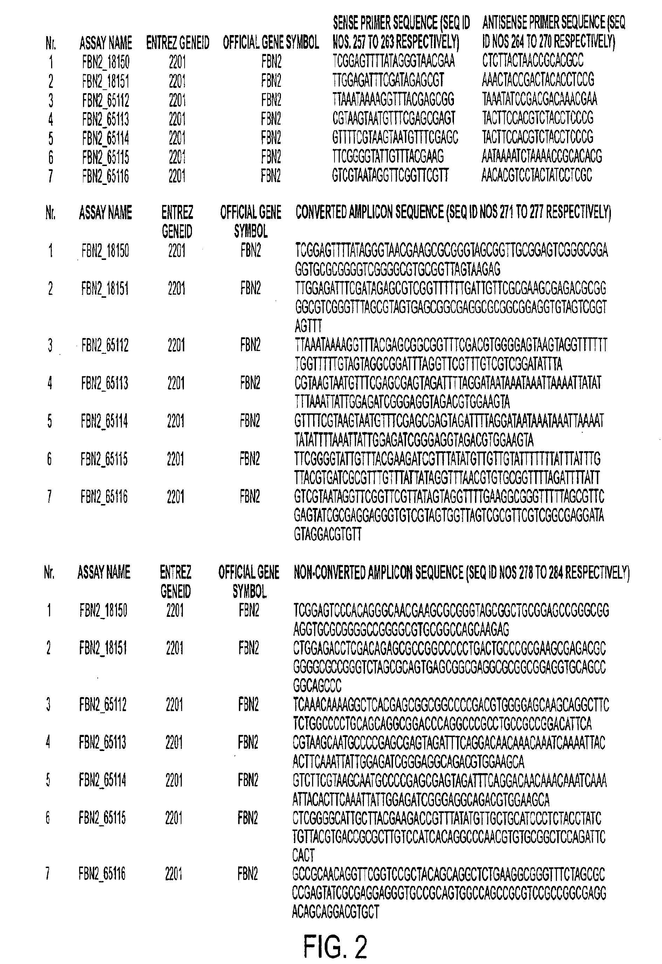

Figure 2 lists the sequences of the different primer sets and converted and unconverted amplicon sequences used in Figure 1.

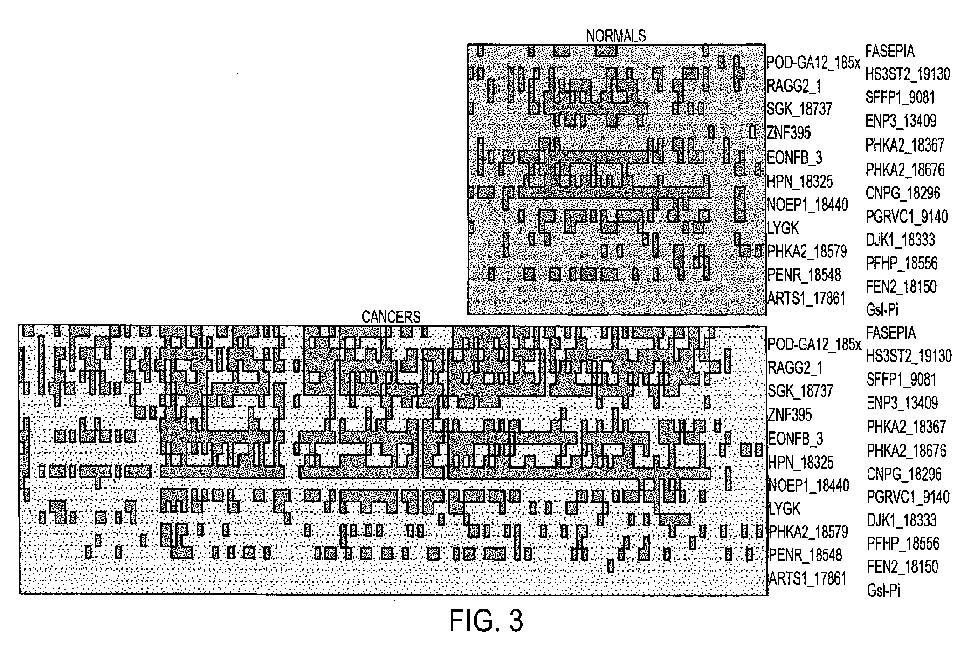

Figure 3: Ranked methylation table obtained with the sample set. 146 methylation profiles from lung cancer samples (right table) are compared against 58 normal tissue samples (left table). Samples are shown along the Y-axis where each horizontal row represents the methylation profile of one individual sample across the 23 different assays (X-axis). Assays demonstrating the best methylation discriminators between the 2 groups are displayed at the left, with discrimination effect decreasing towards the right. The black boxes indicate the methylated results; grey boxes indicate the unmethylated results; white boxes indicate invalid results.

Figure 4: Amplification plot for the standard curve for *JAM3*

Figure 5: Amplification plot for standard curve and samples for *JAM3*

Figure 6: Linear regression of standard curve for *JAM3*

Figure 7: Decision tree for ratio determination

Figure 8: Performance of the individual markers on lung tissue samples using qMSP.

### DETAILED DESCRIPTION OF THE INVENTION

The inventors have found that cytosines within CpG dinucleotides of DNA from particular genes isolated from a test sample are differentially methylated in human lung cancer tissue samples and normal lung tissue control samples. The cancer tissues samples are hypermethylated or hypomethylated with respect to the normal samples (collectively termed epigenetic modification). The differential methylation has been found in genomic DNA of ACSL6, ALS2CL, APC2, ARTS-1, BEX1, BMP7, BNIP3, CBR3, CD248, CD44, CHD5, DLK1, DPYSL4, DSC2, EDNRB, EPB41L3, EPHB6, ERBB3, FBLN2, FBN2, FOXL2, GNAS, GSTP1, HS3ST2, HPN, IGFBP7, IRF7, JAM3; LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, SULF2, TIMP3, TJP2, TRPV2, UCHL1, WDR69, ZFP42, ZNF442, and ZNF655. These genes are all known in the art and fully described by sequence in publicly available databases, e.g., Entrez Gene of the National Center for Biotechnology Information. See Gene ID references provided in Table 1 and Table 3, each of which is incorporated by reference herein.

Epigenetic modification of a gene can be determined by any method known in the art. One method is to determine that a gene which is expressed in normal cells or other control cells is less expressed or not expressed in tumor cells. Conversely, a gene can be more highly expressed in tumor cells than in control cells in the case of hypomethylation. This method does not, on its own, however, indicate that the silencing or activation is epigenetic, as the mechanism of the silencing or activation could be genetic, for example, by somatic mutation. One method to determine that silencing is epigenetic is to treat with a reagent, such as DAC (5'-deazacytidine), or with a reagent which changes the histone acetylation status of cellular DNA or any other treatment affecting epigenetic mechanisms present in cells, and observe that the silencing is reversed, i.e., that the expression of the gene is reactivated or restored. Another means to determine epigenetic modification is to determine the presence of methylated CpG dinucleotide motifs in the silenced gene or the absence of methylation CpG dinucleotide motifs in the activated gene. Typically these methylated motifs reside near the transcription start site, for example, within about 3 kbp, within about 2.5 kbp, within about 2 kbp, within about 1.5 kbp, within about 1 kbp, within about 750 bp, or within about 500 bp. CpG dinucleotides susceptible to methylation are typically concentrated in the promoter region, intron region or exon region of human genes. Thus, the methylation status of the promoter and/or intron and/or exon region of at least one gene can be assessed. Once a gene has been identified as the target of epigenetic modification in tumor cells, determination of reduced or enhanced expression can be used as an indicator of epigenetic modification.

Expression of a gene can be assessed using any means known in the art. Typically expression is assessed and compared in test samples and control samples which may be normal, non-malignant cells. The test samples may contain cancer cells or pre-cancer cells or nucleic acids from them. For example the sample may contain lung adenoma cells, lung advanced adenoma cells, or lung adenocarcinoma cells. Samples may contain squamous cells, and large cell carcinoma. Samples may contain mixtures of different types and stages of lung cancer cells. Either mRNA (nucleic acids) or protein can be measured to detect epigenetic modification. Methods employing hybridization to nucleic acid probes can be employed for measuring specific mRNAs. Such methods include using nucleic acid probe arrays (microarray technology), in situ hybridization, and using Northern blots. Messenger RNA can also be assessed using amplification techniques, such as RT-PCR. In some embodiments oligonucleotide probes are covalently linked to primers for amplification. Advances in genomic technologies now permit the simultaneous analysis of thousands of genes, although many are based on the same concept of specific probe-target hybridization. Sequencing-based methods are an alternative; these methods started with the use of expressed sequence tags (ESTs), and now include methods based on short tags, such as serial analysis of gene expression (SAGE) and massively parallel signature sequencing (MPSS). Differential display techniques provide yet another means of analyzing gene expression; this family of techniques is based on random amplification of cDNA fragments generated by restriction digestion, and bands that differ between two tissues identify cDNAs of interest. Specific proteins can be assessed using any convenient method including immunoassays and immunocytochemistry but are not limited to that. Most such methods will employ antibodies which are specific for the particular protein or protein fragments. The sequences of the mRNA (cDNA) and proteins of the markers of the present invention are known in the art and publicly available.

Methylation-sensitive restriction endonucleases can be used to detect methylated CpG dinucleotide motifs. Such endonucleases may either preferentially cleave methylated recognition sites relative to non-methylated recognition sites or preferentially cleave non-methylated relative to methylated recognition sites. Examples of the former are Acc III, Ban I, BstN I, Msp I, and Xma I. Examples of the latter are Acc II, Ava I, BssH II, BstU I, Hpa II, and Not I. Alternatively, chemical reagents can be used which selectively modify either the methylated or non-methylated form of CpG dinucleotide motifs.

Modified products can be detected directly, or after a further reaction which creates products which are easily distinguishable. Means which detect altered size and/or charge can be used to detect modified products, including but not limited to electrophoresis, chromatography, and mass spectrometry. Examples of such chemical reagents for selective modification include hydrazine and bisulfite ions. Hydrazine-modified DNA can be treated with piperidine to cleave it. Bisulfite ion-treated DNA can be treated with alkali. Other means which are reliant on specific sequences can be used, including but not limited to hybridization, amplification, sequencing, and ligase chain reaction, Combinations of such techniques can be uses as is desired.

The principle behind electrophoresis is the separation of nucleic acids via their size and charge. Many assays exist for detecting methylation and most rely on determining the presence or absence of a specific nucleic acid product. Gel electrophoresis is commonly used in a laboratory for this purpose.

One may use MALDI mass spectrometry in combination with a methylation detection assay to observe the size of a nucleic acid product. The principle behind mass spectrometry is the ionizing of nucleic acids and separating them according to their mass to charge ratio. Similar to electrophoresis, one can use mass spectrometry to detect a specific nucleic acid that was created in an experiment to determine methylation. See (Tost, J. *et al.* 2003).

One form of chromatography, high performance liquid chromatography, is used to separate components of a mixture based on a variety of chemical interactions between a substance being analyzed and a chromatography column. DNA is first treated with sodium bisulfite, which converts an unmethylated cytosine to uracil, while methylated cytosine residues remain unaffected. One may amplify the region containing potential methylation sites via PCR and separate the products via denaturing high performance liquid chromatography (DHPLC). DHPLC has the resolution capabilities to distinguish between methylated (containing cytosine) and unmethylated (containing uracil) DNA sequences. (Deng, *D. et al.* 2002)

Hybridization is a technique for detecting specific nucleic acid sequences that is based on the annealing of two complementary nucleic acid strands to form a double-stranded molecule. One example of the use of hybridization is a microarray assay to determine the methylation status of DNA. After sodium bisulfite treatment of DNA, which converts an unmethylated cytosine to uracil while methylated cytosine residues remain unaffected, oligonucleotides complementary to potential methylation sites can hybridize to the bisulfite-treated DNA. The oligonucleotides are designed to be complimentary to either sequence containing uracil (thymine) or sequence containing cytosine, representing unmethylated and methylated DNA, respectively. Computer-based microarray technology can determine which oligonucleotides hybridize with the DNA sequence and one can deduce the methylation status of the DNA. Similarly primers can be designed to be complimentary to either sequence containing uracil (thymine) or sequence containing cytosine. Primers and probes that recognize the converted methylated form of DNA are dubbed methylation-specific primers or probes (MSP).

An additional method of determining the results after sodium bisulfite treatment involves sequencing the DNA to directly observe any bisulfite-modifications. Pyrosequencing technology is a method of sequencing-by-synthesis in real time. It is based on an indirect bioluminometric assay of the pyrophosphate (PPi) that is released from each deoxynucleotide (dNTP) upon DNA-chain elongation. This method presents a DNA template-primer complex with a dNTP in the presence of an exonuclease-deficient Klenow DNA polymerase. The four nucleotides are sequentially added to the reaction mix in a predetermined order. If the nucleotide is complementary to the template base and thus incorporated, PPi is released. The PPi and other reagents are used as a substrate in a luciferase reaction producing visible light that is detected by either a luminometer or a charge-coupled device. The light produced is proportional to the number of nucleotides added to the DNA primer and results in a peak indicating the number and type of nucleotide present in the form of a pyrogram. Pyrosequencing can exploit the sequence differences that arise following sodium bisulfite-conversion of DNA.

A variety of amplification techniques may be used in a reaction for creating distinguishable products. Some of these techniques employ PCR. Other suitable amplification methods include the ligase chain reaction (LCR) (Barringer *et al*, 1990), transcription amplification (Kwoh et al. 1989; WO88/10315), selective amplification of target polynucleotide sequences (US Patent No. 6,410,276), consensus sequence primed polymerase chain reaction (US Patent No 4,437,975), arbitrarily primed polymerase chain reaction (WO90/06995), nucleic acid based sequence amplification (NASBA) (US Patent Nos. 5,409,818; 5,554,517; 6,063,603), microsatellite length polymorphism (MLP), and nick displacement amplification (W02004/067726).

Sequence variation that reflects the methylation status at CpG dinucleotides in the original genomic DNA offers two approaches to PCR primer design. In the first approach, the primers do not themselves "cover" or hybridize to any potential sites of DNA methylation; sequence variation at sites of differential methylation are located between the two primers. Such primers are used in bisulfite genomic sequencing, COBRA, Ms-SNuPE. In the second approach, the primers are designed to anneal specifically with either the methylated or unmethylated version of the converted sequence. If there is a sufficient region of complementarity, *e.g*., 12, 15, 18, or 20 nucleotides, to the target, then the primer may also contain additional nucleotide residues that do not interfere with hybridization but may be useful for other manipulations. Exemplary of such other residues may be sites for restriction endonuclease cleavage, for ligand binding or for factor binding or linkers or repeats. The oligonucleotide primers may or may not be such that they are specific for modified methylated residues

One way to distinguish between modified and unmodified DNA is to hybridize oligonucleotide primers which specifically bind to one form or the other of the DNA. After hybridization, an amplification reaction can be performed and amplification products assayed. The presence of an amplification product indicates that a sample hybridized to the primer. The specificity of the primer indicates whether the DNA had been modified or not, which in turn indicates whether the DNA had been methylated or not. For example, bisulfite ions modify non-methylated cytosine bases, changing them to uracil bases. Uracil bases hybridize to adenine bases under hybridization conditions. Thus an oligonucleotide primer which comprises adenine bases in place of guanine bases would hybridize to the bisulfite-modified DNA, whereas an oligonucleotide primer containing the guanine bases would hybridize to the non-modified (methylated) cytosine residues in the DNA. Amplification using a DNA polymerase and a second primer yield amplification products which can be readily observed. Such a method is termed MSP (Methylation Specific PCR; Patent Nos 5,786,146; 6,017,704; 6,200,756). The amplification products can be optionally hybridized to specific oligonucleotide probes which may also be specific for certain products. Alternatively, oligonucleotide probes can be used which will hybridize to amplification products from both modified and nonmodified DNA.

In one particular embodiment, primers useful in MSP carried out on the gene selected from ACSL6, ALS2CL, APC2, ARTS-1, BEX1, BMP7, BNIP3, CBR3, CD248, CD44, CHD5, DLK1, DPYSL4, DSC2, EDNRB, EPB41L3, EPHB6, ERBB3, FBLN2, FBN2, FOXL2, GNAS, GSTP1, HS3ST2, HPN, IGFBP7, IRF7, JAM3, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, SULF2, TIMP3, TJP2, TRPV2, UCHL1, WDR69, ZFP42, ZNF442, and ZNF655 are provided. Primers preferably are designed to amplify the genomic sequences in the regions under investigation. Preferred regions may comprise, consist essentially of or consist of the sequences represented by SEQ ID NO. 129-192 and/or SEQ ID NO. 193-256 and/or SEQ ID NO. 315-329 and/or SEQ ID NO. 330-344 and/or SEQ ID NO. 408-428 and/or SEQ ID NO. 429-449 and/or SEQ ID NO. 271-277 and/or SEQ ID NO. 278-284. Preferred sense primers (5' - 3') may comprise, consist essentially of or consist of the sequences represented by SEQ ID NO. 1-64 and/or SEQ ID NO. 285-299 and/or SEQ ID NO. 345-365 and/or SEQ ID NO. 257-263. Preferred antisense primers (5' - 3') comprise, consist essentially of or consist of the sequences represented by SEQ ID NO. 65-128 and/or SEQ ID NO. 300-314 and/or SEQ ID NO. 366-386 and/or SEQ ID NO. 264-270.

Another way to distinguish between modified and nonmodified DNA is to use oligonucleotide probes which may also be specific for certain products. Such probes can be hybridized directly to modified DNA or to amplification products of modified DNA. Oligonucleotide probes can be labeled using any detection system known in the art. These include but are not limited to fluorescent moieties, radioisotope labeled moieties, bioluminescent moieties, luminescent moieties, chemiluminescent moieties, enzymes, substrates, receptors, or ligands.

In one particular embodiment, probes useful in MSP carried out on the gene selected from ACSL6, ALS2CL, APC2, ARTS-1, BEX1, BMP7, BNIP3, CBR3, CD248, CD44, CHD5, DLK1, DPYSL4, DSC2, EDNRB, EPB41L3, EPHB6, ERBB3, FBLN2, FBN2, FOXL2, GNAS, GSTP1, HS3ST2, HPN, IGFBP7, IRF7, JAM3, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, SULF2, TIMP3, TJP2, TRPV2, UCHL1, WDR69, ZFP42, ZNF442, and ZNF655 are provided. Probes preferably are designed to bind to genomic sequences in the regions under investigation. Preferred regions may comprise, consist essentially of or consist of the sequences represented by SEQ ID NO. 129-192 and/or SEQ ID NO. 193-256 and/or SEQ ID NO. 315-329 and/or SEQ ID NO. 330-344 and/or SEQ ID NO. 408-428 and/or SEQ ID NO. 429-449 and/or SEQ ID NO. 271-277 and/or SEQ ID NO. 278-284. Preferred probes (5' - 3') may comprise, consist essentially of or consist of the sequences represented by SEQ ID NO. 387-407.

Still another way for the identification of methylated CpG dinucleotides utilizes the ability of the MBD domain of the McCP2 protein to selectively bind to methylated DNA sequences (Cross et al, 1994; Shiraishi et al, 1999). Restriction enconuclease digested genomic DNA is loaded onto expressed His-tagged methyl-CpG binding domain that is immobilized to a solid matrix and used for preparative column chromatography to isolate highly methylated DNA sequences.

Real time chemistry allows for the detection of PCR amplification during the early phases of the reactions, and makes quantitation of DNA and RNA easier and more precise. A few variations of the real-time PCR are known. They include the TaqMan™ (Roche Molecular Systems) system and Molecular Beacon™ system which have separate probes labeled with a fluorophore and a fuorescence quencher. In the Scorpion™ system the labeled probe in the form of a hairpin structure is linked to the primer. In addition, the Amplifluor™ (Chemicon International) system and the Plexor™ (Promega) system can be used.

DNA methylation analysis has been performed successfully with a number of techniques which include the MALDI-TOFF, MassARRAY, MethyLight, Quantitative analysis of ethylated alleles (QAMA), enzymatic regional methylation assay (ERMA), HeavyMethyl, QBSUPT, MS-SNuPE, MethylQuant, Quantitative PCR sequencing, and Oligonucleotide-based microarray systems.

Subsets of genes for all aspects and embodiments of the invention include ACSL6, ALS2CL, APC2, ARTS-1, BEX1, BMP7, BNIP3, CBR3, CD248, CD44, CHD5, DLK1, DPYSL4, DSC2, EDNRB, EPB41L3, EPHB6, ERBB3, FBLN2, FBN2, FOXL2, GNAS, GSTP1, HS3ST2, HPN, IGFBP7, IRF7, JAM3, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, SULF2, TIMP3, TJP2, TRPV2, UCHL1, WDR69, ZFP42, ZNF442, and ZNF655. By "gene" is meant any gene which is taken from the family to which the named "gene" belongs and includes according to all aspects of the invention not only the particular sequences found in the publicly available database entries, but also encompasses transcript and nucleotide variants of these sequences, with the proviso that methylation or another epigenetic modification of the gene is linked to lung cancer. The number of genes whose modification is tested and/or detected can vary: one, two, three, four, five, or more genes can be tested and/or detected. In some cases at least two genes are selected. In other embodiments at least three genes are selected.

Testing can be performed diagnostically or in conjunction with a therapeutic regimen. Testing can be used to monitor efficacy of a therapeutic regimen, whether a chemotherapeutic agent or a biological agent, such as a polynucleotide. Testing can also be used to determine what therapeutic or preventive regimen to employ on a patient. Moreover, testing can be used to stratify patients into groups for testing agents and determining their efficacy on various groups of patients. The detection may also link to a cancer stage or grade. The "Stage" refers to how far a cancer has progressed anatomically, while the "grade" refers to cell appearance (differentiation) and DNA make up.

Test samples and normal samples for diagnostic, prognostic, or personalized medicine uses can be obtained from surgical samples, such as biopsies or fine needle aspirates, from paraffin embedded lung, or other organ tissues, from a body fluid such as blood, serum, lymph, saliva, sputum, urine, pleural fluid, bronchoalveolar lavage fluid. Such sources are not meant to be exhaustive, but rather exemplary. A test sample obtainable from such specimens or fluids includes detached tumor cells and/or free nucleic acids that are released from dead or damaged tumor cells. Nucleic acids include RNA, genomic DNA, mitochondrial DNA, single or double stranded, and protein-associated nucleic acids. Any nucleic acid specimen in purified or non-purified form obtained from such specimen cell can be utilized as the starting nucleic acid or acids. The test samples may contain cancer cells or pre-cancer cells or nucleic acids from them. For example the sample may contain lung adenoma cells, lung advanced adenoma cells, or lung adenocarcinoma cells. Samples may contain squamous cells or large cell carcinoma. Samples may contain mixtures of different types and stages of lung cancer cells.

The test sample is generally obtained from a (human) subject suspected of being tumorigenic. Alternatively the test sample is obtained from a subject undergoing routine examination and not necessarily being suspected of having a disease. Thus patients at risk can be identified before the disease has a chance to manifest itself in terms of symptoms identifiable in the patient. Alternatively the sample is obtained from a subject undergoing treatment, or from patients being checked for recurrence of disease.

Demethylating agents can be contacted with cells *in vitro* or *in vivo* for the purpose of restoring normal gene expression to the cell. Suitable demethylating agents include, but are not limited to 5-aza-2'-deoxycytidine, 5-aza-cytidine, Zebularine, procaine, and L-ethionine. This reaction may be used for diagnosis, for determining predisposition, and for determining suitable therapeutic regimes.

Although diagnostic and prognostic accuracy and sensitivity may be achieved by using a combination of markers, such as 5 or 6 markers, or 9 or 10 markers, practical considerations may dictate use of smaller combinations. Any combination of markers for a specific cancer may be used which comprises 2, 3, 4, or 5 markers. Combinations of 2, 3, 4, or 5 markers can be readily envisioned given the specific disclosures of individual markers provided herein. Preferably, the invention involves detecting an epigenetic change in a panel of genes comprising a combination of 2, 3, 4 or 5 markers.

Kits according to the present invention are assemblages of reagents for testing methylation. They are typically in a package which contains all elements, optionally including instructions. The package may be divided so that components are not mixed until desired. Components may be in different physical states. For example, some components may be lyophilized and some in aqueous solution. Some may be frozen. Individual components may be separately packaged within the kit. The kit may contain reagents, as described above for differentially modifying methylated and non-methylated cytosine residues. Desirably the kit will contain oligonucleotide primers which specifically hybridize to regions within 3 kb of the transcription start sites of the genes/markers: ACSL6, ALS2CL, APC2, ARTS-1, BEX1, BMP7, BNIP3, CBR3, CD248, CD44, CHD5, DLK1, DPYSL4, DSC2, EDNRB, EPB41L3, EPHB6, ERBB3, FBLN2, FBN2, FOXL2, GNAS, GSTP1, HS3ST2, HPN, IGFBP7, IRF7, JAM3, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, SULF2, TIMP3, TJP2, TRPV2, UCHL1, WDR69, ZFP42, ZNF442, and ZNF655. Additional markers may be used. Typically the kit will contain both a forward and a reverse primer for a single gene or marker. If there is a sufficient region of complementarity, *e.g*., 12, 15, 18, or 20 nucleotides, then the primer may also contain additional nucleotide residues that do not interfere with hybridization but may be useful for other manipulations. Exemplary of such other residues may be sites for restriction endonuclease cleavage, for ligand binding or for factor binding or linkers or repeats. The oligonucleotide primers may or may not be such that they are specific for modified methylated residues. The kit may optionally contain oligonucleotide probes. The probes may be specific for sequences containing modified methylated residues or for sequences containing non-methylated residues. The kit may optionally contain reagents for modifying methylated cytosine residues. The kit may also contain components for performing amplification, such as a DNA polymerase (particularly a thermostable DNA polymerase) and deoxyribonucleotides. Means of detection may also be provided in the kit, including detectable labels on primers or probes. Kits may also contain reagents for detecting gene expression for one of the markers of the present invention (Table 1 and Table 3). Such reagents may include probes, primers, or antibodies, for example. In the case of enzymes or ligands, substrates or binding partners may be sued to assess the presence of the marker. Kits may contain 1, 2, 3, 4, or more of the primers or primer pairs of the invention. Kits that contain probes may have them as separate molecules or covalently linked to a primer for amplifying the region to which the probes hybridize. Other useful tools for performing the methods of the invention or associated testing, therapy, or calibration may also be included in the kits, including buffers, enzymes, gels, plates, detectable labels, vessels, etc.

In one aspect of this embodiment, the gene is contacted with hydrazine, which modifies cytosine residues, but not methylated cytosine residues, then the hydrazine treated gene sequence is contacted with a reagent such as piperidine, which cleaves the nucleic acid molecule at hydrazine modified cytosine residues, thereby generating a product comprising fragments. By separating the fragments according to molecular weight, using, for example, an electrophoretic, chromatographic, or mass spectrographic method, and comparing the separation pattern with that of a similarly treated corresponding non-methylated gene sequence, gaps are apparent at positions in the test gene contained methylated cytosine residues. As such, the presence of gaps is indicative of methylation of a cytosine residue in the CpG dinucleotide in the target gene of the test cell.

Bisulfite ions, for example, sodium bisulfite, convert non-methylated cytosine residues to bisulfite modified cytosine residues. The bisulfite ion treated gene sequence can be exposed to alkaline conditions, which convert bisulfite modified cytosine residues to uracil residues. Sodium bisulfite reacts readily with the 5,6-double bond of cytosine (but poorly with methylated cytosine) to form a sulfonated cytosine reaction intermediate that is susceptible to deamination, giving rise to a sulfonated uracil. The sulfonate group can be removed by exposure to alkaline conditions, resulting in the formation of uracil. The DNA can be amplified, for example, by PCR, and sequenced to determine whether CpG sites are methylated in the DNA of the sample. Uracil is recognized as a thymine by Taq polymerase and, upon PCR, the resultant product contains cytosine only at the position where 5-methylcytosine was present in the starting template DNA. One can compare the amount or distribution of uracil residues in the bisulfite ion treated gene sequence of the test cell with a similarly treated corresponding non-methylated gene sequence. A decrease in the amount or distribution of uracil residues in the gene from the test cell indicates methylation of cytosine residues in CpG dinucleotides in the gene of the test cell. The amount or distribution of uracil residues also can be detected by contacting the bisulfite ion treated target gene sequence, following exposure to alkaline conditions, with an oligonucleotide that selectively hybridizes to a nucleotide sequence of the target gene that either contains uracil residues or that lacks uracil residues, but not both, and detecting selective hybridization (or the absence thereof) of the oligonucleotide.

Test compounds can be tested for their potential to treat cancer. Expression of a gene selected from those listed in Table 1 and Table 3 is determined and if it is increased or decreased by the compound in the cell or if methylation of the gene is decreased or increased by the compound in the cell, one can identify it as having potential as a treatment for cancer. The candidate compound will have the effect of reversing the expression/or methylation modification found in the cancer cell.

The above disclosure generally describes the present invention. All references disclosed herein are expressly incorporated by reference. A more complete understanding can be obtained by reference to the following specific examples which are provided herein for purposes of illustration only, and are not intended to limit the scope of the invention.

### EXAMPLES

### EXAMPLE 1: Selection of candidate genes

Using re-expression profiles of lung cancer cell lines, candidate genes were identified and the most promising markers were tested on tissue using the Base5 methylation profiling platform (Straub et al. 2007). Differential methylation of the particular genes was assessed using Base5 methylation profiling platform as follows: DNA was extracted from lung samples, bisulfite converted, and selected regions of the particular genes were amplified using primers whose sequence represented converted or non-converted DNA sequences. Amplification was monitored in real-time set up using cybergreen. Two robust data analyses designed to cope with inherent variance (*i*.*e*., noise) in measured Ct and Tm values were applied to withhold 64 different assays for detecting differential methylation of ACSL6, ALS2CL, APC2, BEX1, BMP7, CBR3, CD248, CD44, CHD5, DLK1, DPYSL4, DSC2, EPB41L3, EPHB6, ERBB3, FBLN2, FBN2, FOXL2, GSTP1, HS3ST2, IGFBP7, IRF7, JAM3, LOX, LY6D, LY6K, MACF1, MCAM, NEFH, NID2, PCDHB15, PHACTR3, POMC, PRKCA, PSEN1, RBP1, RRAD, SFRP1, SOD3, SOX17, SULF2, TIMP3, TJP2, TRPV2, UCHL1, WDR69, ZFP42, ZNF442, and ZNF655 in lung cancer tissue samples.

### Materials and methods

### Strategy to identify supplementary gene targets for lung cancer

Promoter sequences were linked with gene expression to identify epigenetically silenced genes. An established pharmacologic unmasking strategy (5-aza-2'-deoxycytidine [DAC] and trichostatin A [TSA]) for re-expression analysis of epigenetically targeted genes was combined with proprietary advanced bioinformatics tools to identify genes prone to promoter methylation. To identify differentially methylated markers associated with non-small cell lung cancer (NSCLC), the information derived from 11 cell lines (ATCC, at domain lgcpromochem-atcc.com/, 2006) was used:
1. NCI-H23: adenocarcinoma, cell line derived from a smoker
2. NCI-H1568: adenocarcinoma, cell line derived from a smoker
3. NCI-H1993: adenocarcinoma, cell line derived from a smoker
4. NCI-H2023: adenocarcinoma, cell line derived from a non-smoker
5. NCI-H2085: adenocarcinoma, cell line derived from a non-smoker
6. NCI-H2228: adenocarcinoma, cell line derived from a non-smoker
7. NCI-H520: squamous cell carcinoma, cell line
8. NCI-H838: adenocarcinoma, cell line derived from a smoker
9. NCI-H2170: squamous cell carcinoma, cell line derived from a non-smoker
10. NCI-H1869: squamous cell carcinoma, cell line derived from a smoker
11. SK-MES-1: squamous cell carcinoma, cell line

Cell culture, microarray and data analysis was done as described in Schuebel *et al*, 2007. In short, the cell lines were cultured with 5-aza-2'-deoxycytidine (AZA) and with trichostatin A (TSA) in parallel. Control cells underwent mock treatment. Total RNA was harvested from AZA-, TSA- and mock-treated cells. Amplification and labeling of the RNA were carried out using the Low RNA Input Linear Amplification kit (Agilent Technologies). The complementary labeled RNA was hybridized and processed according the Agilent microarray protocol. All calculations and normalizations of the expression data were performed using the R statistical computing platform (Ihaka *et al*., 1996) and packages from Bioconductor bioinformatics software (Gentleman *et al*., 1996).

A gene was selected as a good candidate if it met the following criteria:
1. Re-expressed under AZA treatment; a gene was termed as a top tier gene if the expression is up regulated by more than two-fold in the AZA treated versus mock sample on the Agilent whole human genome expression microarray platform; if it showed an enrichment between 1.4 and 2 fold it was termed as a next tier gene
2. Silent, *i.e*., having no basal expression, in the mock cells
3. No response to TSA treatment alone

Following this initial candidate selection, 2 main strategies were taken to further select good gene candidates susceptible to hypomethylation and/or hypermethylation: a computational strategy and a verification strategy based on cell lines and primary tumors.

### Computational Strategy

This strategy was applied on the top and next tier genes of the first 6 cell lines (NCI-H23, NCI-H1568, NCI-H1993, NCI-H2023, NCI-H2085, and NCI-H2228).

Different steps were taken towards identification of good candidate genes susceptible to hypomethylation and/or hypermethylation:

Step 1: The promoters of all the selected and clearly annotated top tier genes were separately mapped on the genome-wide alignment of all promoter associated CpG islands. The genes were selected if they were located less than 9 ancestral nodes from an established list of 56 markers (see BROAD analysis). Using this approach, 100 genes were identified.

BROAD analysis: Genome-wide Promoter Alignment

The "Database of Transcription Start Sites" (DBTSS) (Suzuki *et al*., 2004) mapped each transcript sequence on the human draft genome sequence to identify its transcriptional start site, providing more detailed information on distribution patterns of transcriptional start sites and adjacent regulatory regions. From ~14,500 well-characterized human genes present in the Affymetrix GeneChip Human Genome U133A Arrays 8793 sequences were extracted from the DBTSS [5, 6] (DBTSS, version 3.0 based on human assembly build 31). The remaining genes (14,500-8793=5707) on the Affymetrix array contained no reported transcriptional start site (TSS) according to DBTSS. All the promoter sequences were subsequently aligned by clustalw algorithm (Li 2003; Thompson *et al*., 1994) Treeillustrator (Trooskens *et al*., 2005) was used to visualize the large guide tree in addition to indicating the location of the known markers. Some regions on the "circle" are denser in known markers than others, indicating that there might be a sequence mechanism located in the small region around the TSS which makes certain genes more methylation-prone.

Step 2: As shown by Schuebel *et al.* and based on the sequencing project from Sjöblom *et al.* (Sjöblom *et al*., 2006), promoter CpG island methylation and subsequent gene silencing of genes known to be mutated in cancer is more frequent than the mutations themselves. Therefore the genes identified by Sjöblom *et al.* were used to identify possible extra targets from the top or next tiers with a known genetic background in either colon or breast cancer. Taking into account all 6 cell lines, 22 extra genes were found to adhere to this category.

Step 3: A final batch of genes was selected based on their appearance in multiple top tiers of the colorectal cell lines from *Schuebel et al.* and at least one top tier of the lung cancer cell lines. The same approach was used based on multiple breast cancer cell lines, i.e. MDA-MB-231, MDA-MB-468, MCF7 and T-47D. The next tiers of the breast cancer cell lines were also used, since the overlap between multiple top tiers of these breast cancer cell lines and the top tiers of the lung cancer cell lines was minimal compared to the overlap with the colon cancer cell lines. Sixteen genes were selected out of the colon screen and another 17 out of the breast screen.

After removing the duplicates of genes obtained by these different approaches, a list of in total 144 genes was identified by this strategy.

### Verification Strategy

This strategy was applied on a selection of the top and next tier genes of 4 adenocarcinoma cell lines (NCI-H23, NCI-H1568, NCI-H1993, and NCI-H838) and 4 squamous cell carcinoma cell lines (NCI-H520, NCI-H2170, NCI-H1869, and SK-MES-1). These genes were verified in cell lines and/or primary tumors and normal lung samples for expression by reverse transcription-PCR and promoter methylation by MSP. Using this strategy, a list of in total 63 was identified.

Duplicates, imprinted genes and genes for which primer design was not possible were excluded from both lists. This final selection of genes was further analyzed on the Base5 methylation profiling platform (Straub et al. 2007).

### Sample specimen

A total of 132 samples (64 lung cancer samples, the majority derived from lung adenocarcinoma and sqaumous cell carcinomas; and 68 corresponding normal tissues) were used to find markers which distinguish cancer from non-cancer tissue based on methylation status.

### DNA extraction and Bisulfite modification

A high throughput, real-time methylation specific detection platform was applied on two groups of samples totaling 132 genomic DNA samples. The two groups of samples consisted of 64 samples isolated from lung cancer tissue and 68 samples isolated from corresponding normal lung tissue.

From each sample, up to 1 µg of genomic DNA was converted using a bisulfite based protocol (EZ DNA Methylation Kit™, ZYMO Research, Orange, CA.).

### Detection of hypermethylation

After conversion and purification the equivalent of 25-75ng of the starting material was applied per sub-array of an OpenArray™ plate on a real-time qPCR system (BioTrove Inc.) using the DNA double strand-specific dye SYBRgreen for signal detection.

The cycling conditions were: 90°C-10 seconds, (43°C 18 seconds, 49°C 60 seconds, 77°C 22 seconds, 72°C 70seconds, 95°C 28 seconds) for 40 cycles, 70°C for 200 seconds, 45°C for 5 seconds. A melting curve was generated in a temperature range between 45°C and 94°C. Methylation specific PCR (MSP) primers were designed for each of the genes assessed for hypermethylation.

### Analysis of methylation

For each combination of assays and samples two parameters were collected using an algorithm which is part of the standard data analysis package offered by the supplier. The parameters were the Ct value (threshold cycle number) of the assessed amplicon and the melting temperature of the assessed amplicon.

The following data analysis workflow was applied to the results created by the software which came with the system OpenArray™ system. Data was collected for each combination of assays and samples in the two sets of samples used. Results were filtered using the following approach. Read outs from not loaded reaction spaces were removed from analysis. Technical Control assays were removed from the data set. Assays known to not work for other than biological reasons were removed from the analysis. Samples for which Ct calls for the gene beta-Actin were not present were removed from the analysis. Ct values > 0 for each gene were normalized using the Ct values collected for the gene beta-Actin. This resulted in two files containing the results for each set of sample.

Two robust data analyses designed to cope with inherent variance (*i*.*e*., noise) in measured Ct and Tm values were applied which have common features and data analysis steps. Based on the original data, a p-value was assigned to each marker that corresponds to the probability of obtaining Ct/Tm values at least as favorable assuming these values were the result of chance alone. Next, robustness of the above p-value was computed by introducing increasing levels of noise in the data and recomputing the p-value (pVal) as above. The noise level on the x-axis was plotted against (1- pVal) on the y-axis, and the area under the resulting curve was used as the final score for a particular marker. With robust markers, the initial p-value survives for a while, hence (1-pVal) will stay high for a while, hence the area under the curve (AUC) will tend to be high. With not-so-robust markers an initial (1-pVal) will drop quickly with increasing noise levels on the x-axis, which will result in a lower AUC.

The two analysis methods, called "Ranks" and "Squares," differ only in the way the p-values for each noise level are applied.

### The "Ranks" method

For computing p-values with the Ranks method for a particular marker, four lists of ranks of samples are generated: two based on the Ct values determined for each assay applied to all samples (cancer samples as well as non cancer samples) resulting in one ascending list of ranks and on one descending list of ranks; and two based on the Tm values determined for each assay applied to all samples (cancer samples as well as non-cancer samples) resulting in one ascending list of ranks and on one descending list of ranks.

For each of these four lists of ranks, the sum of the ranks of the cancer samples are calculated. The lowest of these four sums is kept. Depending on this lowest sum, we label the marker as a positive/negative Ct/Tm marker. For instance, if the lowest sum is found with the descending Ct ranking, we label the marker as a negative Ct ranker; alternatively, in case the lowest sum is found with the descending Tm ranking, the marker is labeled as a positive Tm ranker.

Next, the rank sum of the cancers is recorded for 10,000 random rankings. The fraction of cases where this sum is at least as low as the rank sum of the cancers in the original ranking is taken to be the p-value.

In order to asses the correlation between added noise and resulting p-values, random noise is introduced into the list Ct values and Tm values and the ranking procedure is repeated. This process resulted in a series of p-values with increasing levels of noise which was used to determine an AUC score. Assays are ranked based on their AUC from high to low.

### The "Squares" method

Applying this method, a lower and/or upper limit is imposed on the Ct and/or Tm values determined for all samples. Such limits correspond to a "square" imposed on the scatter plot of samples where Ct forms the x-axis and Tm forms the y-axis. When considering all possible squares in this scatter plot, we are in fact exploring all combinations of a lower and/or upper limit in the Ct dimension on the one hand and the Tm dimension on the other hand. The sensitivity and specificity for the detection of cancers is determined for the set of all possible squares as defined above.

Next, for each square, the p-value is computed using the Fisher exact test. The square resulting in the highest sensitivity and specificity for determining methylation in cancer and normal samples can thus be determined for each marker candidate.

To test quality of the best square, an increasing amount of noise is injected as described above, and the p-value is recomputed using the Fisher exact test. When plotting the correlation between injected noise and the resulting p-values, the AUC can be determined. The most optimal square will result in the highest AUC. Assays are ranked based on the maximal AUC achievable.

The results of the applied analysis methods are "zipped" together in the following way. The results of applying the two analysis methods described above to two different sample sets are included into four different lists called "sample_set_1_ranks", "sample_set_2_ranks", "sample_set_1_squares", and "sample_set_2_squares"

A new "zipped" list is created by taking the highest scoring assay from the list "sample_set_1_ranks," followed by a comparison of the highest scoring assay from list "sample_set_2_ranks." If the marker is already present in the zipped list, this finding is noted and the next highly scoring marker of the list "sample_set_2_ranks" is used. This selection procedure is applied comparing the highest scoring assay of lists "sample_set_1_squares," noting down if the assay already has scored in the zipped list up to this step. The "sample_set_2_squares" list is used as the source for the next markers in the zipped list. The sequence of lists is maintained until all the assays in all the lists have been assessed.

The cut-offs 0.832, 0.909, 0.687 and 0.743 were applied on the "AUC" determined for each assay and rank in the lists sample_set_1_ranks, sample_set_2_ranks, sample_set_1_squares, and sample_set_2_squares. This resulted in 10 different genes.

### Results

A high throughput, real-time methylation specific detection platform was applied on two groups of samples isolated from lung cancer tissue and from corresponding normal lung tissue. In this study it was shown that a number of genes are differentially methylated in lung cancer, in particular in non-small cell lung cancer, more particularly in lung adenocarcinoma or squamous cell carcinoma. We identified 64 different assays for detecting 49 different genes being differentially methylated in human lung cancer tissue and normal lung tissue control samples. The genes identified are ACSL6, ALS2CL, APC2, BEX1, BMP7, CBR3, CD248, CD44, CHD5, DLK1, DPYSL4, DSC2, EPB41L3, EPHB6, ERBB3, FBLN2, FBN2, FOXL2, GSTP1, HS3ST2, IGFBP7, IRF7, JAM3, LOX, LY6D, LY6K, MACF1, MCAM, NEFH, NID2, PCDHB15, PHACTR3, POMC, PRKCA, PSEN1, RBP1, RRAD, SFRP1, SOD3, SOX17, SULF2, TIMP3, TJP2, TRPV2, UCHL1, WDR69, ZFP42, ZNF442, ZNF655.

The resulting assays have the assay details provided in Table 1.

**Table 1. Methylation Specific PCR (MSP) primers used for the 64 assays:**

| **#** | **Assay Name** | **Entrez GeneID** | **Official Gene symbol** | **Sense primer sequence (SEQ ID NO:1-64, respectively)** | **Antisense primer sequence (SEQ ID NO:65-128, respectively)** |
|---|---|---|---|---|---|
| 1 | ACSL6_17822 | 23305 | ACSL6 | TTTAATGTTACGTTTTGGCGTT | GAACCAACCCTCTCCGACC |
| 2 | ACSL6_17824 | 23305 | ACSL6 | GCGGTTGTAAGGTTTTTGGTC | ATTTTTCCGCAACCTCTCG |
| 3 | ALS2CL_bay | 259173 | ALS2CL | GGACGGGTGTTTGCGTTTTAC | CGAAACCAAAAAACTAAACGAAAACCG |
| 4 | APC2 | 10297 | APC2 | GTCGTTTGTTTAGGTTCGGATC | GACCCGAAATAACCTCGAAACG |
| 5 | BEX1_12842 | 55859 | BEX1 | TCGGGGTTTTTATTTGGTTC | AATCGTCACTCGTATCTCGCT |
| 6 | BMP7_17905 | 655 | BMP7 | GTACGTGCGTTTATTGCGAG | CGTTATCCAAACTAAAATCGACC |
| 7 | CBR3_17931 | 874 | CBR3 | GGTATCGGTTTGGTTATCGC | CGCCTACAACTACTACACGACC |
| 8 | CBR3_17935 | 874 | CBR3 | GTTTTCGATTGATTTATTAAGGTTC | TCAAAATCCGAACTCTAAACCG |
| 9 | CD248_17939 | 57124 | CD248 | TCGTGGGAAGAGAGCGTAG | TTACTAACCTAAACGACCGCAA |
| 10 | CD248_17946 | 57124 | CD248 | TTTTGTTAAGAGTTGTCGTTAGTTC | AATATAAACCCTACGACCGCC |
| 11 | CD248_17947 | 57124 | CD248 | GGGGTAGTCGTTAATTGCGT | TCTTCCCCGAAAACCGCTA |
| 12 | CD44_17961 | 960 | CD44 | CGGGAGAAGAAAGTTAGTGCGT | AAATCGAAAAACCTAAAATATCGC |
| 13 | CHD5_bay | 26038 | CHD5 | GAGCGTTCGGGTTTTGC | CGACCTCGACGAAAAAATAACG |
| 14 | CRBP_1 | 5947 | RBP1 | TTGGGAATTTAGTTGTCGTCGTTTC | AAACAACGACTACCGATACTACGCG |
| 15 | DLK1_18031 | 8788 | DLK1 | GAGGTTTGCGGTTTAGGTTC | CTCACACTATACAACACGCGAC |
| 16 | DLK1_18033 | 8788 | DLK1 | GGAGTTGGGGTTTACGAGAC | ATAATAAATTCCCCGACGACC |
| 17 | DPYSL4_18047 | 10570 | DPYSL4 | GGTGTTTTGATAGAAGTCGTTAGTC | AAAACCATTAACGCCCACG |
| 18 | DPYSL4_18050 | 10570 | DPYSL4 | GGGGTTATAGTTTGGCGTTC | GCTCTAAAAACCACACCCGTC |
| 19 | DSC2_18056 | 1824 | DSC2 | GGTTTCGGTTTCGTTTTGTTC | CTCTACGACTCAAACCTCGCT |
| 20 | EPB41L3_19071 | 23136 | EPB41L3 | GGGATAGTGGGGTTGACGC | ATAAAAATCCCGACGAACGA |
| 21 | EPB41L3_19072 | 23136 | EPB41L3 | GCGTGGGTTTTCGTCGTAG | CCCAAAACTACTCGCCGCT |
| 22 | EPHB6_bay | 2051 | EPHB6 | GGGTGTTCGATTTAAGTCGAGTTC | CGCGAATCTTAACCGAAAAAATCG |
| 23 | ERBB3_18097 | 2065 | ERBB3 | GTTTAGTTAAGTTCGGTTCGGG | GATTACAATTTACAACCTCCGCT |
| 24 | ERBB3_18099 | 2065 | ERBB3 | AGGGAGTTTAGTTAAGTTCGGTTC | TACAACCTCCGCTACCGTC |
| 25 | FBLN2_13328 | 2199 | FBLN2 | TAGAGCGGAGGAAGTTGCG | CAAATACGAACACAAAAACCGA |
| 26 | FBN2_18150 | 2201 | FBN2 | TCGGAGTTTTATAGGGTAACGAA | CTCTTACTAACCGCACGCC |
| 27 | FBN2_18151 | 2201 | FBN2 | TTGGAGATTTCGATAGAGCGT | AAACTACCGACTACACCTCCG |
| 28 | FOX-L2 | 668 | FOXL2 | GCGATAGGTTTTTAGTAAGTAAGCGC | CTCTCCGCTCCAAACGCTAACGCG |
| 29 | Gst-Pi | 2950 | GSTP1 | TTCGGGGTGTAGCGGTCGTC | GCCCCAATACTAAATCACGACG |
| 30 | HS3ST2_19130 | 9956 | HS3ST2 | ACGTAAGAGTTTGGGAGCGT | GACTCCTCGAAAAACAAACGA |
| 31 | HS3ST2_19131 | 9956 | HS3ST2 | GTTTCGGGGTTCGTTTTTC | CGACTCGCTCTATCTCGCAC |
| 32 | IGFBP7_19196 | 3490 | IGFBP7 | TTTGTCGGCGTCGTTATTTTC | AAACTACCTACTAAACGAAACCCG |
| 33 | IGFBP7_19200 | 3490 | IGFBP7 | CGTTTATGGGTCGGTTACGTC | ATAAAAACACGAAAACCCCGC |
| 34 | IRF7_18346 | 3665 | IRF7 | AGTTGAGAATCGGACGGGG | AACGAATCAAACTCCCGAAA |
| 35 | JAM3 | 83700 | JAM3 | GGGATTATAAGTCGCGTCGC | CGAACGCAAAACCGAAATCG |
| 36 | LOX_18967 | 4015 | LOX | GCGCGTAGAGTTGTAAAGGTTC | ACGTCCTCCTCGAACGAAA |
| 37 | LOX_18977 | 4015 | LOX | GGTAGAGGCGAGGAGTTGTTC | TACACAAACCGTTCTAACCCGA |
| 38 | LY6D_18402 | 8581 | LY6D | GATGTCGTTTGGGAGTAGTGC | ACAAAATACCGCTAACTAACGAA |
| 39 | LY6K | 54742 | LY6K | GCGGGGTTTTTTTTATCGGTTAGATTC | CAACGATACCCAAAAAAAATCAACGCG |
| 40 | MACF1_bay | 23499 | MACF1 | GTTTTCGTTGTCGTTACGGGTTC | GCGCAACGAACAAAACG |
| 41 | MCAM | 4162 | MCAM | AGAATTTAGGTCGGTTTTTATCG | ACGCAAAATTCTTCTCCCAAAA |
| 42 | NEFH_18452 | 4744 | NEFH | GTCGGATGAAGTATTCGGG | CCCTACAAACGACGACGAAC |
| 43 | NID2_9093 | 22795 | NID2 | TTATTTCGTTTTTAGGGAGTTTTC | CTTACGAACCATTTAATCCCG |
| 44 | NID2_9094 | 22795 | NID2 | TTTCGTGTGGGAAGAGTTCGT | CGAATAACCGAACGACCGATA |
| 45 | PCDHB15_10763 | 56121 | PCDHB15 | TTTTGGTTATTAGGTAGTTCGGTTC | CACTCTTCGTACTATTCCCGCT |
| 46 | PHACTR3_11692 | 116154 | PHACTR3 | TTATTTTGCGAGCGGTTTC | GAATACTCTAATTCCACGCGACT |
| 47 | POMC | 5443 | POMC | GATTTGGGCGTTTTTGGTTTTTCGC | GACTTCTCATACCGCAATCG |
| 48 | PRKCA_18626 | 5578 | PRKCA | GGGCGTTGAGGTAGAAGAAC | CGACACCTACCAAATAAAATCG |
| 49 | PSEN1_18648 | 5663 | PSEN1 | TTAGGTCGGAGGTTTCGTTT | AAACCCTCACCGTTATCGTC |
| 50 | RRAD_18698 | 6236 | RRAD | GATGTTTCGGTCGAGGTTTC | AAACGACTACAAATAAATACGCCA |
| 51 | SFRP1 | 6422 | SFRP1 | TGTAGTTTTCGGAGTTAGTGTCGCGC | CCTACGATCGAAAACGACGCGAACG |
| 52 | SFRP1_9381 | 6422 | SFRP1 | TTTTGTTCGTCGTATTTTCGG | ATAACGACCCTCGACCTACGAT |
| 53 | SOD3_18740 | 6649 | SOD3 | AGTATAGAGTGGGGAGCGTAGC | CTTTCCTACCACCGAAACGA |
| 54 | SOX17 | 64321 | SOX17 | TTGCGTTAGTCGTTTGCGTTC | CAAAAACGAATCCCGTATCCGACG |
| 55 | SULF2_bay | 55959 | SULF2 | GTTAGTCGAGTTCGGAGGTATC | CAACTCCGAACGAAACAATAAACG |
| 56 | TIMP3 | 7078 | TIMP3 | GCGTCGGAGGTTAAGGTTGTT | CTCTCCAAAATTACCGTACGCG |
| 57 | TJP2_18792 | 9414 | TJP2 | CGGGTTAGAGTATTGTTCGGT | GAACACAAATCCCGCGTAA |
| 58 | TJP2_18797 | 9414 | TJP2 | GATTTTATCGGGGAAATATCG | AAACAAATCCCGCTCCGAA |
| 59 | TRPV2_18803 | 51393 | TRPV2 | TTATTTCGTAGGTTGAGGTTAGGGC | TCCTCTACTATCAACGCCGAC |
| 60 | UCHL1 | 7345 | UCHL1 | GTTGTATTTTCGCGGAGCGTTC | CTCACAATACGTCTAACCGACG |
| 61 | WDR69_18844 | 164781 | WDR69 | GTTTAGGTTGTGGTTTAGGTCGTC | ACACCTCGTATCCTCACTAAAAACG |
| 62 | ZFP42_bay | 132625 | ZFP42 | GGGGTTTTTAGGTATTCGGTTCGTAC | AATACGCAATACCCGACGACCG |
| 63 | ZNF442_bay | 79973 | ZNF442 | TCGGTTTTTAGTTTTTTCGGTCGC | CAATTACTACGCAAAAACGAAACAAAACG |
| 64 | ZNF655 | 79027 | ZNF655 | TTATCGAGAAGCGTCGGTTTC | ACCGAAAAAAAAAACGAACCTAACCG |

**Table 2: Amplicon details**

| Amplicon details (converted sequence): | | | | |
|---|---|---|---|---|
| | **Assay Name** | **Entrez GeneID** | **Official Gene symbol** | **Amplicon Sequence (converted) (SEQ ID NO:129-192, respectively)** |
| 1 | ACSL6_1782 2 | 23305 | ACSL6 | |
| 2 | ACSL6_1782 4 | 23305 | ACSL6 | |
| 3 | ALS2CL_bay | 259173 | ALS2CL | |
| 4 | APC2 | 10297 | APC2 | |
| 5 | BEX1_12842 | 55859 | BEX1 | |
| 6 | BMP7_1790 5 | 655 | BMP7 | |
| 7 | CBR3_1793 1 | 874 | CBR3 | |
| 8 | CBR3_1793 5 | 874 | CBR3 | |
| 9 | CD248_1793 9 | 57124 | CD248 | |
| 10 | CD248 1794 6 | 57124 | CD248 | |
| 11 | CD246_1794 7 | 57124 | CD248 | |
| 12 | CD44_17961 | 960 | CD44 | |
| 13 | CHD5_bay | 26038 | CHD5 | |
| 14 | CRBP_1 | 5947 | RBP1 | |
| 15 | DLK1_18031 | 8788 | DLK1 | |
| 16 | DLK1_18033 | 8788 | DLK1 | |
| 17 | DPYSL4_18 047 | 10570 | DPYSL4 | |
| 18 | DPYSL4_18 050 | 10570 | DPYSL4 | |
| 19 | DSC2_1805 6 | 1824 | DSC2 | |
| 20 | EP841L3_19 071 | 23136 | EPB41L3 | |
| 21 | EPB41L3_19 072 | 23136 | EPB41L3 | |
| 22 | EPHB6_bay | 2051 | EPHB6 | |
| 23 | ERBB3_180 97 | 2065 | ERBB3 | |
| 24 | ERBB3_180 99 | 2065 | ERBB3 | |
| 25 | FBLN2_1332 8 | 2199 | FBLN2 | |
| 26 | FBN2_18150 | 2201 | FBN2 | |
| 27 | FBN2_18151 | 2201 | FBN2 | |
| 28 | FOX-L2 | 668 | FOXL2 | |
| 29 | Gst-Pi | 2950 | GSTP1 | |
| 30 | HS3ST2_19 130 | 9956 | HS3ST2 | |
| 31 | HS3ST2_19 131 | 9956 | HS3ST2 | |
| 32 | IGFBP7_191 96 | 3490 | IGFBP7 | |
| 33 | IGFBP7_192 00 | 3490 | IGFBP7 | |
| 34 | IRF7_18346 | 3665 | IRF7 | |
| 35 | JAM3 | 83700 | JAM3 | |
| 36 | LOX_18967 | 4015 | LOX | |
| 37 | LOX_18977 | 4015 | LOX | |
| 38 | LY6D_18402 | 8581 | LY6D | |
| 39 | LY6K | 54742 | LY6K | |
| 40 | MACF1_bay | 23499 | MACF1 | |
| 41 | MCAM | 4162 | MCAM | |
| 42 | NEFH_1845 2 | 4744 | NEFH | |
| 43 | NID2_9093 | 22795 | NID2 | |
| 44 | NID2_9094 | 22795 | NID2 | |
| 45 | PCDHB15_1 0763 | 56121 | PCDHB15 | |
| 46 | PHACTR3_1 1692 | 116154 | PHACTR3 | |
| 47 | POMC | 5443 | POMC | |
| 48 | PRKCA_186 26 | 5578 | PRKCA | |
| 49 | PSEN1_186 48 | 5663 | PSEN1 | |
| 50 | RRAD_1869 8 | 6236 | RRAD | |
| 51 | SFRP1 | 6422 | SFRP1 | |
| 52 | SFRP1_938 1 | 6422 | SFRP1 | |
| 53 | SOD3_1874 0 | 6649 | SOD3 | |
| 54 | SOX17 | 64321 | SOX17 | |
| 55 | SULF2_bay | 55959 | SULF2 | |
| 56 | TIMP3 | 7078 | TIMP3 | |
| 57 | TJP22_18792 | 9414 | TJP2 | |
| 58 | TJP2_18797 | 9414 | TJP2 | |
| 59 | TRPV2_188 03 | 51393 | TRPV2 | |
| 60 | UCHL1 | 7345 | UCHL1 | |
| 61 | WDR69_188 44 | 164781 | WDR69 | |
| 62 | ZFP42_bay | 132625 | ZFP42 | |
| 63 | ZNF442_bay | 79973 | ZNF442 | |
| 64 | ZNF655 | 79027 | ZNF655 | |

**Amplicon details (non-converted sequence):**

| | **Assay Name** | **Entrez GeneID** | **Official Gene symbol** | **amplicon sequence (not converted) (SEQ ID NO:193-256, respectively)** |
|---|---|---|---|---|
| 1 | ACSL6_17 822 | 23305 | ACSL6 | |
| 2 | ACSL6_17 824 | 23305 | ACSL6 | |
| 3 | ALS2CL_b ay | 259173 | ALS2CL | |
| 4 | APC2 | 10297 | APC2 | |
| 5 | BEX1_128 42 | 55859 | BEX1 | |
| 6 | BMP7_179 05 | 655 | BMP7 | |
| 7 | CBR3_179 31 | 874 | CBR3 | |
| 8 | CBR3_179 35 | 874 | CBR3 | |
| 9 | CD248_17 939 | 57124 | CD248 | |
| 10 | CD248_17 946 | 57124 | CD248 | |
| 11 | CD248_17 947 | 57124 | CD248 | |
| 12 | CD44_179 61 | 960 | CD44 | |
| 13 | CHD5_bay | 26038 | CHD5 | |
| 14 | CRBP_1 | 5947 | RBP1 | |
| 15 | DLK1_180 31 | 8788 | DLK1 | |
| 16 | DLK1_180 33 | 8788 | DLK1 | |
| 17 | DPYSL4_1 8047 | 10570 | DPYSL4 | |
| 18 | DPYSL4_1 8050 | 10570 | DPYSL4 | |
| 19 | DSC2_180 56 | 1824 | DSC2 | |
| 20 | EPB41L3_ 19071 | 23136 | EPB41L3 | |
| 21 | EPB41L3_ 19072 | 23136 | EPB41L3 | |
| 22 | EPHB6_ba y | 2051 | EPHB6 | |
| 23 | ERBB3_18 097 | 2065 | ERBB3 | |
| 24 | ERBB3_18 099 | 2065 | ERBB3 | |
| 25 | FBLN2_13 328 | 2199 | FBLN2 | |
| 26 | FBN2_181 50 | 2201 | FBN2 | |
| 27 | FBN2_181 51 | 2201 | FBN2 | |
| 28 | FOX-L2 | 668 | FOXL2 | |
| 29 | Gst-Pi | 2950 | GSTP1 | |
| 30 | HS3ST2_1 9130 | 9956 | HS3ST2 | |
| 31 | HS3ST2_1 9131 | 9956 | HS3ST2 | |
| 32 | IGFBP7_1 9196 | 3490 | IGFBP7 | |
| 33 | IGFBP7_1 9200 | 3490 | IGFBP7 | |
| 34 | IRF7_1834 6 | 3665 | IRF7 | |
| 35 | JAM3 | 83700 | JAM3 | |
| 36 | LOX_1696 7 | 4015 | LOX | |
| 37 | LOX_1897 7 | 4015 | LOX | |
| 38 | LY6D_184 02 | 8581 | LY6D | |
| 39 | LY6K | 54742 | LY6K | |
| 40 | MACF1_b ay | 23499 | MACF1 | |
| 41 | MCAM | 4162 | MCAM | |
| 42 | NEFH_184 52 | 4744 | NEFH | |
| 43 | NID2_909 3 | 22795 | NID2 | |
| 44 | NID2_909 4 | 22795 | NID2 | |
| 45 | PCDHB15 _10763 | 56121 | PCDHB15 | |
| 46 | PHACTR3 _11692 | 116154 | PHACTR3 | |
| 47 | POMC | 5443 | POMC | |
| 48 | PRKCA_1 8626 | 5578 | PRKCA | |
| 49 | PSEN1_18 648 | 5663 | PSEN1 | |
| 50 | RRAD_18 698 | 6236 | RRAD | |
| 51 | SFRP1 | 6422 | SFRP1 | |
| 52 | SFRP1_93 81 | 6422 | SFRP1 | |
| 53 | SOD3_187 40 | 6649 | SOD3 | |
| 54 | SOX17 | 64321 | SOX17 | |
| 55 | SULF2_ba y | 55959 | SULF2 | |
| 56 | TIMP3 | 7078 | TIMP3 | |
| 57 | TJP2_187 92 | 9414 | TJP2 | |
| 58 | TJP2_187 97 | 9414 | TJP2 | |
| 59 | TRPV2_18 803 | 51393 | TRPV2 | |
| 60 | UCHL1 | 7345 | UCHL1 | |
| 61 | WDR69_1 8844 | 164781 | WDR69 | |
| 62 | ZFP42_ba y | 132625 | ZFP42 | |
| 63 | ZNF442_b ay | 79973 | ZNF442 | |
| 64 | ZNF655 | 79027 | ZNF655 | |

### EXAMPLE 2: Final selection of assays for Base 5

Finally a total number of 80 different assays (62 different genes), comprising:
- 64 assays designed for detecting the methylation status of 49 cancer markers identified by the aforementioned strategy,
- assays for known published markers, and
- good performing assays for cancer markers from other in-house cancer projects, were retained for analysis.

Differential methylation was assessed using the Base 5 platform; genes were ranked based on the best selectivity (sensitivity and specificity) between human lung cancer tissue and normal lung tissue samples. The investigated genes were ACSL6, ALS2CL, APC2, ARTS-1, BEX1, BMP7, BNIP3, CBR3, CD248, CD44, CHD5, DLK1, DPYSL4, DSC2, EDNRB, EPB41L3, EPHB6, ERBB3, FBLN2, FBN2, FOXL2, GNAS, GSTP1, HS3ST2, HPN, IGFBP7, IRF7, JAM3, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, SULF2, TIMP3, TJP2, TRPV2, UCHL1, WDR69, ZFP42, ZNF442, ZNF655.

Primer and amplicon sequences for the 49 genes are summarized in Table 1 and 2. Primer and amplicon sequences for the remainder 13 genes are listed in Table 3 and Table 4.

**Table 3: MSP Primer sequences**

| **Gene ID** | **Symbol** | **Assay** | **Sense primer sequence (5'- 3') (SEQ ID NO: 285-299, respectively)** | **Antisense primer sequence (5' - 3') (SEQ ID NO: 300-314, respectively)** |
|---|---|---|---|---|
| 51752 | ARTS-1 | ARTS-1_17861 | GTAGTGGCGAGATGACGGA | AACCGAAACCAAACAAACG |
| 664 | BNIP3 | BNIP3_13409 | AGTGTTTAGAGAGTTCGTCGGTT | CGTAACGAATAAACTACGCGAT |
| 1910 | EDNRB | EDNRB_3 | GTCGGGTGTTATATGGTGCGT | AAAAACAATCCTCGTCCGAAA |
| 2778 | GNAS | GNAS_18295 | TTTTGAGAGGTCGTTATCGTGT | TTACTCGAACTATTCCCCGATT |
| 3249 | HPN | HPN_18326 | CGTTAGGTAGGGAGGAGGC | |
| 4686 | NCBP1 | NCBP1_18440 | ATTTGGGTAGAAAAGTTCGTTC | CTCAATAATTTTCCCGACGAC |
| 26025 | PCDHGA12 | PCDHGA12_18516 | AACGATTTGGGGTTAGAGTTTC | TAACCAAACTACCGCTTTACGA |
| 5214 | PFKP | PFKP_18555 | TTTTCGTTATGGACGCGGA | ATAACCTTACCGACCCCGAA |
| 10857 | PGRMC1 | PGRMC1_9140 | | CCTATAACTAAACGCGACGCAC |
| 5256 | PHKA2 | PHKA2_18567 | CGTTTTTGGTTTTGTTTTCGT | AACCTAATTCCCGCCCGTT |
| 5256 | PHKA2 | PHKA2_18576 | TTTAGTAGGTTTGGTCGAGGC | ACGCTAACCCCAAAATCCG |
| 5256 | PHKA2 | PHKA2_18579 | TATAGGTAAGGGGGCGGTTTC | GCGACTCTAAAAATTCCGCT |
| 11186 | RASSF1A | RASSF1A | GCGTTGAAGTCGGGGTTC | CCCGTACTTCGCTAACTTTAAACG |
| 9770 | RASSF2 | RASSF2_1 | TTAGAGGGGCGTAGGGTGC | GCCAAACTAAAATCCCAACGA |
| 6446 | SGK | SGK_18737 | CGTTGTAGGATTTTGGGGGTC | ACCCTTCTCCCGCTCGATA |

**Table 4: MSP amplicon sequences**

| **Assay** | **Amplicon Sequence (converted) (5'- 3') (SEQ ID NO: 315-329 respectivety)** | **Amplicon Sequence (not converted) (5'- 3') (SEQ ID NO: 330-344, respectively)** |
|---|---|---|
| ARTS-1_17861 | | |
| BNIP3_13409 | | |
| EDNRB_3 | | |
| GNAS_18295 | | |
| HPN_18326 | | |
| NCBP1_18440 | | |
| PCDHGA12_18 516 | | |
| PFKP_18555 | | |
| PGRMC1_9140 | | |
| PHKA2_18567 | | |
| PHKA2_18576 | | |
| PHKA2_18579 | | |
| RASSF1A | | |
| RASSF2_1 | | |
| SGK_18737 | | |

### EXAMPLE 3: Lightcycler

Twenty three assays issuing from the Base 5 analysis were selected and transferred to the Lightcycler platform in order to confirm the Base 5 results using 3 independent sample sets (JHU, Baltimore, USA; UMCG, Groningen, The Netherlands and Ulg, Liège, Belgium) and to define the best lung cancer methylation markers (Table 5). A beta-actin (ACTB) assay was included as an internal control. The assays were applied on a 384 well plate. The samples were randomized per plate. On this platform Ct values (cycle number at which the amplification curves cross the threshold value, set automatically by the software) and melting curves (Tm) were generated on the Roche LightCycler 480 using SYBR green as detector and for verification of the melting temperature. The size of the amplicon and intensity of the signal detected were analyzed using the Caliper LabChip electrophoretic separation system. Well-defined cut offs were set up on Ct, Tm, amplicon size and signal to get similar methylation calls when using the final Molecular Beacon (MB) detection system for further verification of the markers. DNA methylation calls were compared between 146 lung cancer and 58 normal tissue samples. DNA was isolated using proteinase K digestion and phenol/chloroform extraction method. DNA concentration was measured using NanoDrop Spectrophotometer. From each sample, up to 3 µg of genomic DNA was converted using a bisulphite based protocol (EZ DNA Methylation Kit™, ZYMO Research). After conversion and purification the equivalent of 20 ng of gDNA was used per reaction. An assay ranking was generated and the results are summarized in a methylation table (Figure 3).

A sample was considered methylated if Ct is under 40 and if Tm and amplicon size are within the boundaries of Tm +/- 2 degrees and amplicon size +/- 10 bp. The intensity of the band detected by capillary electrophoresis had to be higher than 20. Those cut offs were set up to get similar methylation calls after Lightcycler analysis and real time PCR with Beacon detection system.

DNA methylation calls were compared between lung cancer tissue and normal lung tissue. An assay ranking with the set of samples was generated and the results are summarized in a methylation table (Figure 3). A one-tailed Fisher's exact test was used as a scoring function to rank the candidate markers. The calculation of Fisher's exact test was based on a formula as described by Haseeb Ahmad Khan in "A visual basic software for computing Fisher's exact probability" (Journal of Statistical Software, vol. 08, issue i21, 2003).

A general overview of the ranking is given in Table 6.

**Table 5: The 23 selected assays which were applied on the Lightcycler platform**

| **N°** | **Assays** | **N°** | **Assays** |
|---|---|---|---|
| **1** | ARTS-1_17861 | **13** | PFKP 18555 |
| **2** | BNIP3_13409 | **14** | PGRMC1_9140 |
| **3** | DLK1_18033 | **15** | PHKA2_18567 |
| **4** | EDNRB_3 | **16** | PHKA2_18576 |
| **5** | FBN2_18150 | **17** | PHKA2_18579 |
| **6** | GNAS_18295 | **18** | PSEN1_18648 |
| **7** | GSTP1 | **19** | RASSF1A |
| **8** | HPN 18326 | **20** | RASSF2 I |
| **9** | HS3ST2 19130 | **21** | SFRP1_9381 |
| **10** | LY6K | **22** | SGK 18737 |
| **11** | NCBP1_18440 | **23** | ZNF655 |
| **12** | PCDHGA12_18516 | | |

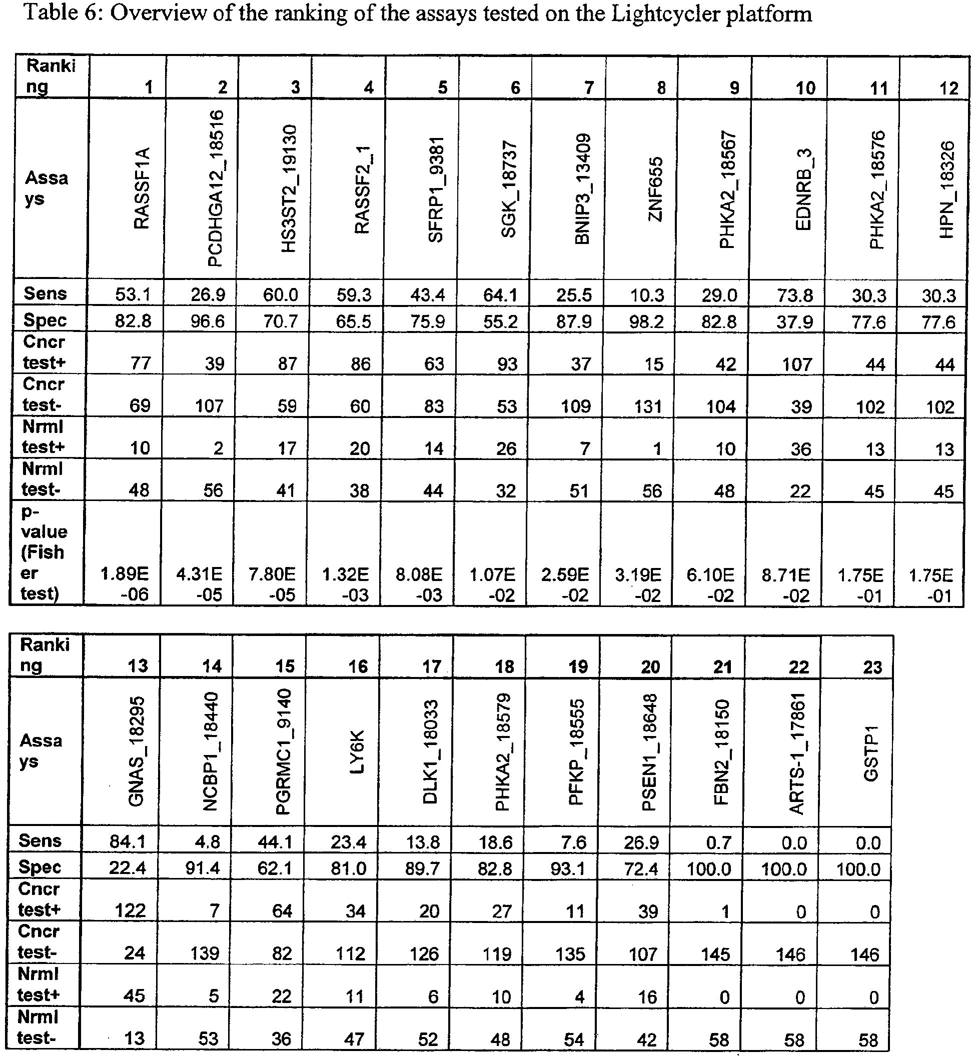

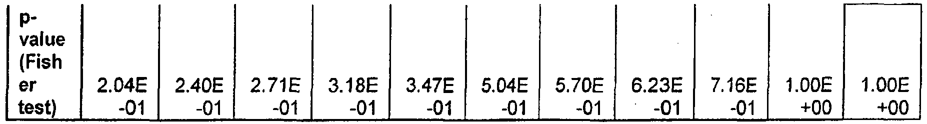

A comparison between the results coming from the Base 5 and the Lightcycler platforms has been performed.

Most of the interesting assays discovered on the Base 5 platform were confirmed on the Lightcycler platform.

### EXAMPLE 4: QMSP

Nineteen genes (APC2, BMP7, BNIP3, DLK1, DPYSL4, GSTP1, HS3ST2, JAM3, LOX, LY6K, NID2, PCDHGA12, PGRMC1, PHKA2, RASSF1A, RASSF2, SFRP1, SOX17, SULF2), were further selected based on the ranking on the Base 5 and/or Lightcycler platforms (marker discovery). For these assays, qMSPs using molecular beacons as detection system were designed (3 designs are evaluated per assay) and tested on control samples (cell lines). Several parameters (background, dynamic of the curve, highest range in fluorescence between beginning of the amplification and plateau phase, etc) were checked. In this phase of assay development, PCR material was used for generating the standard curves (instead of plasmids).

These assays were further verified on lung tissue samples collected by Ulg (Liège, Belgium), VUmc (Amsterdam, The Netherlands), UMCG (Groningen, The Netherlands) and Durham VA Medical Center (Durham, NC, USA) (normal PE tissue samples #60, cancer PE tissue samples #86 (adenocarcinoma #30, squamous cell carcinoma #15, large cell carcinoma #6, carcinoid #1, neuroendocrine #1, NSCLC #33)). DNA was isolated from the lung tissue samples using a phenol-chloroform procedure, quantified using the picogreen method and 1 µg of DNA was bisulphite treated using the ZYMO kit.

The primers and molecular beacons used for the different qMSPs are summarized in Table 7. The amplicons are summarized in Table 8. qMSPs were carried out in a total volume of 12 µl in 384 well plates in an ABI PRISM 7900HT instrument (Applied Biosystems). The final reaction mixture consisted of in-house qMSP buffer (including 80.4 nmol of MgCl2), 60 nmol of each dNTPs, 0.5 U of Jump Start Taq polymerase (SIGMA), 72ng of forward primer, 216 ng of reverse primer, 1.92 pmol of molecular beacon, 6.0 pmol of ROX (passive reference dye) and 50 ng of bisulphite converted genomic DNA. Thermal cycling was initiated with an incubation step of 5 minutes at 95°C, followed by 45 cycles (95°C for 30 seconds, 57°C for 30 seconds, 72°C for 30 seconds). The last step was performed at 72°C for 5 minutes. These conditions were similar for all the test genes as well as for *ACTB.*

Ct values were determined using the SDS software (version 2.2.2) supplied by Applied Biosystems with automatic baseline settings and threshold. The slopes and R² values for the different standard curves were determined after exporting data into excel.

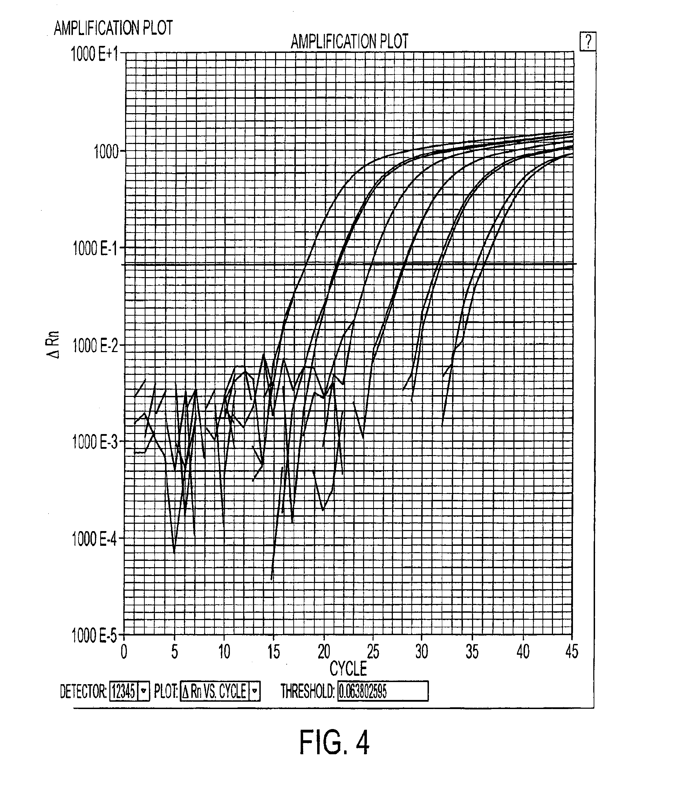

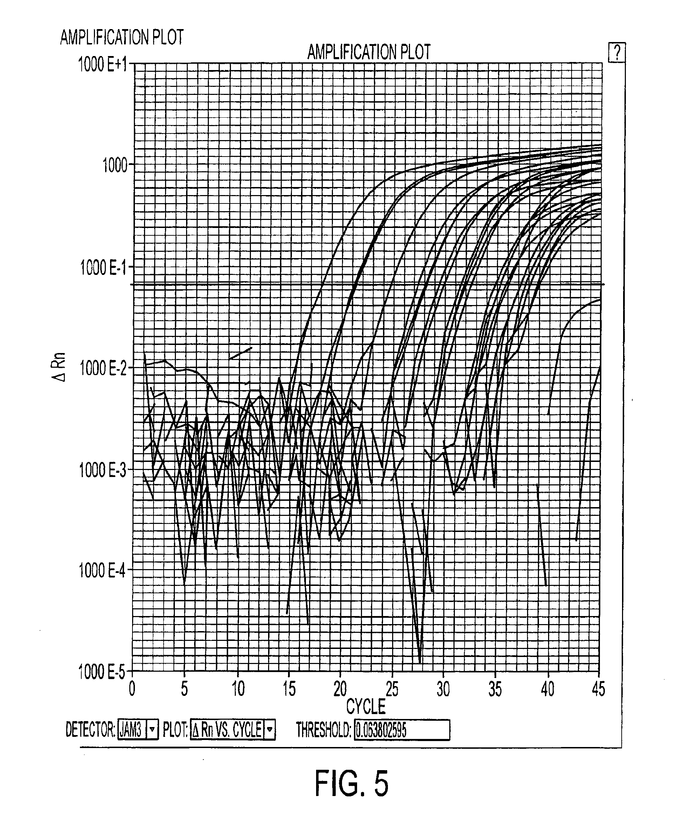

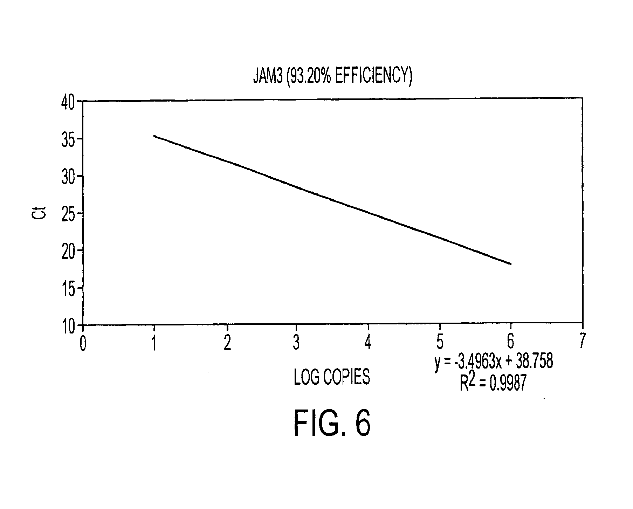

As an example, Figure 4 shows the amplification plot for *JAM3* obtained for the standard curve (960000 copies to 9.6 copies of the gene) and Figure 5 shows the amplification plot for *JAM3* obtained for the standard curve and for some samples. The Ct values plotted against the Log Copies of *JAM3* (Figure 6) give a R² of 0.9987 and the efficiency of the reaction is 93.20%.

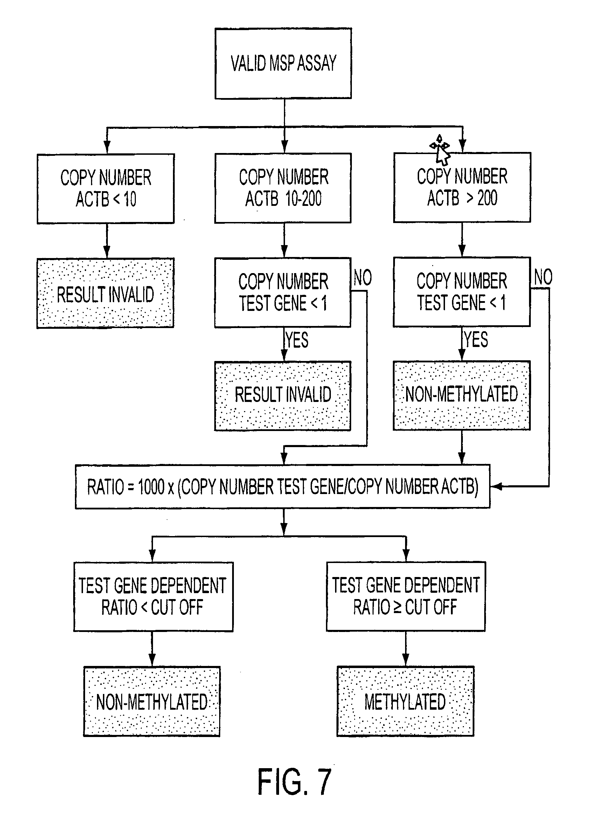

In addition to the test genes, the independent reference gene ACTB was also measured. The ratios between the test genes and ACTB were calculated to generate the test result. The samples were classified as methylated, unmethylated, or invalid based on the decision tree shown in Figure 7.

**Table 7: qMSP primers and molecular beacons sequences**

| **Gene ID** | **Symbol** | **Assay** | **Sense primer sequence (5' - 3') (SEQ ID NO:345-365, respectively)** | **Antisense primer sequence (5' - 3') (SEQ ID NO: 366-386, respectively)** | **Molecular Beacon (5' - 3') (modification beacons : 5' FAM, 3' DABCYL) (SEQ ID NO: 387-407, respectively)** |
|---|---|---|---|---|---|
| 10297 | *APC2* | APC2 | | | |
| 655 | *BMP7* | BMP7_17911 | | | |
| 664 | *BNIP3* | BNIP3 | | | |
| 8788 | *DLK1* | DLK1_68536 | | | |
| 10570 | *DPYSL4* | DPYSL4_18050 | | | |
| 2950 | *GSTP1* | GSTPi current | | | |
| 9956 | *HS3ST2* | HS3ST2_2 | | | |
| 9956 | *HS3ST2* | HS3ST2_8 | | | |
| 9956 | *HS3ST2* | HS3ST2_nor | | | |
| 83700 | *JAM3* | JAM3 | | | |
| 4015 | *LOX* | LOX_25068 | | | |
| 54742 | *LY6K* | LY6K | | | |
| 22795 | *NID2* | NID2_9091 | | | |
| 26025 | *PCDHGA12* | PCDHGA12_1851 6 | | | |
| 10857 | *PGRMC1* | PGRMC1_9140 | | | |
| 5256 | *PHKA2* | PHKA2_70210 | | | |
| 11186 | *RASSF1A* | RASSF1A | | | |
| 9770 | *RASSF2* | RASSF2_2b | | | |
| 6422 | *SFRP1* | SFRP1 | | | |
| 64321 | *SOXI* | SOX17_66072 | | | |
| 55959 | *SULF2* | SULF2_Bay | | | |

**Table 8: qMSP amplicon sequences**

| **Assay** | **Amplicon Sequence (converted) (5'-3') (SEQ ID NO: 408-428, respectively)** | **Amplicon Sequence (non converted) (5'-3') (SEQ ID NO: 429-449, respectively)** |
|---|---|---|
| APC2 | | |
| BMP7_17911 | | |
| BNIP3 | | |
| DLK1_68536 | | |
| DPYSL4_18050 | | |
| GSTPi current | | |
| HS3ST2_2 | | |
| HS3ST2_8 | | |
| HS3ST2_nor | | |
| JAM3 | | |
| LOX_25068 | | |
| LY6K | | |
| NID2_9091 | | |
| PCDHGA12_185 16 | | |
| PGRMC1_9140 | | |
| PHKA2_70210 | | |
| RASSF1A | | |
| RASSF2_2b | | |
| SFRP1 | | |
| SOX17_66072 | | |
| SULF2_Bay | | |

The highest methylation value of the normal tissue specimens was taken as a directive to define a cut off above which the cases were considered to be methylated. The analytical cut off was finally set to give the highest possible specificity and/or above 3 times STDEV (Normal) (excluding outliers).

The one-tailed Fisher's exact test as described above was used as a scoring function to rank the candidate markers (Journal of Statistical Software, vol. 08, issue i21, 2003).

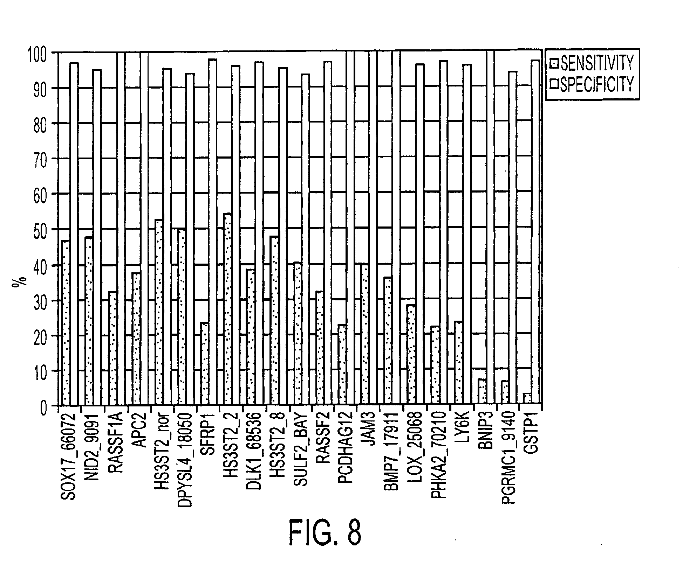

Table 9 summarizes the results obtained for *JAM3.* Table 10 summarizes the results obtained for all the tested markers on tissue samples. The individual performances of the assays are shown in Figure 8 and the assays are ranked according their p-value (Fisher's exact test). The best performing markers were further tested on clinical samples (sputum samples).

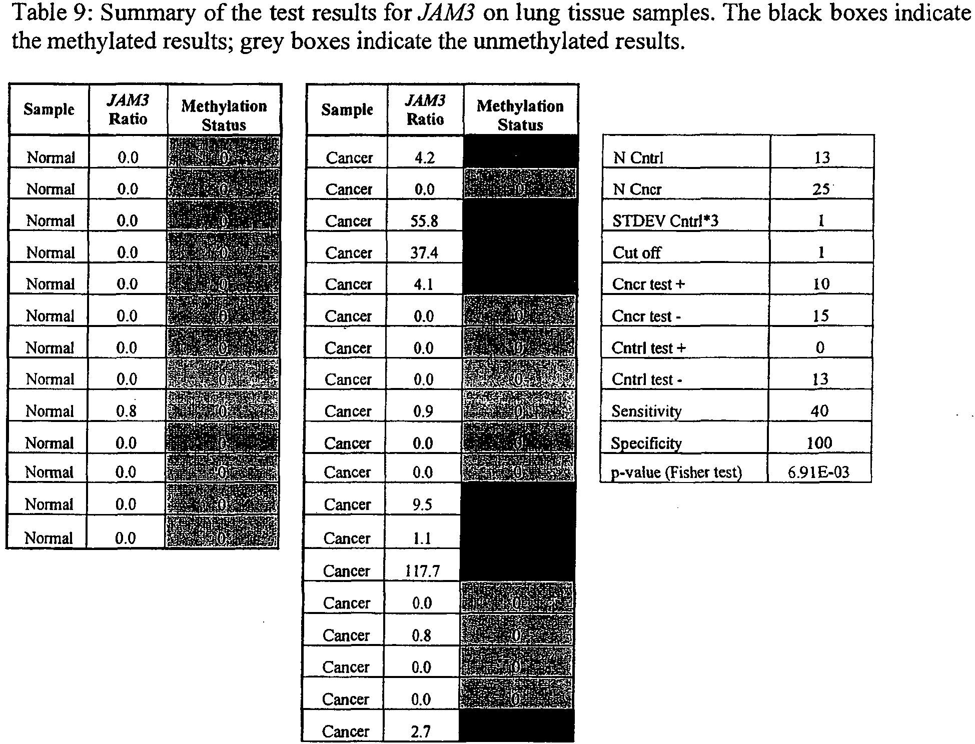

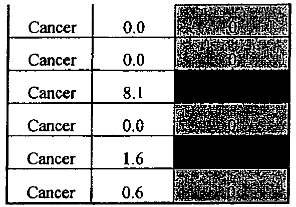

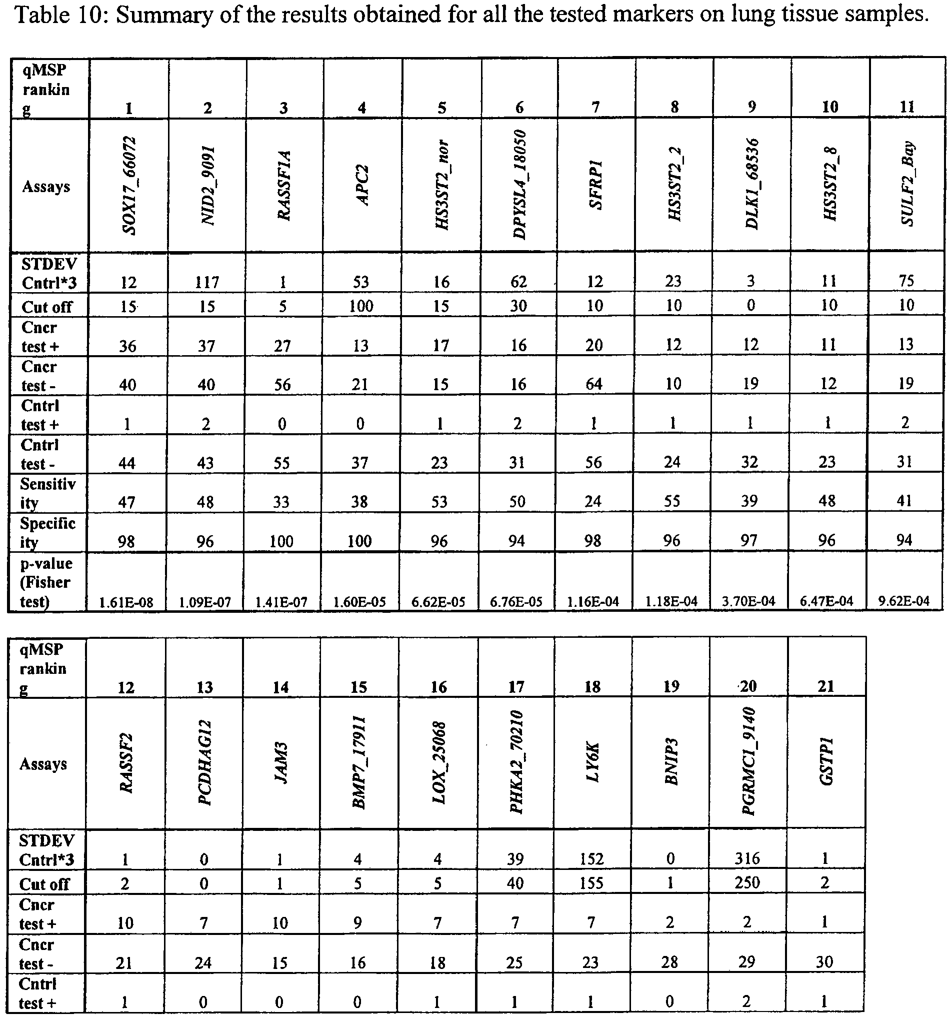

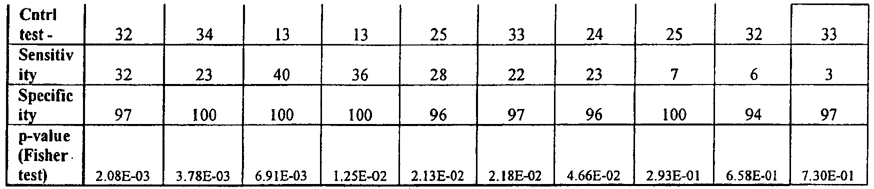

### EXAMPLE 5: Best performing markers tested on sputum samples

The control sputum samples were collected from the Lung Cancer Clinical Collaborative Research Agreement study of ONCO with the UMCG hospital (Groningen, The Netherlands). These samples were taken from participants to the NELSON screening program (a randomized controlled screening trial for lung cancer using multi-slice low-dose CT in high risk subjects - current smokers (55%) and former smokers (45%) who (had) smoked at least 16 cigarettes a day for at least 26 years or at least 11 cigarettes a day for at least 31 years).

The cancer sputum samples (stage IA #2, stage IIIA #3, stage IIIB #1, stage IV #1, stage unknown #1) were collected from the Lung Cancer Clinical Collaborative Research Agreement study of ONCO with Durham VA Medical Center (Durham, NC, USA). Patients with histologically proven NSCLC or patients suspected of having NSCLC planning to undergo resection and who have a predicted probability of 75% or more of having NSCLC (e.g., using nomograms such as at the worldwide web domain chestxray.com, at the page SPN/SPNProb.html) were included in the study.

Subjects were provided with a sterile cup containing Saccomanno's fixative and instructed to take a deep breath, cough deeply, and expectorate into the cup for 3 consecutive days. The samples were centrifuged at 1500xg for 15min to sediment all cellular material, the supernatants were removed and the cell pellet was washed with PBS. DNA was extracted from the sputum cells using standard salt-chloroform extraction and ethanol precipitation for high molecular DNA and dissolved in 250 µL TE buffer (10 mM Tris; 1 mM EDTA (pH 8.0)). DNA was quantified using the picogreen method and 20µg (or maximum amount if less than 20µg recovered from DNA extraction) of DNA was bisulphite treated using the EpiTect bisulfite kit (QIAGEN).

QMSP was performed after bisulphite treatment on denatured genomic DNA. The assays were carried out as described above, except that 960 ng of bisulphite converted genomic DNA was added in the reaction mixture. The samples were classified as methylated, unmethylated, or invalid as described above. The results based on **ratio** (copy number gene tested / copy number ACTB) and based on **copy number** obtained for all the tested markers on sputum samples from lung cancer patients and from control patients were ranked according their p-value (Fisher's exact test) (Table 11 - ratio, Table 12 - copy number).

Several combinations of markers were investigated to maximize sensitivity of detection, without significantly compromising specificity. The samples were classified as methylated if at least one of the tested markers scored positive based on ratio or based on copy number. Examples of the performance of combination of markers are summarized in Table 13 (ratio) and in Table 14 (copy number). Specificity above 90% is obtained for some combinations of markers (based on ratio and copy number). Sensitivity of 100% is obtained for some combinations of markers (based copy number).

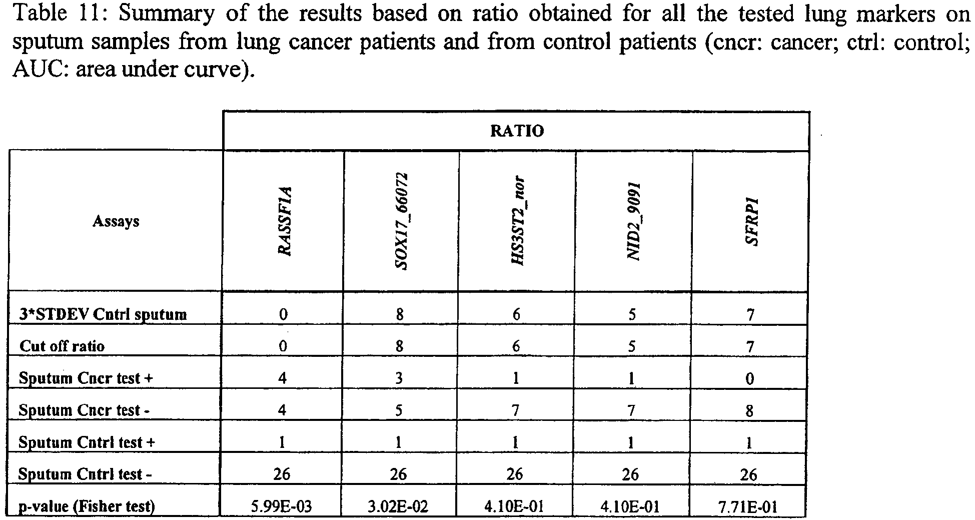

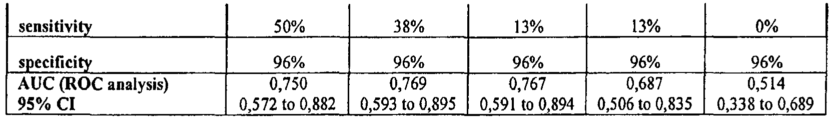

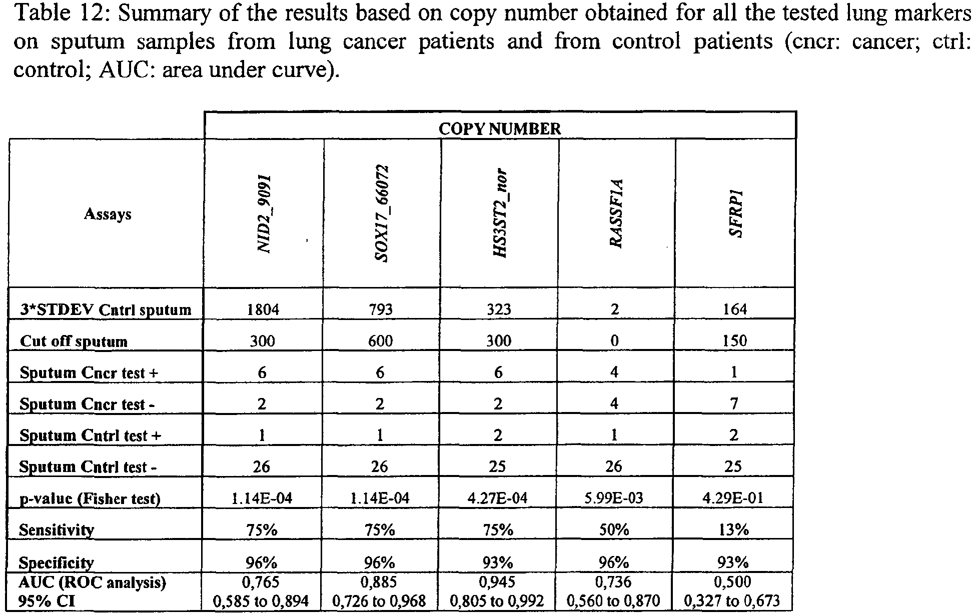

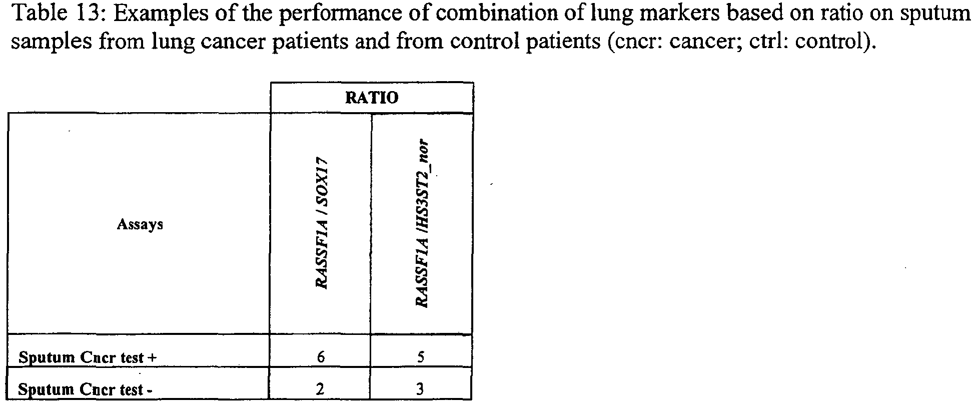

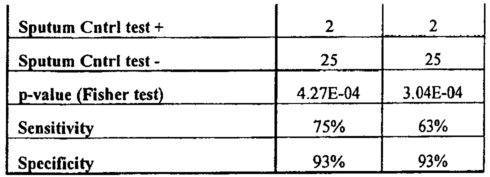

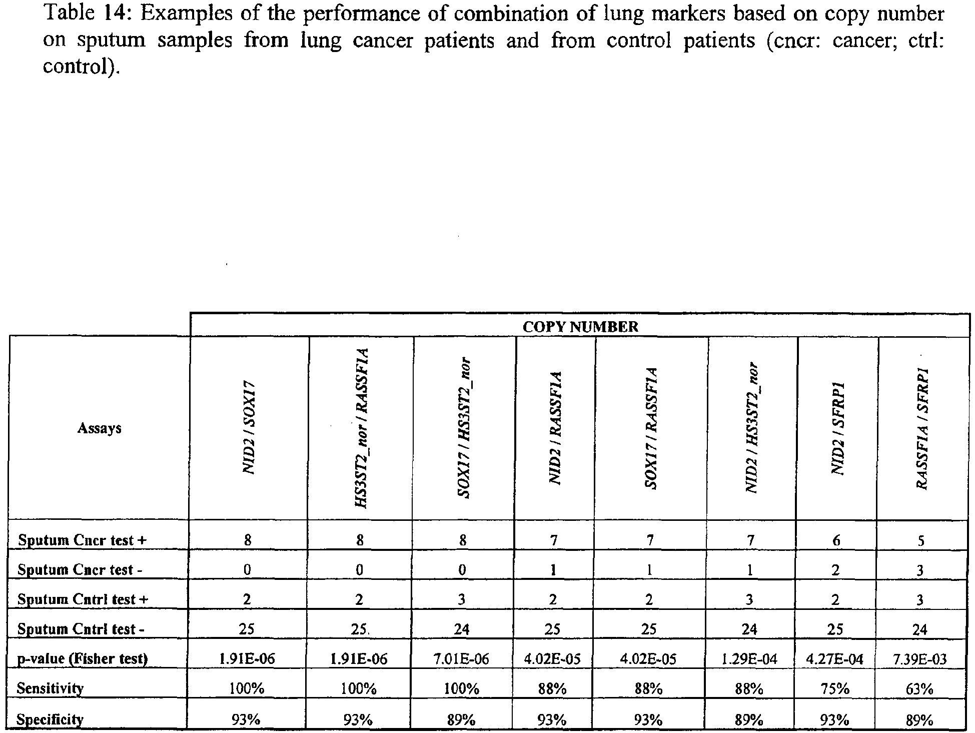

### References

The disclosure of each reference cited in this disclosure is expressly incorporated herein.
Barringer KJ, Orgel L, Wahl G, Gingeras TR.Gene. 1990 Apr 30;89(1):117-22
Esteller M, Corn PG, Baylin SB, Herman JG. A gene hypermethylation profile of human cancer. Cancer Res. 2001 Apr 15;61(8):3225-9.
Cross, SH et al. Nature Genetics 1994, 6, 236-244
Deng, D. et al. Simultaneous detection of CpG methylation and single nucleotide polymorphism by denaturing high performance liquid chromatography. 2002 Nuc. Acid Res, 30, 3.
Ganti, and Mulshine. Lung cancer screening. The Oncologist 2006, Vol. 11, No. 5, 481-487
Gentleman RC, Carey VJ, Bates DM, Bolstad B, Dettling M, et al. (2004) Bioconductor: open software development for computational biology and bioinformatics. Genome Biol 5: R80.
Greenberg and Lee. Biomarkers for Lung Cancer: Clinical Uses Curr Opin Pulm Med. 2007;13(4):249-255.
Greenlee, Hill-Harmon, Murray and Thun, Cancer statistics 2001, CA Cancer J. Clin. 2001; 51: 15-36.
Ihaka R, Gentleman RC (1996) A language for data analysis and graphics. Journal of Computational and Graphical Statistics 5: 299-314.
Kwoh DY, Davis GR, Whitfield KM, Chappelle HL, DiMichele LJ, Gingeras TR. Transcription-based amplification system and detection of amplified human immunodeficiency virus type 1 with a bead-based sandwich hybridization format. Proc Natl Acad Sci U S A. 1989 Feb;86(4):1173-7.
Li KB. ClustalW-MPI: ClustalW analysis using distributed and parallel computing. Bioinformatics 2003;19(12):1585-6.
Schuebel KE, Chen W, Cope L, Glöckner SC, Suzuki H, Yi JM, Chan TA, Van Neste L, Van Criekinge W, van den Bosch S, van Engeland M, Ting AH, Jair K, Yu W, Toyota M, Imai K, Ahuja N, Herman JG & Baylin SB (2007). Comparing the DNA hypermethylome with Gene Mutations in Human Colorectal Cancer. PLoS Genetics, 3 (8), Early Online Release*.*
Shiraisi, M et al. Biol Chem. 1999, 380(9):1127-1131
Sjöblom T, Jones S, Wood LD, Parsons DW, Lin J, Barber TD, Mandelker D, Leary RJ, Ptak J, Silliman N, Szabo S, Buckhaults P, Farrell C, Meeh P, Markowitz SD, Willis J, Dawson D, Willson JK, Gazdar AF, Hartigan J, Wu L, Liu C, Parmigiani G, Park BH, Bachman KE, Papadopoulos N, Vogelstein B, Kinzler KW & Velculescu VE (2006). The Consensus Coding Sequences of Human Breast and Colorectal Cancers. Science, 314 (5797), 268-274.
Straub, J. et al., A64-AACRMD (2007): Base5, a versatile, highly integrated high-throughput methylation profiling platform for Methylation-Specific PCR based marker identification applied to CRC
Suzuki Y, Yamashita R, Sugano S, Nakai K. DBTSS, DataBase of Transcriptional Start Sites: progress report 2004. Nucleic Acids Res 2004;32(Database issue):D78-81.
Suzuki Y, Yamashita R, Nakai K, Sugano S. DBTSS: DataBase of human Transcriptional Start Sites and full-length cDNAs. Nucleic Acids Res 2002;30(1):328-31.
Thompson JD, Higgins DG, Gibson TJ. CLUSTAL W: improving the sensitivity of progressive multiple sequence alignment through sequence weighting, position-specific gap penalties and weight matrix choice. Nucleic Acids Res 1994; 22(22):4673-80.
Tost, J. et al. Analysis and accurate quantification of CpG methylation by MALDI mass spectrometry. Nuc. Acid Res, 2003, 31(9): e50
Travis W.D., Pathology of lung cancer, Clin. Chest Med. 23 (2002), 65-81.
Trooskens G, De Beule D, Decouttere F, Van Criekinge W. Phylogenetic trees: visualizing, customizing and detecting incongruence. Bioinformatics 2005; 21(19):3801-2.

## Claims

1. A method for identifying lung cancer or its precursor, or predisposition to lung cancer, comprising:
detecting in a test sample containing lung cells or nucleic acids from lung cells, epigenetic modification of DPYSL4; and
identifying the test sample as containing cells that are neoplastic, precursor to neoplastic, or predisposed to neoplasia, or as containing nucleic acids from cells that are neoplastic, precursor to neoplastic, or predisposed to neoplasia.

2. The method of claim 1, further comprising detecting in the test sample epigenetic modification of at least one gene selected from the group consisting of SULF2, JAM3, APC2, BMP7, ACSL6, ALS2CL, ARTS-1, BEX1, NIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442, and ZNF655.

3. The method of claim 1 or 2 wherein:
(1) the test sample contains squamous cells or nucleic acids from squamous cells;
(2) the test sample contains adenocarcinoma cells or nucleic acids from adenocarcinoma cells;
(3) the test sample contains large cell carcinoma cells or nucleic acids from large cell carcinoma cells;
(4) the test sample contains a mixture of squamous cells, adenocarcinoma cells, and large cell carcinoma cells; or
(5) the test sample is from a specimen selected from the group consisting of a tissue specimen, a biopsy specimen, a surgical specimen, a cytological specimen, sputum specimen, pleural fluid and a bronchoalveolar lavage, wherein preferably the test sample is from a biopsy specimen and surgical removal of neoplastic tissue is recommended to the patient or the specimen is a surgical specimen and adjuvant chemotherapy or adjuvant radiation therapy is recommended to the patient.

4. The method of claim 2, wherein an epigenetic modification in a panel of genes comprising two, three, four or five genes is detected, wherein detection of an epigenetic change in at least DPYSL4 gene in the panel is indicative of a predisposition to, or the incidence of lung cancer, preferably epigenetic modification of RASSF1A and/or SOX17 and/or HS3ST2-nor and/or NID2 and/or SFRP1 is further detected.

5. The method of claim 1 or 2 wherein epigenetic modification is detected by detecting methylation of a CpG dinucleotide motif in the gene, and optionally
(1) methylation is detected by contacting at least a portion of the gene with a methylation-sensitive restriction endonuclease, said endonuclease preferentially cleaving methylated recognition sites relative to non-methylated recognition sites, whereby cleavage of the portion of the gene indicates methylation of the portion of the gene;
(2) methylation is detected by contacting at least a portion of the gene with a methylation-sensitive restriction endonuclease, said endonuclease preferentially cleaving non-methylated recognition sites relative to methylated recognition sites, whereby cleavage of the portion of the gene indicates non-methylation of the portion of the gene provided that the gene comprises a recognition site for the methylation-sensitive restriction endonuclease; or
(3) contacting at least a portion of the gene of the test sample with a chemical reagent that selectively modifies a non-methylated cytosine residue relative to a methylated cytosine residue, or selectively modifies a methylated cytosine residue relative to a non-methylated cytosine residue; and detecting a product generated due to said contacting, and preferably
(a) the step of detecting a product employs amplification with at least one primer that hybridizes to a sequence comprising a modified non-methylated CpG dinucleotide motif but not to a sequence comprising an unmodified methylated CpG dinucleotide motif thereby forming amplification products;
(b) the step of detecting a product comprises amplification with at least one primer that hybridizes to a sequence comprising an unmodified methylated CpG dinucleotide motif but not to a sequence comprising a modified non-methylated CpG dinucleotide motif thereby forming amplification products;
(c) the product is detected by a method selected from the group consisting of electrophoresis, hybridization, amplification, sequencing, ligase chain reaction, chromatography, mass spectrometry, and combinations thereof;
(d) the chemical reagent is hydrazine, wherein preferably the method further comprises cleavage of the hydrazine contacted portion of the gene with piperidine; or
(e) the chemical reagent comprises bisulfite ions, wherein preferably the method further comprises treating the bisulfite ion-contacted portion of the gene with alkali.

6. The method of claim 1 or 2 wherein epigenetic modification is detected by
(1) detecting methylation of a CpG dinucleotide motif in a promoter, intron or exon of the gene;
(2) detecting diminished expression of mRNA of the gene;
(3) detecting hypomethylation of a CpG dinucleotide motif in the gene;
(4) detecting hypomethylation of a CpG dinucleotide motif in a promoter of the gene; or
(5) detecting increased expression of mRNA of the gene.

7. The method of claim 1 wherein
(1) the step of detecting employs amplification of at least a portion of the DPYSL4 gene using an oligonucleotide primer that specifically hybridizes under amplification conditions to a region of the DPYSL4 gene, wherein the region is within about 3kb of DPYSL4's transcription start site;
(2) the step of detecting employs amplification of at least a portion the DPYSL4 gene using at least one pair of oligonucleotide primers that specifically hybridizes under amplification conditions to a region of the DPYSL4 gene; wherein the region is within about 3 kb of DPYSL4's transcription start site, wherein optionally an oligonucleotide probe is covalently linked to the oligonucleotide primer, and wherein it is further preferred that the region comprises, consists essentially of or consists of the sequences represented by SEQ ID NO. 145 and/or SEQ ID NO. 146 and/or SEQ ID NO. 209 and/or SEQ ID NO. 210 and/or SEQ ID NO. 412 and/or SEQ ID NO. 433;
(3) the step of detecting a product comprises amplification with at least one sense primer comprising, consisting essentially of or consisting of SEQ ID NO. 17-18 and/or SEQ ID NO. 349;
(4) the step of detecting a product comprises amplification with at least one antisense primer comprising, consisting essentially of or consisting of SEQ ID NO. 81-82 and/or SEQ ID NO. 370;
(5) the step of detecting employs amplification of at least a portion of the DPYSL4 gene, and further employs at least one oligonucleotide probe which hybridizes to an amplicon selected from the group consisting of SEQ ID NO. 145 and/or SEQ ID NO. 146 and/or SEQ ID NO. 209 and/or SEQ ID NO. 210 and/or SEQ ID NO. 412 and/or SEQ ID NO. 433 under amplification conditions, wherein preferably the probe comprises, consists essentially of or consists of sequences represented by SEQ ID NO. 391; or
(6) the step of detecting employs amplification of at least a portion of the DPYSL4 gene and a detectable reagent which preferentially binds to double stranded DNA relative to single stranded DNA.

8. The method of claim 2 wherein
(1) the step of detecting employs amplification of at least a portion of the at least one gene using an oligonucleotide primer that specifically hybridizes under amplification conditions to a region of a gene selected from the group consisting of SULF2, JAM3, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442, and ZNF655; wherein the region is within about 3 kb of said gene's transcription start site;
(2) the step of detecting employs amplification of at least a portion of the at least one gene using at least one pair of oligonucleotide primers that specifically hybridizes under amplification conditions to a region of a gene selected from the group consisting of SULF2, JAM3, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442, and ZNF655; wherein the region is within about 3 kb of said gene's transcription start site, wherein optionally an oligonucleotide probe is covalently linked to the oligonucleotide primer, and wherein it is further preferred that the region comprises, consists essentially of or consists of the sequences represented by SEQ ID NO. 129-144 and/or SEQ ID NO. 147 - 208 and/or SEQ ID NO. 211-256 and/or SEQ ID NO. 315-329 and/or SEQ ID NO. 330-344 and/or SEQ ID NO. 408-411 and/or SEQ ID NO. 413-428 and/or SEQ ID NO. 429-432 and/or SEQ ID NO. 434-449 and/or SEQ ID NO. 271-277 and/or SEQ ID NO. 278-284;
(3) the step of detecting a product comprises amplification with at least one sense primer comprising, consisting essentially of or consisting of SEQ ID NO. 1-16 and/or SEQ ID NO. 19-64 and/or SEQ ID NO. 285-299 and/or SEQ ID NO. 345-348 and/or SEQ ID NO. 350-365 and/or SEQ ID NO. 257-263;
(4) the step of detecting a product comprises amplification with at least one antisense primer comprising, consisting essentially of or consisting of SEQ ID NO. 65-80 and/or SEQ ID NO. 83-128 and/or SEQ ID NO. 300-314 and/or SEQ ID NO. 366-369 and/or SEQ ID NO. 371-386 and/or SEQ ID NO. 264-270;
(5) the step of detecting employs amplification of at least a portion of the at least one gene, and further employs at least one oligonucleotide probe which hybridizes to an amplicon selected from the group consisting of SEQ ID NO: 129-144 and/or SEQ ID NO. 147 - 208 and/or SEQ ID NO. 211-256 and/or SEQ ID NO. 315-329 and/or SEQ ID NO. 330-344 and/or SEQ ID NO. 408-411 and/or SEQ ID NO. 413-428 and/or SEQ ID NO. 429-432 and/or SEQ ID NO. 434-449 and/or SEQ ID NO. 271-277 and/or SEQ ID NO. 278-284 under amplification conditions, wherein preferably the probe comprises, consists essentially of or consists of sequences represented by SEQ ID NO. 387-390 and/or SEQ ID NO. 392-407; or
(6) the step of detecting employs amplification of at least a portion of the at least one gene and a detectable reagent which preferentially binds to double stranded DNA relative to single stranded DNA.

9. A kit for assessing lung cancer or its precursor, or predisposition to lung cancer in a test sample containing lung cells or nucleic acids from lung cells, said kit comprising in a package:
a reagent that (a) modifies methylated cytosine residues but not non-methylated cytosine residues, or that (b) modifies non-methylated cytosine residues but not methylated cytosine residues; and
at least one pair of oligonucleotide primers that specifically hybridizes under amplification conditions to a region of a DPYSL4 gene; wherein the region is within about 3 kb of DPYSL4's transcription start site.

10. The kit of claim 9 wherein
(1) the at least one pair of oligonucleotide primers is selected from SEQ ID NO: 17, 18, 81, 82, 349 and 370; or
(2) the at least one pair of oligonucleotide primers amplifies an amplicon selected from SEQ ID No. 145, 146, 209, 210, 412 and 433.

11. The kit of claim 9 or 10, further comprising at least one pair of oligonucleotide primers that specifically hybridizes under amplification conditions to a region of a gene selected from the group consisting of SULF2, JAM3, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442, and ZNF655; wherein the region is within about 3 kb of said gene's transcription start site; and optionally
(1) the at least one pair of primers is selected from Table 1 (SEQ ID NO: 1-16, 19-80 and 83-128), Figure 2 (SEQ ID NO: 257-270), Table 3 (SEQ ID NO: 285-314) and Table 7 (SEQ ID NO: 345-348, 350-369 and 371-386); or
(2) the at least one pair of oligonucleotide primers amplifies an amplicon selected from Table 2 (SEQ ID NO: 129-144 and 147 - 208 and 211-256), Figure 2 (SEQ ID NO: 271-284),Table 4 (SEQ ID NO: 315-344) and Table 8 (SEQ ID NO: 408-411, 413-432 and 434-449).

12. A kit for assessing lung cancer or its precursor, or predisposition to lung cancer in a test sample containing lung cells or nucleic acids from lung cells, said kit comprising in a package:
at least two pairs of oligonucleotide primers that specifically hybridize under amplification conditions to a region of a DPYSL4 gene, wherein the region is within about 3kb of DPYSL4's transcription start site.

13. The kit of claim 12, wherein
(1) the at least two pairs of primers are selected from SEQ ID NO: 17, 18, 81, 82, 349 and 370; or
(2) the at least two pairs of oligonucleotide primers amplify amplicons selected from SEQ ID NO: 145, 146, 209, 210, 412 and 433.

14. The kit of claim 12 or 13 further comprising at least two pairs of oligonucleotide primers that specifically hybridize under amplification conditions to a region of a gene selected from the group consisting of, SULF2, JAM3, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442, and ZNF655; wherein the region is within about 3 kb of said gene's transcription start site; and optionally
(1) the at least two pairs of primers are selected from SEQ ID NO: 1-16, 19-80 and 83-128 (Table 1), SEQ ID NO: 257-270 (Figure 2), SEQ ID NO:285-314 (Table 3), SEQ ID NO: 345-348, 350-369 and 371-386 (Table 7); or
(2) the at least two pairs of oligonucleotide primers amplify amplicons selected from Table 2 (SEQ ID NO: 129-144, 147-208 and 211 - 256), Figure 2 (SEQ ID NO: 271-284), Table 4 (SEQ ID NO: 315-344) and Table 8 (SEQ ID NO: 408-411, 413-432 and 434-449).

15. The kit of claim 9 or 12 further comprising at least one oligonuleotide probe which hybridizes to an amplicon selected from the group consisting of SEQ ID NO. 146, 146, 209, 210, 412 and 433 under amplification conditions, wherein preferably the oligonucleotide probe is SEQ ID NO. 391 and more preferably the oligonucleotide probe comprises a fluorescent label, a fluorescence quenching agent, or a fluorescent label and fluorescence quenching agent.

16. The kit of claim 11 or 14 further comprising at least one oligonucleotide probe which hybridizes to an amplicon selected from the group consisting of Table 2 (SEQ ID NO: 129-144, 147-208 and 211-256), Figure 2 (SEQ ID NO: 271-284), Table 4 (SEQ ID NO: 315-344) and Table 8 (SEQ ID NO: 408-411, 413-432 and 434-449) under amplification conditions,
wherein preferably the oligonucleotide probe is selected from the group consisting of SEQ ID NO: 387-390 and 392-407, and more preferably the oligonucleotide probe comprises a fluorescent label, a fluorescence quenching agent, or a fluorescent label and fluorescence quenching agent.

17. The kit of claim 9, 11, 12 or 14 which comprises:
(1) a detectable reagent which preferentially binds to double stranded DNA relative to single stranded DNA;
(2) a DNA polymerase for amplifying DNA; and/or
(3) at least one oligonucleotide probe which is covalently linked to at least one of said oligonucleotide primers.

18. An isolated polynucleotide that:
(1) comprises a nucleotide sequence selected from the group consisting of SEQ ID NO: 17, 81, 145 and 391, wherein preferably the isolated polynucleotide consists of the selected nucleotide sequence; or
(2) consists of a nucleotide sequence selected from SEQ ID NO. 18, 82, 146, 209, 210, 349, 370, 412 and 433
wherein optionally the isolated polynucleotide is detectably labeled, preferably with a fluorescent label.

## Patentansprüche

1. Verfahren zum Identifizieren von Lungenkrebs oder einer Vorstufe davon oder einer Veranlagung für Lungenkrebs, umfassend:
Detektieren einer epigenetischen Modifikation von DPYSL4 in einer Testprobe, die Lungenzellen oder Nucleinsäuren von Lungenzellen enthält; und
Identifizieren der Testprobe, dass sie Zellen enthält, die neoplastisch, Vorläufer für neoplastische Zellen oder veranlagt für Neoplasie sind, oder dass sie Nucleinsäuren von Zellen enthält, die neoplastisch, Vorläufer für neoplastische Zellen oder veranlagt für Neoplasie sind.

2. Verfahren nach Anspruch 1, das ferner ein Detektieren einer epigenetischen Modifikation von mindestens einem Gen, ausgewählt aus der Gruppe, bestehend aus SULF2, JAM3, APC2, BMP7, ACSL6, ALS2CL, ARTS-1, BEX1, BNIP3, CBR3, CD248, CD44, CHD5, DLK1, DSC2, EDNRB, EPB41 L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442 und ZNF655, in der Testprobe umfasst.

3. Verfahren nach Anspruch 1 oder 2, wobei:
(1) die Testprobe Plattenepithelzellen oder Nucleinsäuren von Plattenepithelzellen enthält;
(2) die Testprobe Adenokarzinomzellen oder Nucleinsäuren von Adenokarzinomzellen enthält;
(3) die Testprobe großzellige Karzinomzellen oder Nucleinsäuren von großzelligen Karzinomzellen enthält;
(4) die Testprobe eine Mischung von Plattenepithelzellen, Adenokarzinomzellen und großzelligen Karzinomzellen enthält; oder
(5) die Testprobe von einem Untersuchungsmaterial ist, ausgewählt aus der Gruppe, bestehend aus einer Gewebeprobe, einer Biopsieprobe, einer chirurgischen Probe, einer zytologischen Probe, einer Sputumprobe, einer Flüssigkeit im Pleuraraum und einer bronchoalveolären Spülung, wobei die Testprobe vorzugsweise von einer Biopsieprobe ist und eine chirurgische Entfernung des neoplastischen Gewebes dem Patienten empfohlen wird oder das Untersuchungsmaterial eine chirurgische Probe ist und eine adjuvante Chemotherapie oder adjuvante Strahlentherapie dem Patienten empfohlen wird.

4. Verfahren nach Anspruch 2, wobei eine epigenetische Modifikation in einem Panel an Genen, das zwei, drei, vier oder fünf Gene umfasst, detektiert wird, wobei die Detektion einer epigenetischen Veränderung bei zumindest dem DPYSL4-Gen in dem Panel eine Veranlagung für oder das Auftreten von Lungenkrebs indiziert, wobei vorzugsweise ferner eine epigenetische Modifikation von RASSF1A und/oder SOX17 und/oder HS3ST2-nor und/oder NID2 und/oder SFRP1 detektiert wird.

5. Verfahren nach Anspruch 1 oder 2, wobei eine epigenetische Modifikation detektiert wird, indem die Methylierung eines CpG-Dinucleotidmotivs in dem Gen detektiert wird, und optional
(1) die Methylierung detektiert wird durch Kontaktieren von mindestens einem Teil des Gens mit einer Methylierungs-empfindlichen Restriktionsendonuclease, wobei die Endonuclease bevorzugt methylierte Erkennungsstellen gegenüber nichtmethylierten Erkennungsstellen spaltet, wobei eine Spaltung des Teils des Gens eine Methylierung des Teils des Gens anzeigt;
(2) die Methylierung detektiert wird durch Kontaktieren von mindestens einem Teil des Gens mit einer Methylierungs-empfindlichen Restriktionsendonuclease, wobei die Endonuclease bevorzugt nicht-methylierte Erkennungsstellen gegenüber methylierten Erkennungsstellen spaltet, wobei eine Spaltung des Teils des Gens eine Nicht-Methylierung des Teils des Gens anzeigt, vorausgesetzt, dass das Gen eine Erkennungsstelle für die Methylierungs-empfindliche Restriktionsendonuclease umfasst; oder
(3) Kontaktieren von mindestens einem Teil des Gens der Testprobe mit einem chemischen Reagenz, das selektiv einen nicht-methylierten Cytosinrest gegenüber einem methylierten Cytosinrest modifiziert oder selektiv einen methylierten Cytosinrest gegenüber einem nicht-methylierten Cytosinrest modifiziert; und Detektieren eines auf Grund des Kontaktierens erzeugten Produkts, und vorzugsweise (a) verwendet der Schritt des Detektierens eines Produkts eine Amplifikation mit mindestens einem Primer, der an eine Sequenz, die ein modifiziertes nicht-methyliertes CpG-Dinucleotidmotiv umfasst, aber nicht an eine Sequenz, die ein unmodifiziertes methyliertes CpG-Dinucleotidmotiv umfasst, hybridisiert, wodurch Amplifikationsprodukte gebildet werden;
(b) umfasst der Schritt des Detektierens eines Produkts eine Amplifikation mit mindestens einem Primer, der an eine Sequenz, die ein unmodifiziertes methyliertes CpG-Dinucleotidmotiv umfasst, aber nicht an eine Sequenz, die ein modifiziertes nichtmethyliertes CpG-Dinucleotidmotiv umfasst, hybridisiert, wodurch Amplifikationsprodukte gebildet werden;
(c) wird das Produkt, durch ein Verfahren, ausgewählt aus der Gruppe, bestehend aus Elektrophorese, Hybridisierung, Amplifikation, Sequenzierung, Ligasekettenreaktion, Chromatographie, Massenspektrometrie und Kombinationen davon, detektiert;
(d) ist das chemische Reagenz Hydrazin, wobei des Verfahren vorzugsweise ferner das Abspalten des mit Hydrazin kontaktierten Teils des Gens mit Piperidin umfasst; oder
(e) umfasst das chemische Reagenz Bisulfitionen, wobei des Verfahren vorzugsweise ferner das Behandeln des mit Bisulfitionen kontaktierten Teils des Gens mit Lauge umfasst.

6. Verfahren nach Anspruch 1 oder 2, wobei die epigenetische Modifikation detektiert wird durch
(1) Detektieren der Methylierung eines CpG-Dinucleotidmotivs in einem Promotor, Intron oder Exon des Gens;
(2) Detektieren einer verminderten Expression von mRNA des Gens;
(3) Detektieren einer Hypomethylierung eines CpG-Dinucleotidmotivs in dem Gen;
(4) Detektieren einer Hypomethylierung eines CpG-Dinucleotidmotivs in einem Promotor des Gens; oder
(5) Detektieren einer erhöhten Expression von mRNA des Gens.

7. Verfahren nach Anspruch 1, wobei
(1) der Schritt des Detektierens eine Amplifikation von mindestens einem Teil des DPYSL4-Gens unter Verwendung eines Oligonucleotidprimers, der spezifisch unter Amplifikationsbedingungen an einen Bereich des DPYSL4-Gens hybridisiert, wobei der Bereich innerhalb von etwa 3 kB der Transkriptionsstartstelle von DPYSL4 liegt, verwendet;
(2) der Schritt des Detektierens eine Amplifikation von mindestens einem Teil des DPYSL4-Gens unter Verwendung von mindestens einem Paar an Oligonucleotidprimern, das spezifisch unter Amplifikationsbedingungen an einen Bereich des DPYSL4-Gens hybridisiert, wobei der Bereich innerhalb von etwa 3 kB der Transkriptionsstartstelle von DPYSL4 liegt, verwendet, wobei optional eine Oligonucleotidsonde kovalent an den Oligonucleotidprimer gebunden ist, und wobei es ferner bevorzugt ist, dass der Bereich die durch SEQ ID No. 145 und/oder SEQ ID No. 146 und/oder SEQ ID No. 209 und/oder SEQ ID No. 210 und/oder SEQ ID No. 412 und/oder SEQ ID No. 433 dargestellten Sequenzen umfasst, im wesentlichen daraus besteht oder daraus besteht;
(3) der Schritt des Detektierens eines Produkts eine Amplifikation mit mindestens einem sense-Primer umfasst, der SEQ ID No. 17-18 und/oder SEQ ID No. 349 umfasst, im wesentlichen daraus besteht oder daraus besteht;
(4) der Schritt des Detektierens eines Produkts eine Amplifikation mit mindestens einem antisense-Primer umfasst, der SEQ ID No. 81-82 und/oder SEQ ID No. 370 umfasst, im wesentlichen daraus besteht oder daraus besteht;
(5) der Schritt des Detektierens eine Amplifikation von mindestens einem Teil des DPYSL4-Gens verwendet und ferner mindestens eine Oligonucleotidsonde verwendet, die an ein Amplikon, ausgewählt aus der Gruppe, bestehend aus SEQ ID No. 145 und/oder SEQ ID No. 146 und/oder SEQ ID No. 209 und/oder SEQ ID No. 210 und/oder SEQ ID No. 412 und/oder SEQ ID No. 433, unter Amplifikationsbedingungen hybridisiert, wobei die Sonde vorzugsweise die durch SEQ ID No. 391 dargestellte Sequenz umfasst, im wesentlichen daraus besteht oder daraus besteht; oder
(6) der Schritt des Detektierens eine Amplifikation von mindestens einem Teil des DPYSL4-Gens und einem detektierbaren Reagenz, das bevorzugt an doppelsträngiger DNA gegenüber einzelsträngiger DNA bindet, verwendet.

8. Verfahren nach Anspruch 2, wobei
(1) der Schritt des Detektierens eine Amplifikation von mindestens einem Teil von dem mindestens einem Gen unter Verwendung eines Oligonucleotidprimers, der spezifisch unter Amplifikationsbedingungen an einen Bereich eines Gens, ausgewählt aus der Gruppe, bestehend aus SULF2, JAM3, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442 und ZNF655, hybridisiert, wobei der Bereich innerhalb von etwa 3 kB der Transkriptionsstartstelle des Gens liegt, verwendet;
(2) der Schritt des Detektierens eine Amplifikation von mindestens einem Teil von dem mindestens einem Gen unter Verwendung von mindestens einem Paar an Oligonucleotidprimern, das spezifisch unter Amplifikationsbedingungen an einen Bereich eines Gens, ausgewählt aus der Gruppe, bestehend aus SULF2, JAM3, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442 und ZNF655, hybridisiert, wobei der Bereich innerhalb von etwa 3 kB der Transkriptionsstartstelle des Gens liegt, verwendet, wobei optional eine Oligonucleotidsonde kovalent an den Oligonucleotidprimer gebunden ist, und wobei es ferner bevorzugt ist, dass der Bereich die durch SEQ ID No. 129-144 und/oder SEQ ID No. 147-208 und/oder SEQ ID No. 211-256 und/oder SEQ ID No. 315-329 und/oder SEQ ID No. 330-344 und/oder SEQ ID No. 408-411 und/oder SEQ ID No. 413-428 und/oder SEQ ID No. 429-432 und/oder SEQ ID No. 434-449 und/oder SEQ ID No. 271-277 und/oder SEQ ID No. 278-284 dargestellten Sequenzen umfasst, im wesentlichen daraus besteht oder daraus besteht;
(3) der Schritt des Detektierens eines Produkts eine Amplifikation mit mindestens einem sense-Primer umfasst, der SEQ ID No. 1-16 und/oder SEQ ID No. 19-64 und/oder SEQ ID No. 285-299 und/oder SEQ ID No. 345-348 und/oder SEQ ID No. 350-365 und/oder SEQ ID No. 257-263 umfasst, im wesentlichen daraus besteht oder daraus besteht;
(4) der Schritt des Detektierens eines Produkts eine Amplifikation mit mindestens einem antisense-Primer umfasst, der SEQ ID No. 65-80 und/oder SEQ ID No. 83-128 und/oder SEQ ID No. 300-314 und/oder SEQ ID No. 366-369 und/oder SEQ ID No. 371-386 und/oder SEQ ID No. 264-270 umfasst, im wesentlichen daraus besteht oder daraus besteht;
(5) der Schritt des Detektierens eine Amplifikation von mindestens einem Teil von dem mindestens einem Gen verwendet und ferner mindestens eine Oligonucleotidsonde verwendet, die an ein Amplikon, ausgewählt aus der Gruppe, bestehend aus SEQ ID No. 129-144 und/oder SEQ ID No. 147-208 und/oder SEQ ID No. 211-256 und/oder SEQ ID No. 315-329 und/oder SEQ ID No. 330-344 und/oder SEQ ID No. 408-411 und/oder SEQ ID No. 413-428 und/oder SEQ ID No. 429-432 und/oder SEQ ID No. 434-449 und/oder SEQ ID No. 271-277 und/oder SEQ ID No. 278-284, unter Amplifikationsbedingungen hybridisiert, wobei die Sonde vorzugsweise die durch SEQ ID No. 387-390 und/oder SEQ ID No. 392-407 dargestellten Sequenzen umfasst, im wesentlichen daraus besteht oder daraus besteht; oder
(6) der Schritt des Detektierens eine Amplifikation von mindestens einem Teil von dem mindestens einem Gen und einem detektierbaren Reagenz, das bevorzugt an doppelsträngiger DNA gegenüber einzelsträngiger DNA bindet, verwendet.

9. Kit zum Beurteilen von Lungenkrebs oder einer Vorstufe davon oder einer Veranlagung für Lungenkrebs in einer Testprobe, die Lungenzellen oder Nucleinsäuren von Lungenzellen enthält; wobei das Kit in einer Packung folgendes umfasst:
ein Reagenz, das (a) methylierte Cytosinreste, aber nicht nicht-methylierte Cytosinreste modifiziert oder das (b) nicht-methylierte Cytosinreste, aber nicht methylierte Cytosinreste modifiziert; und
mindestens ein Paar an Oligonucleotidprimern, das spezifisch unter Amplifikationsbedingungen an einen Bereich eines DPYSL4-Gens hybridisiert, wobei der Bereich innerhalb von etwa 3 kB der Transkriptionsstartstelle von DPYSL4 liegt.

10. Kit nach Anspruch 9, wobei
(1) das mindestens eine Paar an Oligonucleotidprimern aus SEQ ID No. 17, 18, 81, 82, 349 und 370 ausgewählt ist; oder
(2) das mindestens eine Paar an Oligonucleotidprimern ein Amplikon, ausgewählt aus SEQ ID No. 145, 146, 209, 210, 412 und 433, amplifiziert.

11. Kit nach Anspruch 9 oder 10, das ferner mindestens ein Paar an Oligonucleotidprimern umfasst, das spezifisch unter Amplifikationsbedingungen an einen Bereich eines Gens, ausgewählt aus der Gruppe, bestehend aus SULF2, JAM3, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442 und ZNF655, hybridisiert, wobei der Bereich innerhalb von etwa 3 kB der Transkriptionsstartstelle des Gens liegt; und optional
(1) das mindestens eine Paar an Oligonucleotidprimern aus Tabelle 1 (SEQ ID No. 1-16, 19-80 und 83-128), Figur 2 (SEQ ID No. 257-270), Tabelle 3 (SEQ ID No. 285-314) und Tabelle 7 (SEQ ID No. 345-348, 350-369 und 371-386) ausgewählt ist; oder
(2) das mindestens eine Paar an Oligonucleotidprimern ein Amplikon, ausgewählt aus Tabelle 2 (SEQ ID No. 129-144 und 147-208 und 211-256), Figur 2 (SEQ ID No. 271-284), Tabelle 4 (SEQ ID No. 315-344) und Tabelle 8 (SEQ ID No. 408-411, 413-432 und 434-449), amplifiziert.

12. Kit zum Beurteilen von Lungenkrebs oder einer Vorstufe davon oder einer Veranlagung für Lungenkrebs in einer Testprobe, die Lungenzellen oder Nucleinsäuren von Lungenzellen enthält; wobei das Kit in einer Packung folgendes umfasst:
mindestens zwei Paare an Oligonucleotidprimern, die spezifisch unter Amplifikationsbedingungen an einen Bereich eines DPYSL4-Gens hybridisieren, wobei der Bereich innerhalb von etwa 3 kB der Transkriptionsstartstelle von DPYSL4 liegt.

13. Kit nach Anspruch 12, wobei
(1) die mindestens zwei Paare an Oligonucleotidprimern aus SEQ ID No. 17, 18, 81, 82, 349 und 370 ausgewählt sind; oder
(2) die mindestens zwei Paare an Oligonucleotidprimern Amplikone, ausgewählt aus SEQ ID No. 145, 146, 209, 210, 412 und 433, amplifizieren.

14. Kit nach Anspruch 12 oder 13, das ferner mindestens zwei Paare an Oligonucleotidprimern umfasst, die spezifisch unter Amplifikationsbedingungen an einen Bereich eines Gens, ausgewählt aus der Gruppe, bestehend aus SULF2, JAM3, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442 und ZNF655, hybridisieren, wobei der Bereich innerhalb von etwa 3 kB der Transkriptionsstartstelle des Gens liegt; und optional
(1) die mindestens zwei Paare an Oligonucleotidprimern aus SEQ ID No. 1-16, 19-80 und 83-128 (Tabelle 1), SEQ ID No. 257-270 (Figur 2), SEQ ID No. 285-314 (Tabelle 3) und SEQ ID No. 345-348, 350-369 und 371-386 (Tabelle 7) ausgewählt sind; oder
(2) die mindestens zwei Paare an Oligonucleotidprimern Amplikone, ausgewählt aus Tabelle 2 (SEQ ID No. 129-144, 147-208 und 211-256), Figur 2 (SEQ ID No. 271-284), Tabelle 4 (SEQ ID No. 315-344) und Tabelle 8 (SEQ ID No. 408-411, 413-432 und 434-449), amplifizieren.

15. Kit nach Anspruch 9 oder 12, das ferner mindestens eine Oligonucleotidsonde umfasst, die an ein Amplikon, ausgewählt aus SEQ ID No. 146, 146, 209, 210, 412 und 433, unter Amplifikationsbedingungen hybridisiert, wobei die Oligonucleotidsonde vorzugsweise SEQ ID No. 391 ist und stärker bevorzugt die Oligonucleotidsonde eine Fluoreszenzmarkierung, ein Fluoreszenz quenchendes Mittel oder eine Fluoreszenzmarkierung und ein Fluoreszenz quenchendes Mittel umfasst.

16. Kit nach Anspruch 11 oder 14, das ferner mindestens eine Oligonucleotidsonde umfasst, die an ein Amplikon, ausgewählt aus der Gruppe, bestehend aus Tabelle 2 (SEQ ID No. 129-144, 147-208 und 211-256), Figur 2 (SEQ ID No. 271-284), Tabelle 4 (SEQ ID No. 315-344) und Tabelle 8 (SEQ ID No. 408-411, 413-432 und 434-449), unter Amplifikationsbedingungen hybridisiert,
wobei die Oligonucleotidsonde vorzugsweise aus der Gruppe, bestehend aus SEQ ID No. 387-390 und 392-407, ausgewählt ist und stärker bevorzugt die Oligonucleotidsonde eine Fluoreszenzmarkierung, ein Fluoreszenz quenchendes Mittel oder eine Fluoreszenzmarkierung und ein Fluoreszenz quenchendes Mittel umfasst.

17. Kit nach Anspruch 9, 11, 12 oder 14, das folgendes umfasst:
(1) ein detektierbares Reagenz, das bevorzugt an doppelsträngiger DNA gegenüber einzelsträngiger DNA bindet;
(2) eine DNA-Polymerase zur Amplifizierung von DNA; und/oder
(3) mindestens eine Oligonucleotidsonde, die kovalent an mindestens einen der Oligonucleotidprimer bindet.

18. Isoliertes Polynucleotid, das:
(1) eine Nucleotidsequenz, ausgewählt aus der Gruppe, bestehend aus SEQ ID No. 17, 81, 145 und 391, umfasst, wobei vorzugsweise das isolierte Polynucleotid aus der ausgewählten Nucleotidsequenz besteht; oder
(2) aus einer Nucleotidsequenz, ausgewählt aus SEQ ID No. 18, 82, 146, 209, 210, 349, 370, 412 und 433, besteht,
wobei optional das isolierte Polynucleotid detektierbar markiert ist, vorzugsweise mit einer Fluoreszenzmarkierung.

## Revendications

1. Procédé pour identifier le cancer du poumon ou son précurseur, ou une prédisposition au cancer du poumon, comprenant:
la détection dans un échantillon test contenant des cellules pulmonaires ou des acides nucléiques provenant de cellules pulmonaires, d'une modification épigénétique de DPYSL4; et
l'identification de l'échantillon test comme contenant des cellules qui sont néoplasiques, précurseurs de néoplasiques, ou prédisposées à la néoplasie, ou comme contenant des acides nucléiques provenant de cellules qui sont néoplasiques, précurseurs de néoplasiques, ou prédisposées à la néoplasie.

2. Procédé selon la revendication 1, comprenant en outre la détection dans l'échantillon test d'une modification épigénétique d'au moins un gène choisi dans le groupe consistant en SULF2, JAM3, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRP1, SGK, SOD3, SOX17, TIMP3, TJP2, TRPV2, UCHL1, WDR69, ZFP42, ZNF442 et ZNF655.

3. Procédé selon la revendication 1 ou 2 où:
(1) l'échantillon test contient des cellules d'épithélium pavimenteux ou des acides nucléiques provenant de cellules d'épithélium pavimenteux;
(2)l'échantillon test contient des cellules d'adénocarcinome ou des acides nucléiques provenant de cellules d'adénocarcinome;
(3) l'échantillon test contient des cellules de carcinome anaplasique à grandes cellules ou des acides nucléiques provenant de cellules de carcinome anaplasique à grandes cellules;
(4)l'échantillon test contient un mélange de cellules d'épithélium pavimenteux, de cellules d'adénocarcinome et de cellules de carcinome anaplasique à grandes cellules; ou
(5) l'échantillon test provient d'un prélèvement choisi dans le groupe consistant en un prélèvement tissulaire, un prélèvement biopsique, un prélèvement chirurgical, un prélèvement cytologique, un prélèvement de crachat, le liquide pleural et un lavage bronchoalvéolaire, où de préférence l'échantillon test provient d'un prélèvement biopsique et un retrait chirurgical de tissu néoplasique est recommandé au patient ou le prélèvement est un prélèvement chirurgical et une chimiothérapie adjuvante ou une radiothérapie adjuvante est recommandée au patient.

4. Procédé selon la revendication 2, où une modification épigénétique dans une série de gènes comprenant deux, trois, quatre ou cinq gènes est détectée, où la détection d'un changement épigénétique dans au moins le gène DPYSL4 dans la série indique une prédisposition à, ou l'incidence du cancer du poumon, de préférence une modification épigénétique de RASSF1A et/ou SOX17 et/ou HS3ST2-nor et/ou NID2 et/ou SFRPl est détectée en outre.

5. Procédé selon la revendication 1 ou 2 où une modification épigénétique est détectée par la détection d'une méthylation d'un motif dinucléotidique CpG dans le gène, et éventuellement
(1)la méthylation est détectée par mise en contact d'au moins une partie du gène avec une endonucléase de restriction sensible à la méthylation, ladite endonucléase clivant préférentiellement les sites de reconnaissance méthylés par rapport aux sites de reconnaissance non méthylés, de sorte que le clivage de la partie du gène indique une méthylation de la partie du gène;
(2)la méthylation est détectée par mise en contact d'au moins une partie du gène avec une endonucléase de restriction sensible à la méthylation, ladite endonucléase clivant préférentiellement les sites de reconnaissance non méthylés par rapport aux sites de reconnaissance méthylés, de sorte que le clivage de la partie du gène indique la non-méthylation de la partie du gène à condition que le gène comprenne un site de reconnaissance pour l'endonucléase de restriction sensible à la méthylation; ou
(3)la mise en contact d'au moins une partie du gène de l'échantillon test avec un réactif chimique qui modifie sélectivement un résidu cytosine non méthylé par rapport à un résidu cytosine méthylé, ou modifie sélectivement un résidu cytosine méthylé par rapport à un résidu cytosine non méthylé; et la détection d'un produit généré du fait de ladite mise en contact, et de préférence
(a) l'étape de détection d'un produit emploie une amplification avec au moins une amorce qui s'hybride à une séquence comprenant un motif dinucléotidique CpG non méthylé modifié mais pas à une séquence comprenant un motif dinucléotidique CpG méthylé non modifié en formant ainsi des produits d'amplification;
(b) l'étape de détection d'un produit comprend l'amplification avec au moins une amorce qui s'hybride à une séquence comprenant un motif dinucléotidique CpG méthylé non modifié mais pas à une séquence comprenant un motif dinucléotidique CpG non méthylé modifié en formant ainsi
des produits d'amplification;
(c) le produit est détecté par un procédé choisi dans le groupe consistant en l'électrophorèse, l'hybridation, l'amplification, le séquençage, la réaction en chaîne par ligase, la chromatographie, la spectrométrie de masse et leurs combinaisons ;
(d) le réactif chimique est l'hydrazine, où de préférence le procédé comprend en outre le clivage de la partie du gène mise en contact avec l'hydrazine avec la pipéridine; ou
(e) le réactif chimique comprend des ions bisulfite, où de préférence le procédé comprend en outre le traitement de la partie du gène mise en contact avec des ions bisulfite avec un alcali.

6. Procédé selon la revendication 1 ou 2 où une modification épigénétique est détectée par
(1) la détection de la méthylation d'un motif dinucléotidique CpG dans un promoteur, intron ou exon du gène;
(2) la détection de l'expression diminuée de l'ARNm du gène;
(3) la détection de l'hypométhylation d'un motif dinucléotidique CpG dans le gène;
(4) la détection de l'hypométhylation d'un motif dinucléotidique CpG dans un promoteur du gène; ou
(5) la détection de l'expression accrue de l'ARNm du gène.

7. Procédé selon la revendication 1 où
(1) l'étape de détection emploie l'amplification d'au moins une partie du gène DPYSL4 gène au moyen d'une amorce oligonucléotidique qui s'hybride spécifiquement dans des conditions d'amplification à une région du gène DPYSL4, où la région est dans les limites d'environ 3kb du site d'initiation de la transcription de DPYSL4;
(2) l'étape de détection emploie l'amplification d'au moins une partie du gène DPYSL4 au moyen d'au moins une paire d'amorces oligonucléotidiques qui s'hybride spécifiquement dans des conditions d'amplification à une région du gène DPYSL4 ; où la région est dans les limites d'environ 3 kb du site d'initiation de la transcription de DPYSL4, où éventuellement une sonde oligonucléotidique est liée de manière covalente à l'amorce oligonucléotidique, et où il est préféré en outre que la région comprenne, consiste essentiellement en ou consiste en les séquences représentées par SEQ ID NO. 145 et/ou SEQ ID NO. 146 et/ou SEQ ID NO. 209 et/ou SEQ ID NO. 210 et/ou SEQ ID NO. 412 et/ou SEQ ID NO. 433;
(3) l'étape de détection d'un produit comprend l'amplification avec au moins une amorce sens comprenant, consistant essentiellement en ou consistant en SEQ ID NO. 17-18 et/ou SEQ ID NO. 349;
(4) l'étape de détection d'un produit comprend l'amplification avec au moins une amorce antisens comprenant, consistant essentiellement en ou consistant en SEQ ID NO. 81-82 et/ou SEQ ID NO. 370;
(5) l'étape de détection emploie l'amplification d'au moins une partie du gène DPYSL4, et emploie en outre au moins une sonde oligonucléotidique qui s'hybride à un amplicon choisi dans le groupe consistant en SEQ ID NO. 145 et/ou SEQ ID NO. 146 et/ou SEQ ID NO. 209 et/ou SEQ ID NO. 210 et/ou SEQ ID NO. 412 et/ou SEQ ID NO. 433 dans des conditions d'amplification, où de préférence la sonde comprend, consiste essentiellement en ou consiste en les séquences représentées par SEQ ID NO. 391; ou
(6) l'étape de détection emploie l'amplification d'au moins une partie du gène DPYSL4 et un réactif détectable qui se lie préférentiellement à l'ADN double brin par rapport à l'ADN simple brin.

8. Procédé selon la revendication 2 où
(1) l'étape de détection emploie l'amplification d'au moins une partie du au moins un gène au moyen d'une amorce oligonucléotidique qui s'hybride spécifiquement dans des conditions d'amplification à une région d'un gène choisi dans le groupe consistant en SULF2, JAM3, APC2, BMP7, ACSL6, ALS2CL, ARTS-1, BEX1, BNTP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRPl, SGK, SOD3, SOX17, TTMP3, TJP2, TRPV2, UCHL1, WDR69, ZFP42, ZNF442 et ZNF655; où la région est dans les limites d'environ 3 kb du site d'initiation de la transcription dudit gène;
(2) l'étape de détection emploie l'amplification d'au moins une partie du au moins un gène au moyen d'au moins une paire d'amorces oligonucléotidiques qui s'hybride spécifiquement dans des conditions d'amplification à une région d'un gène choisi dans le groupe consistant en SULF2, JAM3, APC2, BMP7, ACSL6, ALS2CL, ARTS-1, BEX1, BNIP3, CBR3, CD248, CD44, CHD5, DLK1, DSC2, EDNRB, EPB41 L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, N1D2, PCDHB15, PCDHGA12, PFKP, PGRMC1, PHACTR3, PHKA2, POMC, PRKCA, PSEN1, RASSF1 A, RASSF2, RBP1, RRAD, SFRPI, SGK, SOD3, SOX17, TTMP3, TJP2, TRPV2, UCHL1, WDR69, ZFP42, ZNF442 et ZNF655; où la région est dans les limites d'environ 3 kb du site d'initiation de la transcription dudit gène, où éventuellement une sonde oligonucléotidique est liée de manière covalente à l'amorce oligonucléotidique, et où il est préféré en outre que la région comprenne, consiste essentiellement en ou consiste en les séquences représentées par SEQ ID NO. 129-144 et/ou SEQ ID NO. 147 - 208 et/ou SEQ ID NO. 211-256 et/ou SEQ ID NO. 315-329 et/ou SEQ ID NO. 330-344 et/ou SEQ ID NO. 408-411 et/ou SEQ ID NO. 413-428 et/ou SEQ ID NO. 429-432 et/ou SEQ ID NO. 434-449 et/ou SEQ ID NO. 271-277 et/ou SEQ ID NO. 278-284;
(3) l'étape de détection d'un produit comprend l'amplification avec au moins une amorce sens comprenant, consistant essentiellement en ou consistant en SEQ ID NO. 1-16 et/ou SEQ ID NO. 19-64 et/ou SEQ ID NO. 285-299 et/ou SEQ ID NO. 345-348 et/ou SEQ ID NO. 350-365 et/ou SEQ ID NO. 257-263;
(4) l'étape de détection d'un produit comprend l'amplification avec au moins une amorce antisens comprenant, consistant essentiellement en ou consistant en SEQ ID NO. 65-80 et/ou SEQ ID NO. 83-128 et/ou SEQ ID NO. 300-314 et/ou SEQ ID NO. 366-369 et/ou SEQ ID NO. 371-386 et/ou SEQ ID NO. 264-270;
(5) l'étape de détection emploie l'amplification d'au moins une partie du au moins un gène, et emploie en outre au moins une sonde oligonucléotidique qui s'hybride à un amplicon choisi dans le groupe consistant en SEQ ID NO: 129-144 et/ou SEQ ID NO. 147 - 208 et/ou SEQ ID NO. 211-256 et/ou SEQ ID NO. 315-329 et/ou SEQ ID NO. 330-344 et/ou SEQ ID NO. 408-411 et/ou SEQ ID NO. 413-428 et/ou SEQ ID NO. 429-432 et/ou SEQ ID NO. 434-449 et/ou SEQ ID NO. 271-277 et/ou SEQ ID NO. 278-284 dans des conditions d'amplification, où de préférence la sonde comprend, consiste essentiellement en ou consiste en les séquences représentées par SEQ ID NO. 387-390 et/ou SEQ ID NO. 392-407; ou
(6) l'étape de détection emploie l'amplification d'au moins une partie du au moins un gène et un réactif détectable qui se lie préférentiellement à l'ADN double brin par rapport à l'ADN simple brin.

9. Kit pour évaluer le cancer du poumon ou son précurseur, ou une prédisposition au cancer du poumon dans un échantillon test contenant des cellules pulmonaires ou des acides nucléiques provenant de cellules pulmonaires, ledit kit comprenant dans un emballage
un réactif qui (a) modifie les résidus cytosine méthylés mais pas les résidus cytosine non méthylés, ou qui (b) modifie les résidus cytosine non méthylés mais pas les résidus cytosine méthylés; et
au moins une paire d'amorces oligonucléotidiques qui s'hybride spécifiquement dans des conditions d'amplification à une région d'un gène DPYSL4; où la région est dans les limites d'environ 3 kb du site d'initiation de la transcription de DPYSL4.

10. Kit selon la revendication 9 où
(1)la au moins une paire d'amorces oligonucléotidiques est choisie parmi SEQ ID NO. 17, 18, 81, 82, 349 et 370; ou
(2)la au moins une paire d'amorces oligonucléotidiques amplifie un amplicon choisi parmi SEQ ID No. 145, 146, 209, 210, 412 et 433.

11. Kit selon la revendication 9 ou 10, comprenant en outre au moins une paire d'amorces oligonucléotidiques qui s'hybride spécifiquement dans des conditions d'amplification à une région d'un gène choisi dans le groupe consistant en SULF2, JAM3, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRPl, SGK, SOD3, SOX17, TIMP3, TJP2, TRPV2, UCHL1, WDR69, ZFP42, ZNF442 et ZNF655; où la région est dans les limites d'environ 3 kb du site d'initiation de la transcription dudit gène; et éventuellement
(1) la au moins une paire d'amorces est choisie dans le tableau 1 (SEQ ID NO: 1-16, 19-80 et 83-128), la figure 2 (SEQ ID NO: 257-270), le tableau 3 (SEQ ID NO: 285-314) et le tableau 7 (SEQ ID NO: 345-348, 350-369 et 371-386); ou
(2)la au moins une paire d'amorces oligonucléotidiques amplifie un amplicon choisi dans le tableau 2 (SEQ ID NO: 129-144 et 147 - 208 et 211-256), la figure 2 (SEQ ID NO: 271-284), le tableau 4 (SEQ ID NO: 315-344) et le tableau 8 (SEQ ID NO: 408-411, 413-432 et 434-449).

12. Kit pour évaluer le cancer du poumon ou son précurseur, ou une prédisposition au cancer du poumon dans un échantillon test contenant des cellules pulmonaires ou des acides nucléiques provenant de cellules pulmonaires, ledit kit comprenant dans un emballage:
au moins deux paires d'amorces oligonucléotidiques qui s'hybrident spécifiquement dans des conditions d'amplification à une région d'un gène DPYSL4, où la région est dans les limites d'environ 3kb du site d'initiation de la transcription de DPYSL4.

13. Kit selon la revendication 12, où
(1) les au moins deux paires d'amorces sont choisies parmi SEQ ID NO: 17, 18, 81, 82, 349 et 370; ou
(2) les au moins deux paires d'amorces oligonucléotidiques amplifient des amplicons choisis parmi SEQ ID NO: 145, 146, 209, 210, 412 et 433.

14. Kit selon la revendication 12 ou 13 comprenant en outre au moins deux paires d'amorces oligonucléotidiques qui s'hybrident spécifiquement dans des conditions d'amplification à une région d'un gène choisi dans le groupe consistant en, SULF2, JAM3, APC2, BMP7, ACSL6, ALS2CL, ARTS-1, BEX1, BNIP3, CBR3, CD248, CD44, CHD5, DLK1, DSC2, EDNRB, EPB41L3, EPHB6, ERBB3, FBLN2, FBN2, FOXL2, GNAS, GSTP1, HS3ST2, HPN, IGFBP7, IRF7, LOX, LY6D, LY6K, MACF1, MCAM, NCBP1, NEFH, NID2, PCDHB15, PCDHGA12, PFKP, PGRMC1, PHACTR3, PHKA2, POMC, PRKCA, PSEN1, RASSF1A, RASSF2, RBP1, RRAD, SFRPl, SGK, SOD3, SOX17, TIMP3, TJP2, TRPV2, UCHL1, WDR69, ZFP42, ZNF442 et ZNF655; où la région est dans les limites d'environ 3 kb du site d'initiation de la transcription dudit gène; et éventuellement
(1) les au moins deux paires d'amorces sont choisies parmi SEQ ID NO: 1-16, 19-80 et 83-128 (tableau 1), SEQ ID NO: 257-270 (figure 2), SEQ ID NO:285-314 (tableau 3), SEQ ID NO: 345-348, 350-369 et 371-386 (tableau 7); ou
(2) les au moins deux paires d'amorces oligonucléotidiques amplifient des amplicons choisis dans le tableau 2 (SEQ ID NO: 129-144, 147-208 et 211 - 256), la figure 2 (SEQ ID NO: 271-284), le tableau 4 (SEQ ED NO: 315-344) et le tableau 8 (SEQ ID NO: 408-411, 413-432 et 434-449).

15. Kit selon la revendication 9 ou 12 comprenant en outre au moins une sonde oligonucléotidique qui s'hybride à un amplicon choisi dans le groupe consistant en SEQ ID NO. 146, 146, 209, 210,412 et 433 dans des conditions d'amplification, où de préférence la sonde oligonucléotidique est SEQ ID NO. 391 et de préférence encore la sonde oligonucléotidique comprend un marqueur fluorescent, un agent d'extinction de fluorescence, ou un marqueur fluorescent et un agent d'extinction de fluorescence.

16. Kit selon la revendication 11 ou 14 comprenant en outre au moins une sonde oligonucléotidique qui s'hybride à un amplicon choisi dans le groupe consistant en le tableau 2 (SEQ ID NO: 129-144, 147-208 et 211-256), la figure 2 (SEQ ID NO: 271-284), le tableau 4 (SEQ ID NO: 315-344) et le tableau 8 (SEQ ID NO: 408-411, 413-432 et 434-449) dans des conditions d'amplification,
où de préférence la sonde oligonucléotidique est choisie dans le groupe consistant en SEQ ID NO: 387-390 et 392-407, et de préférence encore la sonde oligonucléotidique comprend un marqueur fluorescent, un agent d'extinction de fluorescence, ou un marqueur fluorescent et un agent d'extinction de fluorescence.

17. Kit selon la revendication 9, 11, 12 ou 14 qui comprend:
(1) un réactif détectable qui se lie préférentiellement à l'ADN double brin par rapport à l'ADN simple brin;
(2) une ADN polymérase for amplifier l'ADN; et/ou
(3) au moins une sonde oligonucléotidique qui est liée de manière covalente à au moins l'une desdites amorces oligonucléotidiques.

18. Polynucléotide isolé qui:
(1) comprend une séquence nucléotidique choisie dans le groupe consistant en SEQ ID NO: 17, 81, 145 et 391, où de préférence le polynucléotide isolé consiste en la séquence nucléotidique choisie;
ou
(2) consiste en une séquence nucléotidique choisie parmi SEQ ID NO. 18, 82, 146, 209, 210, 349, 370, 412 et 433 où éventuellement le polynucléotide isolé est marqué de manière détectable, de préférence avec un marqueur fluorescent.


### SEQUENCE LISTING

<110> BAYLIN, Stephen HERMAN, James SCHUEBEL, Kornel COPE, Leslie VAN CRIEKINGE, Wim STRAUB, Josef TROOSKENS, Geert VAN NESTE, Leander
<120> DETECTION AND PROGNOSIS OF LUNG CANCER
<130> 006791.00038
<150> 61/029693
   <151> 2008-02-19
<160> 449
<170> FastSEQ for windows version 4.0
<210> 1
   <211> 22
   <212> DNA
   <213> Homo sapiens
<400> 1
   tttaatgtta cgttttggcg tt 22
<210> 2
   <211> 21
   <212> DNA
   <213> Homo sapiens
<400> 2
   gcggttgtaa ggtttttggt c 21
<210> 3
   <211> 21
   <212> DNA
   <213> Homo sapiens
<400> 3
   ggacgggtgt ttgcgtttta c 21
<210> 4
   <211> 22
   <212> DNA
   <213> Homo sapiens
<400> 4
   gtcgtttgtt taggttcgga tc 22
<210> 5
   <211> 20
   <212> DNA
   <213> Homo sapiens
<400> 5
   tcggggtttt tatttggttc 20
<210> 6
   <211> 20
   <212> DNA
   <213> Homo sapiens
<400> 6
   gtacgtgcgt ttattgcgag 20
<210> 7
   <211> 20
   <212> DNA
   <213> Homo sapiens
<400> 7
   ggtatcggtt tggttatcgc 20
<210> 8
   <211> 25
   <212> DNA
   <213> Homo sapiens
<400> 8
   gttttcgatt gatttattaa ggttc 25
<210> 9
   <211> 19
   <212> DNA
   <213> Homo sapiens
<400> 9
   tcgtgggaag agagcgtag 19
<210> 10
   <211> 25
   <212> DNA
   <213> Homo sapiens
<400> 10
   ttttgttaag agttgtcgtt agttc 25
<210> 11
   <211> 20
   <212> DNA
   <213> Homo sapiens
<400> 11
   ggggtagtcg ttaattgcgt 20
<210> 12
   <211> 22
   <212> DNA
   <213> Homo sapiens
<400> 12
   cgggagaaga aagttagtgc gt 22
<210> 13
   <211> 17
   <212> DNA
   <213> Homo sapiens
<400> 13
   gagcgttcgg gttttgc 17
<210> 14
   <211> 25
   <212> DNA
   <213> Homo sapiens
<400> 14
   ttgggaattt agttgtcgtc gtttc 25
<210> 15
   <211> 20
   <212> DNA
   <213> Homo sapiens
<400> 15
   gaggtttgcg gtttaggttc 20
<210> 16
   <211> 20
   <212> DNA
   <213> Homo sapiens
<400> 16
   ggagttgggg tttacgagac 20
<210> 17
   <211> 25
   <212> DNA
   <213> Homo sapiens
<400> 17
   ggtgttttga tagaagtcgt tagtc 25
<210> 18
   <211> 20
   <212> DNA
   <213> Homo sapiens
<400> 18
   ggggttatag tttggcgttc 20
<210> 19
   <211> 21
   <212> DNA
   <213> Homo sapiens
<400> 19
   ggtttcggtt tcgttttgtt c 21
<210> 20
   <211> 19
   <212> DNA
   <213> Homo sapiens
<400> 20
   gggatagtgg ggttgacgc 19
<210> 21
   <211> 19
   <212> DNA
   <213> Homo sapiens
<400> 21
   gcgtgggttt tcgtcgtag 19
<210> 22
   <211> 24
   <212> DNA
   <213> Homo sapiens
<400> 22
   gggtgttcga tttaagtcga gttc 24
<210> 23
   <211> 22
   <212> DNA
   <213> Homo sapiens
<400> 23
   gtttagttaa gttcggttcg gg 22
<210> 24
   <211> 24
   <212> DNA
   <213> Homo sapiens
<400> 24
   agggagttta gttaagttcg gttc 24
<210> 25
   <211> 19
   <212> DNA
   <213> Homo sapiens
<400> 25
   tagagcggag gaagttgcg 19
<210> 26
   <211> 23
   <212> DNA
   <213> Homo sapiens
<400> 26
   tcggagtttt atagggtaac gaa 23
<210> 27
   <211> 21
   <212> DNA
   <213> Homo sapiens
<400> 27
   ttggagattt cgatagagcg t 21
<210> 28
   <211> 26
   <212> DNA
   <213> Homo sapiens
<400> 28
   gcgataggtt tttagtaagt aagcgc 26
<210> 29
   <211> 20
   <212> DNA
   <213> Homo sapiens
<400> 29
   ttcggggtgt agcggtcgtc 20
<210> 30
   <211> 20
   <212> DNA
   <213> Homo sapiens
<400> 30
   acgtaagagt ttgggagcgt 20
<210> 31
   <211> 19
   <212> DNA
   <213> Homo sapiens
<400> 31
   gtttcggggt tcgtttttc 19
<210> 32
   <211> 21
   <212> DNA
   <213> Homo sapiens
<400> 32
   tttgtcggcg tcgttatttt c 21
<210> 33
   <211> 21
   <212> DNA
   <213> Homo sapiens
<400> 33
   cgtttatggg tcggttacgt c 21
<210> 34
   <211> 19
   <212> DNA
   <213> Homo sapiens
<400> 34
   agttgagaat cggacgggg 19
<210> 35
   <211> 20
   <212> DNA
   <213> Homo sapiens
<400> 35
   gggattataa gtcgcgtcgc 20
<210> 36
   <211> 22
   <212> DNA
   <213> Homo sapiens
<400> 36
   gcgcgtagag ttgtaaaggt tc 22
<210> 37
   <211> 21
   <212> DNA
   <213> Homo sapiens
<400> 37
   ggtagaggcg aggagttgtt c 21
<210> 38
   <211> 21
   <212> DNA
   <213> Homo sapiens
<400> 38
   gatgtcgttt gggagtagtg c 21
<210> 39
   <211> 27
   <212> DNA
   <213> Homo sapiens
<400> 39
   gcggggtttt ttttatcggt tagattc 27
<210> 40
   <211> 23
   <212> DNA
   <213> Homo sapiens
<400> 40
   gttttcgttg tcgttacggg ttc 23
<210> 41
   <211> 23
   <212> DNA
   <213> Homo sapiens
<400> 41
   agaatttagg tcggttttta tcg 23
<210> 42
   <211> 19
   <212> DNA
   <213> Homo sapiens
<400> 42
   gtcggatgaa gtattcggg 19
<210> 43
   <211> 24
   <212> DNA
   <213> Homo sapiens
<400> 43
   ttatttcgtt tttagggagt tttc 24
<210> 44
   <211> 21
   <212> DNA
   <213> Homo sapiens
<400> 44
   tttcgtgtgg gaagagttcg t 21
<210> 45
   <211> 25
   <212> DNA
   <213> Homo sapiens
<400> 45
   ttttggttat taggtagttc ggttc 25
<210> 46
   <211> 19
   <212> DNA
   <213> Homo sapiens
<400> 46
   ttattttgcg agcggtttc 19
<210> 47
   <211> 25
   <212> DNA
   <213> Homo sapiens
<400> 47
   gatttgggcg tttttggttt ttcgc 25
<210> 48
   <211> 20
   <212> DNA
   <213> Homo sapiens
<400> 48
   gggcgttgag gtagaagaac 20
<210> 49
   <211> 20
   <212> DNA
   <213> Homo sapiens
<400> 49
   ttaggtcgga ggtttcgttt 20
<210> 50
   <211> 20
   <212> DNA
   <213> Homo sapiens
<400> 50
   gatgtttcgg tcgaggtttc 20
<210> 51
   <211> 26
   <212> DNA
   <213> Homo sapiens
<400> 51
   tgtagttttc ggagttagtg tcgcgc 26
<210> 52
   <211> 21
   <212> DNA
   <213> Homo sapiens
<400> 52
   ttttgttcgt cgtattttcg g 21
<210> 53
   <211> 22
   <212> DNA
   <213> Homo sapiens
<400> 53
   agtatagagt ggggagcgta gc 22
<210> 54
   <211> 21
   <212> DNA
   <213> Homo sapiens
<400> 54
   ttgcgttagt cgtttgcgtt c 21
<210> 55
   <211> 22
   <212> DNA
   <213> Homo sapiens
<400> 55
   gttagtcgag ttcggaggta tc 22
<210> 56
   <211> 21
   <212> DNA
   <213> Homo sapiens
<400> 56
   gcgtcggagg ttaaggttgt t 21
<210> 57
   <211> 21
   <212> DNA
   <213> Homo sapiens
<400> 57
   cgggttagag tattgttcgg t 21
<210> 58
   <211> 21
   <212> DNA
   <213> Homo sapiens
<400> 58
   gattttatcg gggaaatatc g 21
<210> 59
   <211> 25
   <212> DNA
   <213> Homo sapiens
<400> 59
   ttatttcgta ggttgaggtt agggc 25
<210> 60
   <211> 22
   <212> DNA
   <213> Homo sapiens
<400> 60
   gttgtatttt cgcggagcgt tc 22
<210> 61
   <211> 24
   <212> DNA
   <213> Homo sapiens
<400> 61
   gtttaggttg tggtttaggt cgtc 24
<210> 62
   <211> 26
   <212> DNA
   <213> Homo sapiens
<400> 62
   ggggttttta ggtattcggt tcgtac 26
<210> 63
   <211> 24
   <212> DNA
   <213> Homo sapiens
<400> 63
   tcggttttta gttttttcgg tcgc 24
<210> 64
   <211> 21
   <212> DNA
   <213> Homo sapiens
<400> 64
   ttatcgagaa gcgtcggttt c 21
<210> 65
   <211> 19
   <212> DNA
   <213> Homo sapiens
<400> 65
   gaaccaaccc tctccgacc 19
<210> 66
   <211> 19
   <212> DNA
   <213> Homo sapiens
<400> 66
   atttttccgc aacctctcg 19
<210> 67
   <211> 27
   <212> DNA
   <213> Homo sapiens
<400> 67
   cgaaaccaaa aaactaaacg aaaaccg 27
<210> 68
   <211> 22
   <212> DNA
   <213> Homo sapiens
<400> 68
   gacccgaaat aacctcgaaa cg 22
<210> 69
   <211> 21
   <212> DNA
   <213> Homo sapiens
<400> 69
   aatcgtcact cgtatctcgc t 21
<210> 70
   <211> 23
   <212> DNA
   <213> Homo sapiens
<400> 70
   cgttatccaa actaaaatcg acc 23
<210> 71
   <211> 22
   <212> DNA
   <213> Homo sapiens
<400> 71
   cgcctacaac tactacacga cc 22
<210> 72
   <211> 22
   <212> DNA
   <213> Homo sapiens
<400> 72
   tcaaaatccg aactctaaac cg 22
<210> 73
   <211> 22
   <212> DNA
   <213> Homo sapiens
<400> 73
   ttactaacct aaacgaccgc aa 22
<210> 74
   <211> 21
   <212> DNA
   <213> Homo sapiens
<400> 74
   aatataaacc ctacgaccgc c 21
<210> 75
   <211> 19
   <212> DNA
   <213> Homo sapiens
<400> 75
   tcttccccga aaaccgcta 19
<210> 76
   <211> 24
   <212> DNA
   <213> Homo sapiens
<400> 76
   aaatcgaaaa acctaaaata tcgc 24
<210> 77
   <211> 22
   <212> DNA
   <213> Homo sapiens
<400> 77
   cgacctcgac gaaaaaataa cg 22
<210> 78
   <211> 25
   <212> DNA
   <213> Homo sapiens
<400> 78
   aaacaacgac taccgatact acgcg 25
<210> 79
   <211> 22
   <212> DNA
   <213> Homo sapiens
<400> 79
   ctcacactat acaacacgcg ac 22
<210> 80
   <211> 21
   <212> DNA
   <213> Homo sapiens
<400> 80
   ataataaatt ccccgacgac c 21
<210> 81
   <211> 19
   <212> DNA
   <213> Homo sapiens
<400> 81
   aaaaccatta acgcccacg 19
<210> 82
   <211> 21
   <212> DNA
   <213> Homo sapiens
<400> 82
   gctctaaaaa ccacacccgt c 21
<210> 83
   <211> 21
   <212> DNA
   <213> Homo sapiens
<400> 83
   ctctacgact caaacctcgc t 21
<210> 84
   <211> 20
   <212> DNA
   <213> Homo sapiens
<400> 84
   ataaaaatcc cgacgaacga 20
<210> 85
   <211> 19
   <212> DNA
   <213> Homo sapiens
<400> 85
   cccaaaacta ctcgccgct 19
<210> 86
   <211> 24
   <212> DNA
   <213> Homo sapiens
<400> 86
   cgcgaatctt aaccgaaaaa atcg 24
<210> 87
   <211> 23
   <212> DNA
   <213> Homo sapiens
<400> 87
   gattacaatt tacaacctcc gct 23
<210> 88
   <211> 19
   <212> DNA
   <213> Homo sapiens
<400> 88
   tacaacctcc gctaccgtc 19
<210> 89
   <211> 22
   <212> DNA
   <213> Homo sapiens
<400> 89
   caaatacgaa cacaaaaacc ga 22
<210> 90
   <211> 19
   <212> DNA
   <213> Homo sapiens
<400> 90
   ctcttactaa ccgcacgcc 19
<210> 91
   <211> 21
   <212> DNA
   <213> Homo sapiens
<400> 91
   aaactaccga ctacacctcc g 21
<210> 92
   <211> 24
   <212> DNA
   <213> Homo sapiens
<400> 92
   ctctccgctc caaacgctaa cgcg 24
<210> 93
   <211> 22
   <212> DNA
   <213> Homo sapiens
<400> 93
   gccccaatac taaatcacga cg 22
<210> 94
   <211> 21
   <212> DNA
   <213> Homo sapiens
<400> 94
   gactcctcga aaaacaaacg a 21
<210> 95
   <211> 20
   <212> DNA
   <213> Homo sapiens
<400> 95
   cgactcgctc tatctcgcac 20
<210> 96
   <211> 24
   <212> DNA
   <213> Homo sapiens
<400> 96
   aaactaccta ctaaacgaaa cccg 24
<210> 97
   <211> 21
   <212> DNA
   <213> Homo sapiens
<400> 97
   ataaaaacac gaaaaccccg c 21
<210> 98
   <211> 20
   <212> DNA
   <213> Homo sapiens
<400> 98
   aacgaatcaa actcccgaaa 20
<210> 99
   <211> 20
   <212> DNA
   <213> Homo sapiens
<400> 99
   cgaacgcaaa accgaaatcg 20
<210> 100
   <211> 19
   <212> DNA
   <213> Homo sapiens
<400> 100
   acgtcctcct cgaacgaaa 19
<210> 101
   <211> 22
   <212> DNA
   <213> Homo sapiens
<400> 101
   tacacaaacc gttctaaccc ga 22
<210> 102
   <211> 23
   <212> DNA
   <213> Homo sapiens
<400> 102
   acaaaatacc gctaactaac gaa 23
<210> 103
   <211> 27
   <212> DNA
   <213> Homo sapiens
<400> 103 27
   caacgatacc caaaaaaaat caacgcg 27
<210> 104
   <211> 17
   <212> DNA
   <213> Homo sapiens
<400> 104
   gcgcaacgaa caaaacg 17
<210> 105
   <211> 22
   <212> DNA
   <213> Homo sapiens
<400> 105 22
   acgcaaaatt cttctcccaa aa 22
<210> 106
   <211> 20
   <212> DNA
   <213> Homo sapiens
<400> 106
   ccctacaaac gacgacgaac 20
<210> 107
   <211> 21
   <212> DNA
   <213> Homo sapiens
<400> 107
   cttacgaacc atttaatccc g 21
<210> 108
   <211> 21
   <212> DNA
   <213> Homo sapiens
<400> 108
   cgaataaccg aacgaccgat a 21
<210> 109
   <211> 22
   <212> DNA
   <213> Homo sapiens
<400> 109
   cactcttcgt actattcccg ct 22
<210> 110
   <211> 23
   <212> DNA
   <213> Homo sapiens
<400> 110
   gaatactcta attccacgcg act 23
<210> 111
   <211> 20
   <212> DNA
   <213> Homo sapiens
<400> 111
   gacttctcat accgcaatcg 20
<210> 112
   <211> 22
   <212> DNA
   <213> Homo sapiens
<400> 112
   cgacacctac caaataaaat cg 22
<210> 113
   <211> 20
   <212> DNA
   <213> Homo sapiens
<400> 113
   aaaccctcac cgttatcgtc 20
<210> 114
   <211> 24
   <212> DNA
   <213> Homo sapiens
<400> 114
   aaacgactac aaataaatac gcca 24
<210> 115
   <211> 25
   <212> DNA
   <213> Homo sapiens
<400> 115
   cctacgatcg aaaacgacgc gaacg 25
<210> 116
   <211> 22
   <212> DNA
   <213> Homo sapiens
<400> 116 22
   ataacgaccc tcgacctacg at 22
<210> 117
   <211> 20
   <212> DNA
   <213> Homo sapiens
<400> 117
   ctttcctacc accgaaacga 20
<210> 118
   <211> 24
   <212> DNA
   <213> Homo sapiens
<400> 118
   caaaaacgaa tcccgtatcc gacg 24
<210> 119
   <211> 24
   <212> DNA
   <213> Homo sapiens
<400> 119
   caactccgaa cgaaacaata aacg 24
<210> 120
   <211> 22
   <212> DNA
   <213> Homo sapiens
<400> 120
   ctctccaaaa ttaccgtacg cg 22
<210> 121
   <211> 19
   <212> DNA
   <213> Homo sapiens
<400> 121
   gaacacaaat cccgcgtaa 19
<210> 122
   <211> 19
   <212> DNA
   <213> Homo sapiens
<400> 122 19
   aaacaaatcc cgctccgaa 19
<210> 123
   <211> 21
   <212> DNA
   <213> Homo sapiens
<400> 123 21
   tcctctacta tcaacgccga c 21
<210> 124
   <211> 22
   <212> DNA
   <213> Homo sapiens
<400> 124
   ctcacaatac gtctaaccga cg 22
<210> 125
   <211> 25
   <212> DNA
   <213> Homo sapiens
<400> 125
   acacctcgta tcctcactaa aaacg 25
<210> 126
   <211> 22
   <212> DNA
   <213> Homo sapiens
<400> 126
   aatacgcaat acccgacgac cg 22
<210> 127
   <211> 29
   <212> DNA
   <213> Homo sapiens
<400> 127
   caattactac gcaaaaacga aacaaaacg 29
<210> 128
   <211> 26
   <212> DNA
   <213> Homo sapiens
<400> 128
   accgaaaaaa aaaacgaacc taaccg 26
<210> 129
   <211> 106
   <212> DNA
   <213> Homo sapiens
<400> 129
   tttaatgtta cgttttggcg ttcgtcgttc gtgttttttt ttttagtcgg ttttcgtaga 60
   atgttaggta ttgacgttgg agagcggggt cggagagggt tggttc 106
<210> 130
   <211> 101
   <212> DNA
   <213> Homo sapiens
<400> 130
   gcggttgtaa ggtttttggt cggtgagtga attagtaggt aaggatggta gttagggtat 60
   ttatatttac gagggtggtg gtcgagaggt tgcggaaaaa t 101
<210> 131
   <211> 91
   <212> DNA
   <213> Homo sapiens
<400> 131
   ggacgggtgt ttgcgtttta cgtttagttc gtttaggtgg gggttttcgt tttttcggtt 60
   gttgcggttt tcgtttagtt ttttggtttc g 91
<210> 132
   <211> 99
   <212> DNA
   <213> Homo sapiens
<400> 132
   gtcgtttgtt taggttcgga tcgggttttg ttcgtttcgg agtttttgtt cgcgtcgcgg 60
   agatttcgga gttcgcgcgt ttcgaggtta tttcgggtc                        99
<210> 133
   <211> 96
   <212> DNA
   <213> Homo sapiens
<400> 133
   tcggggtttt tatttggttc gttttttttc gggtcggatg ttagttcgtc gagcgtaggg 60
   tagcggggag ttggtagcga gatacgagtg acgatt                           96
<210> 134
   <211> 104
   <212> DNA
   <213> Homo sapiens
<400> 134
   gtacgtgcgt ttattgcgag ttgcggcgtc gtatagtttc gtggcgtttt gggtattttt 60
   gtttttgttg cgtttcgttt tggtcgattt tagtttggat aacg                 104
<210> 135
   <211> 111
   <212> DNA
   <213> Homo sapiens
<400> 135
   ggtatcggtt tggttatcgc gcgcgaattg tgtcgatagt tttttgggga tgtggtgttt 60
   atcgcgcggg acgtggcgcg gggttaggcg gtcgtgtagt agttgtaggc g         111
<210> 136
   <211> 80
   <212> DNA
   <213> Homo sapiens
<400> 136
   gttttcgatt gatttattaa ggttcgattt ggtttcggat atttcgtaga ttatttcgcg 60
   gtttagagtt cggattttga                                             80
<210> 137
   <211> 105
   <212> DNA
   <213> Homo sapiens
<400> 137
   tcgtgggaag agagcgtagt agttgttggg gtcgtaggcg gtacggggtt tagtagttta 60
   ggggttttgg tttagtgtgg gttttgcggt cgtttaggtt agtaa 105
<210> 138
   <211> 100
   <212> DNA
   <213> Homo sapiens
<400> 138
   ttttgttaag agttgtcgtt agttcggggt cggattagtt cgggggtatc gcgatgttgt 60
   tgcgtttgtt gttggtttgg gcggtcgtag ggtttatatt 100
<210> 139
   <211> 95
   <212> DNA
   <213> Homo sapiens
<400> 139
   ggggtagtcg ttaattgcgt tttttttttt ttttcgtttt taattttaga gttttttatt 60
   ttattgtttt ttgttttagc ggttttcggg gaaga 95
<210> 140
   <211> 90
   <212> DNA
   <213> Homo sapiens
<400> 140
   cgggagaaga aagttagtgc gtttttgggc gtaggggtta gtggggttcg gaggtatagg 60
   tatttcgcga tattttaggt ttttcgattt 90
<210> 141
   <211> 120
   <212> DNA
   <213> Homo sapiens
<400> 141
   gagcgttcgg gttttgcggg gagtaggtta aggcggtcga gagaaagggg ggtcgagacg 60
   ggggggtgga ggtttggggg ggtggggggg taggcggtcg ttattttttc gtcgaggtcg 120
<210> 142
   <211> 100
   <212> DNA
   <213> Homo sapiens
<400> 142
   ttgggaattt agttgtcgtc gtttcgtaga gttttttgtt ttcggagggc gtttattttc 60
   gggtcgttta ttattcgcgt agtatcggta gtcgttgttt 100
<210> 143
   <211> 85
   <212> DNA
   <213> Homo sapiens
<400> 143
   gaggtttgcg gtttaggttc gatttttgcg atttgtttta ggtaggtttg tatgtgcgcg 60
   gcggtcgcgt gttgtatagt gtgag                                       85
<210> 144
   <211> 106
   <212> DNA
   <213> Homo sapiens
<400> 144
   ggagttgggg tttacgagac ggggcgtgcg gggtatcggg cggtcggcgg ggagtcgtag 60
   gtttttttag agggggcgcg agtcgggtcg tcggggaatt tattat               106
<210> 145
   <211> 114
   <212> DNA
   <213> Homo sapiens
<400> 145
   ggtgttttga tagaagtcgt tagtcggtgt tatgtttagg ataggtattt gtagttttgt 60
   gtggacgtta acgttattag gaaggattat taggtcgtgg gcgttaatgg tttt       114
<210> 146
   <211> 97
   <212> DNA
   <213> Homo sapiens
<400> 146
   ggggttatag tttggcgttc ggattttggt tcgggttatt tgcgaaggag tcggttttgg 60
   ttaaggtgtt tttttggacg ggtgtggttt ttagagc                          97
<210> 147
   <211> 89
   <212> DNA
   <213> Homo sapiens
<400> 147
   ggtttcggtt tcgttttgtt cgttgttttc ggcgacggtc gtggtttttg ttttggggtt 60
   aattatagag cgaggtttga gtcgtagag                                   89
<210> 148
   <211> 113
   <212> DNA
   <213> Homo sapiens
<400> 148
   gggatagtgg ggttgacgcg tggtttcggc gtcgcgcggt ttttcgaatt tcgagtttcg 60
   cgttcggcgc ggtcggggtt tttaatcgtt ttttcgttcg tcgggatttt tat        113
<210> 149
   <211> 83
   <212> DNA
   <213> Homo sapiens
<400> 149
   gcgtgggttt tcgtcgtagt ttcgcggagt ttcggtgttt tttgtaatag ggggcggggg 60
   gaatagcggc gagtagtttt ggg                                          83
<210> 150
   <211> 120
   <212> DNA
   <213> Homo sapiens
<400> 150
   gggtgttcga tttaagtcga gttcgagttc gagtttaggt aggagtttta tagatagttt 60
   tttttttttt tattttttgt aggcgtttta cgcgtgcgat tttttcggtt aagattcgcg 120
<210> 151
   <211> 118
   <212> DNA
   <213> Homo sapiens
<400> 151
   gtttagttaa gttcggttcg ggggttttta ggttaggata tcgaggtaag agttatttga 60
   atcgttggcg aattggtggt tgttgcggcg acggtagcgg aggttgtaaa ttgtaatc   118
<210> 152
   <211> 113
   <212> DNA
   <213> Homo sapiens
<400> 152
   agggagttta gttaagttcg gttcgggggt ttttaggtta ggatatcgag gtaagagtta 60
   tttgaatcgt tggcgaattg gtggttgttg cggcgacggt agcggaggtt gta        113
<210> 153
   <211> 90
   <212> DNA
   <213> Homo sapiens
<400> 153
   tagagcggag gaagttgcgg atttggggtg ggggaattcg ttcgcggatt tttggttttt 60
   atttcgcgtc ggtttttgtg ttcgtatttg                                  90
<210> 154
   <211> 87
   <212> DNA
   <213> Homo sapiens
<400> 154
   tcggagtttt atagggtaac gaagcgcggg tagcggttgc ggagtcgggc ggaggtgcgc 60
   ggggtcgggg cgtgcggtta gtaagag                                     87
<210> 155
   <211> 111
   <212> DNA
   <213> Homo sapiens
<400> 155
   ttggagattt cgatagagcg tcggtttttt gattgttcgc gaagcgagac gcggggcgtc 60
   gggtttagcg tagtgagcgg cgaggcgcgg cggaggtgta gtcggtagtt t 111
<210> 156
   <211> 120
   <212> DNA
   <213> Homo sapiens
<400> 156
   gcgataggtt tttagtaagt aagcgcgggc ggtattcgta gtttttagaa gtttgagatt 60
   tggtcgtaag cggattcgtg cgttttaatt ttttgtcgcg ttagcgtttg gagcggagag 120
<210> 157
   <211> 91
   <212> DNA
   <213> Homo sapiens
<400> 157
   ttcggggtgt agcggtcgtc ggggttgggg tcggcgggag ttcgcgggat tttttagaag 60
   agcggtcggc gtcgtgattt agtattgggg c 91
<210> 158
   <211> 101
   <212> DNA
   <213> Homo sapiens
<400> 158
   acgtaagagt ttgggagcgt tcgagtcgtt cggttgttcg gagttttatc gtttaggatc 60
   gggagatgtt ggaaatgtaa tcgtttgttt ttcgaggagt c 101
<210> 159
   <211> 96
   <212> DNA
   <213> Homo sapiens
<400> 159
   gtttcggggt tcgtttttcg gtaggttcgg ggagaggtgg ggtgataatg ggttggggtg 60
   cgcgcgtgtt ttataggtgc gagatagagc gagtcg 96
<210> 160
   <211> 97
   <212> DNA
   <213> Homo sapiens
<400> 160
   tttgtcggcg tcgttatttt cgtacggttc gttttcgtcg cgggcgtata tagggtagta 60
   gtcgtacgcg tcgcgggttt cgtttagtag gtagttt 97
<210> 161
   <211> 114
   <212> DNA
   <213> Homo sapiens
<400> 161
   cgtttatggg tcggttacgt cgggtgttcg tttatttttc gacgttagta ggagcgcgcg 60
   cgtaggtttc gcggggtcgg gagggcggta cgggcggggt tttcgtgttt ttat 114
<210> 162
   <211> 83
   <212> DNA
   <213> Homo sapiens
<400> 162
   agttgagaat cggacggggt gggatcgagg agggtgcgaa gcgttattgt ttaggtttcg 60
   ttttttcggg agtttgattc gtt 83
<210> 163
   <211> 91
   <212> DNA
   <213> Homo sapiens
<400> 163
   gggattataa gtcgcgtcgc gttgtcgttg gttttttagt aattttcgat atggcgttga 60
   ggcggttatc gcgatttcgg ttttgcgttc g 91
<210> 164
   <211> 91
   <212> DNA
   <213> Homo sapiens
<400> 164
   gcgcgtagag ttgtaaaggt tcgagtagga gtacggttta ggcgaagcgt attatttttt 60
   ttgttagatt gatttcgttc gaggaggacg t 91
<210> 165
   <211> 92
   <212> DNA
   <213> Homo sapiens
<400> 165
   ggtagaggcg aggagttgtt cgttttgtac gtttttaatc gtattacgtg aataaatagt 60
   tgaggggcgg tcgggttaga acggtttgtg ta 92
<210> 166
   <211> 97
   <212> DNA
   <213> Homo sapiens
<400> 166
   gatgtcgttt gggagtagtg cgggtttttg tattgttaag gttttatagg tacgggttgg 60
   gcgggggtgg gtagttcgtt agttagcggt attttgt 97
<210> 167
   <211> 109
   <212> DNA
   <213> Homo sapiens
<400> 167
   gcggggtttt ttttatcggt tagattcggg gagaggcgcg cggaggttgc gaaggtttta 60
   gaagggcggg gagggggcgt cgcgcgttga ttttttttgg gtatcgttg 109
<210> 168
   <211> 97
   <212> DNA
   <213> Homo sapiens
<400> 168
   gttttcgttg tcgttacggg ttcgtttttt ttttttttcg gtttttaggg taaggcgcgg 60
   ggcgcggggt tggatgtagg cgttttgttc gttgcgc 97
<210> 169
   <211> 113
   <212> DNA
   <213> Homo sapiens
<400> 169
   agaatttagg tcggttttta tcgtttttta gaacgattgt attattgtcg ttgtcgtcgg 60
   tttgatattg ttttagtttt agtgttggta gttttgggag aagaattttg cgt 113
<210> 170
   <211> 82
   <212> DNA
   <213> Homo sapiens
<400> 170
   gtcggatgaa gtattcgggc gtttttattg cggaagggcg gggatggttg tgacgtaggc 60
   gtgttcgtcg tcgtttgtag gg 82
<210> 171
   <211> 96
   <212> DNA
   <213> Homo sapiens
<400> 171
   ttatttcgtt tttagggagt tttcgggtta tttttttatt cgggttgttt cgcggttttt 60
   aaggagtttt attttcggga ttaaatggtt cgtaag 96
<210> 172
   <211> 109
   <212> DNA
   <213> Homo sapiens
<400> 172
   tttcgtgtgg gaagagttcg tttgggtgta gcgtcgcggt tcgtaatatt agtaacggta 60
   gtagtagtag tattggtaac gacgatagta tcggtcgttc ggttattcg             109
<210> 173
   <211> 97
   <212> DNA
   <213> Homo sapiens
<400> 173
   ttttggttat taggtagttc ggttcggcgg ttcgttcggg gtattagttc ggtgtagggc 60
   gcggagtcgt tttgtagcgg gaatagtacg aagagtg                          97
<210> 174
   <211> 114
   <212> DNA
   <213> Homo sapiens
<400> 174
   ttattttgcg agcggtttcg cgatacgagg tagtcgtttt cgtttttcga cgcggttatg 60
   ggttcggtcg gcgcgggggt aagttagagc gagtcgcgtg gaattagagt attc       114
<210> 175
   <211> 75
   <212> DNA
   <213> Homo sapiens
<400> 175
   gatttgggcg tttttggttt ttcgcggttt cgagttttcg ataaattttt tgcgtcgatt 60
   gcggtatgag aagtc                                                  75
<210> 176
   <211> 113
   <212> DNA
   <213> Homo sapiens
<400> 176
   gggcgttgag gtagaagaac gtgtacgagg tgaaggatta taaatttatc gcgcgttttt 60
   ttaagtagtt tattttttgt agttattgta tcgattttat ttggtaggtg tcg        113
<210> 177
   <211> 102
   <212> DNA
   <213> Homo sapiens
<400> 177
   ttaggtcgga ggtttcgttt tttttttttt ggtttttttt ttttttcgtg ggtcggtcgt 60
   taacgacgtt agagtcggaa atgacgataa cggtgagggt tt                    102
<210> 178
   <211> 103
   <212> DNA
   <213> Homo sapiens
<400> 178
   gatgtttcgg tcgaggtttc gtcgtagttt ttttttagtt tttaggtcgc ggcgttttta 60
   ttcgggattt tttcggattt ggcgtattta tttgtagtcg ttt                    103
<210> 179
   <211> 126
   <212> DNA
   <213> Homo sapiens
<400> 179
   tgtagttttc ggagttagtg tcgcgcgttc gtcgtttcgc gtttttttgt tcgtcgtatt 60
   ttcgggagtc ggggcgtatt tagttcgtag cgtcgttttt tcgttcgcgt cgttttcgat 120
   cgtagg                                                            126
<210> 180
   <211> 96
   <212> DNA
   <213> Homo sapiens
<400> 180
   ttttgttcgt cgtattttcg ggagtcgggg cgtatttagt tcgtagcgtc gttttttcgt 60
   tcgcgtcgtt ttcgatcgta ggtcgagggt cgttat                            96
<210> 181
   <211> 80
   <212> DNA
   <213> Homo sapiens
<400> 181
   agtatagagt ggggagcgta gcgacgaaga atgaataggg tttcgtgagg ttttaaatat 60
   tcgtttcggt ggtaggaaag                                              80
<210> 182
   <211> 79
   <212> DNA
   <213> Homo sapiens
<400> 182
   ttgcgttagt cgtttgcgtt cgtttttagt ttatattatg aaagcgttta tcggtcgtcg 60
   gatacgggat tcgtttttg                                               79
<210> 183
   <211> 79
   <212> DNA
   <213> Homo sapiens
<400> 183
   gttagtcgag ttcggaggta tcgggaggtc gagagtcgtc gggattttag ttttgcgttt 60
   attgtttcgt tcggagttg 79
<210> 184
   <211> 95
   <212> DNA
   <213> Homo sapiens
<400> 184
   gcgtcggagg ttaaggttgt ttcgtacggt tcggcgggcg agcgagttcg ggttgtagta 60
   gtttcgtcgg cggcgcgtac ggtaattttg gagag 95
<210> 185
   <211> 99
   <212> DNA
   <213> Homo sapiens
<400> 185
   cgggttagag tattgttcgg tggtgtttag gaggagtagg agtaggagta gaagtagaag 60
   cggggttcgg agttgcgcgt ttacgcggga tttgtgttc 99
<210> 186
   <211> 114
   <212> DNA
   <213> Homo sapiens
<400> 186
   gattttatcg gggaaatatc gcggatagtc gggttagtag cgttcggagt ttattttagg 60
   tttttaaatt tgtagtattt tttagagcgc gcgcgttcgg agcgggattt gttt 114
<210> 187
   <211> 117
   <212> DNA
   <213> Homo sapiens
<400> 187
   ttatttcgta ggttgaggtt agggcgtggc ggttgttggg atttcggagt tttttagtag 60
   taggggttgc gggaggaagt gaagtcggga ggggttgtcg gcgttgatag tagagga 117
<210> 188
   <211> 108
   <212> DNA
   <213> Homo sapiens
<400> 188
   gttgtatttt cgcggagcgt tcggtagaaa tagtttaggg aagacgaaaa atagttagcg 60
   gagtcgttta ggttgtagtt ataaagcgtc ggttagacgt attgtgag 108
<210> 189
   <211> 80
   <212> DNA
   <213> Homo sapiens
<400> 189
   gtttaggttg tggtttaggt cgtcggtttt cggttatgtt tagttttttt gaggtcgttt 60
   ttagtgagga tacgaggtgt                                             80
<210> 190
   <211> 97
   <212> DNA
   <213> Homo sapiens
<400> 190
   ggggttttta ggtattcggt tcgtacgtaa atttttagtt cggggttttt tgattttcgc 60
   gtttattttt tagttcggtc gtcgggtatt gcgtatt                          97
<210> 191
   <211> 95
   <212> DNA
   <213> Homo sapiens
<400> 191
   tcggttttta gttttttcgg tcgcggggtg ggagttgggg gttgggtcgg tagtcgggat 60
   ttcgggcgtt ttgtttcgtt tttgcgtagt aattg                            95
<210> 192
   <211> 116
   <212> DNA
   <213> Homo sapiens
<400> 192
   ttatcgagaa gcgtcggttt cggggttgtt tatagcggtt cgggagaggt tgtggtggtt 60
   tcgagcgcga gtgtgtaggt gataggatag cggttaggtt cgtttttttt ttcggt    116
<210> 193
   <211> 106
   <212> DNA
   <213> Homo sapiens
<400> 193
   ctcaatgtca cgctctggcg ctcgtcgccc gtgctccccc ttccagccgg tttccgcaga 60
   atgccaggta ctgacgttgg agagcggggc cggagagggc tggttc                106
<210> 194
   <211> 101
   <212> DNA
   <213> Homo sapiens
<400> 194
   gcggctgcaa ggcctttggc cggtgagtga accagtaggc aaggatggca gccagggcac 60
   ccatactcac gagggtggtg gccgagaggc tgcggaaaaa c                     101
<210> 195
   <211> 91
   <212> DNA
   <213> Homo sapiens
<400> 195
   ggacgggtgt ctgcgctcca cgcttagctc gtccaggtgg gggctcccgc ctcctcggct 60
   gctgcggtcc ccgcccagct ccttggtccc g                                91
<210> 196
   <211> 99
   <212> DNA
   <213> Homo sapiens
<400> 196
   gccgcctgcc caggcccgga ccgggctttg tccgccccgg agcccctgcc cgcgccgcgg 60
   agaccccgga gcccgcgcgc tccgaggcca ccccgggcc                        99
<210> 197
   <211> 96
   <212> DNA
   <213> Homo sapiens
<400> 197
   ccggggccct tacctggtcc gctttccccc gggccggatg ccagcccgcc gagcgcaggg 60
   cagcggggag ctggtagcga gacacgagtg acgact                           96
<210> 198
   <211> 104
   <212> DNA
   <213> Homo sapiens
<400> 198
   gcacgtgcgc tcactgcgag ctgcggcgcc gcacagcttc gtggcgctct gggcacccct 60
   gttcctgctg cgctccgccc tggccgactt cagcctggac aacg                  104
<210> 199
   <211> 111
   <212> DNA
   <213> Homo sapiens
<400> 199
   ggcatcggct tggccatcgc gcgcgaactg tgccgacagt tctctgggga tgtggtgctc 60
   accgcgcggg acgtggcgcg gggccaggcg gccgtgcagc agctgcaggc g          111
<210> 200
   <211> 80
   <212> DNA
   <213> Homo sapiens
<400> 200
   gcccccgact gacccatcaa ggtccgattt ggcttcggac acctcgcaga tcaccccgcg 60
   gctcagagcc cggatcctga 80
<210> 201
   <211> 105
   <212> DNA
   <213> Homo sapiens
<400> 201
   ccgtgggaag agagcgtagc agctgctggg gccgcaggcg gcacggggct cagcagccca 60
   ggggtcctgg cccagtgtgg gccctgcggc cgcccaggcc agcaa 105
<210> 202
   <211> 100
   <212> DNA
   <213> Homo sapiens
<400> 202
   ccctgtcaag agctgccgcc agcccggggc cggaccagtc cgggggcatc gcgatgctgc 60
   tgcgcctgtt gctggcctgg gcggccgcag ggcccacact 100
<210> 203
   <211> 95
   <212> DNA
   <213> Homo sapiens
<400> 203
   ggggcagccg tcaactgcgc cttctcccct cctccgcccc caaccttaga gccccccacc 60
   ccactgcttc ctgctctagc ggcccccggg gaaga 95
<210> 204
   <211> 90
   <212> DNA
   <213> Homo sapiens
<400> 204
   cgggagaaga aagccagtgc gtctctgggc gcaggggcca gtggggctcg gaggcacagg 60
   caccccgcga cactccaggt tccccgaccc 90
<210> 205
   <211> 120
   <212> DNA
   <213> Homo sapiens
<400> 205
   gagcgcccgg gctttgcggg gagcaggcta aggcggccga gagaaagggg ggtcgagacg 60
   ggggggtgga ggtttggggg ggtggggggg caggcggccg ccatcttctc gccgaggccg 120
<210> 206
   <211> 100
   <212> DNA
   <213> Homo sapiens
<400> 206
   ctgggaatcc agctgtcgcc gccccgcaga gccccctgtc cccggagggc gctcatttcc 60
   gggccgccca ccacccgcgt agcaccggca gccgctgtcc 100
<210> 207
   <211> 85
   <212> DNA
   <213> Homo sapiens
<400> 207
   gaggtctgcg gcccaggttc gattcctgcg acttgtccta ggcaggcctg tatgtgcgcg 60
   gcggccgcgt gctgtacagt gtgag 85
<210> 208
   <211> 106
   <212> DNA
   <213> Homo sapiens
<400> 208
   ggagttgggg ctcacgagac ggggcgtgcg gggcaccggg cggccggcgg ggagtcgcag 60
   gcttccccag agggggcgcg agccgggccg ccggggaact caccat 106
<210> 209
   <211> 114
   <212> DNA
   <213> Homo sapiens
<400> 209
   ggtgccctga cagaagtcgt cagccggtgt catgcccagg acaggcatct gcagccttgt 60
   gtggacgtca acgccaccag gaaggaccat caggccgtgg gcgtcaatgg tctt 114
<210> 210
   <211> 97
   <212> DNA
   <213> Homo sapiens
<400> 210
   ggggtcacag cctggcgctc ggaccctggc ccgggtcatc tgcgaaggag ccggctttgg 60
   ccaaggtgcc ttcctggacg ggtgtggttc ccagagc 97
<210> 211
   <211> 89
   <212> DNA
   <213> Homo sapiens
<400> 211
   ggccccggct ccgccctgcc cgctgccctc ggcgacggcc gtggtccctg ccctggggtc 60
   aattacagag cgaggtctga gccgcagag 89
<210> 212
   <211> 113
   <212> DNA
   <213> Homo sapiens
<400> 212
   gggacagtgg ggctgacgcg tggcttcggc gccgcgcggt ctcccgaatc ccgagccccg 60
   cgcccggcgc ggccggggtc cccaaccgcc ctcccgctcg ccgggacccc cac       113
<210> 213
   <211> 83
   <212> DNA
   <213> Homo sapiens
<400> 213
   gcgtgggccc ccgccgcagc tccgcggagc ctcggtgtct cctgcaacag ggggcggggg 60
   gaacagcggc gagcagccct ggg                                         83
<210> 214
   <211> 120
   <212> DNA
   <213> Homo sapiens
<400> 214
   gggtgtccga cccaagccga gcccgagccc gagcccaggc aggagcttta cagacagcct 60
   cttcccttcc cacttcctgc aggcgcccca cgcgtgcgat cctcccggcc aagacccgcg 120
<210> 215
   <211> 118
   <212> DNA
   <213> Homo sapiens
<400> 215
   gcccagccaa gtccggcccg ggggccccta ggctaggaca tcgaggcaag agccacctga 60
   accgctggcg aattggtggc tgctgcggcg acggcagcgg aggttgcaaa ttgcaatc   118
<210> 216
   <211> 113
   <212> DNA
   <213> Homo sapiens
<400> 216
   agggagccca gccaagtccg gcccgggggc ccctaggcta ggacatcgag gcaagagcca 60
   cctgaaccgc tggcgaattg gtggctgctg cggcgacggc agcggaggtt gca        113
<210> 217
   <211> 90
   <212> DNA
   <213> Homo sapiens
<400> 217
   cagagcggag gaagctgcgg acctggggtg ggggaacccg cccgcggacc cctggccccc 60
   accccgcgcc ggcctctgtg cccgcatctg                                  90
<210> 218
   <211> 87
   <212> DNA
   <213> Homo sapiens
<400> 218
   tcggagtccc acagggcaac gaagcgcggg tagcggctgc ggagccgggc ggaggtgcgc 60
   ggggccgggg cgtgcggcca gcaagag 87
<210> 219
   <211> 111
   <212> DNA
   <213> Homo sapiens
<400> 219
   ctggagacct cgacagagcg ccggccccct gactgcccgc gaagcgagac gcggggcgcc 60
   gggtctagcg cagtgagcgg cgaggcgcgg cggaggtgca gccggcagcc c 111
<210> 220
   <211> 120
   <212> DNA
   <213> Homo sapiens
<400> 220
   gcgacaggcc tccagcaagc aagcgcgggc ggcatccgca gtctccagaa gtttgagact 60
   tggccgtaag cggactcgtg cgccccaact ctttgccgcg ccagcgcctg gagcggagag 120
<210> 221
   <211> 91
   <212> DNA
   <213> Homo sapiens

<400> 221
   cccggggtgc agcggccgcc ggggctgggg ccggcgggag tccgcgggac cctccagaag 60
   agcggccggc gccgtgactc agcactgggg c 91
<210> 222
   <211> 101
   <212> DNA
   <213> Homo sapiens
<400> 222
   acgtaagagc ctgggagcgc ccgagccgcc cggctgcccg gagccccatc gcctaggacc 60
   gggagatgct ggaaatgcaa ccgcctgttc cccgaggagc c 101
<210> 223
   <211> 96
   <212> DNA
   <213> Homo sapiens
<400> 223
   gctccggggc tcgctctccg gcaggcccgg ggagaggtgg ggtgacaatg ggttggggtg 60
   cgcgcgtgcc tcataggtgc gagacagagc gagccg 96
<210> 224
   <211> 97
   <212> DNA
   <213> Homo sapiens
<400> 224
   cctgccggcg ccgccacccc cgcacggctc gccctcgccg cgggcgcaca tagggcagca 60
   gccgcacgcg tcgcgggtct cgcccagcag gcagccc 97
<210> 225
   <211> 114
   <212> DNA
   <213> Homo sapiens
<400> 225
   cgcccatggg ccggtcacgc cgggtgcccg ctcacccccc gacgccagca ggagcgcgcg 60
   cgcaggcccc gcggggccgg gagggcggca cgggcggggc ccccgtgctc tcac 114
<210> 226
   <211> 83
   <212> DNA
   <213> Homo sapiens
<400> 226
   agctgagaac cggacggggt gggatcgagg agggtgcgaa gcgccactgt ttaggtttcg 60
   ctttcccggg agcctgaccc gcc 83
<210> 227
   <211> 91
   <212> DNA
   <213> Homo sapiens
<400> 227
   gggactacaa gccgcgccgc gctgccgctg gcccctcagc aaccctcgac atggcgctga 60
   ggcggccacc gcgactccgg ctctgcgctc g 91
<210> 228
   <211> 91
   <212> DNA
   <213> Homo sapiens
<400> 228
   gcgcgcagag ctgcaaaggc ccgagcagga gcacggtcca ggcgaagcgc atcactcctt 60
   ttgccagatt gaccccgctc gaggaggacg t 91
<210> 229
   <211> 92
   <212> DNA
   <213> Homo sapiens
<400> 229
   ggcagaggcg aggagctgtc cgccttgcac gtttccaatc gcattacgtg aacaaatagc 60
   tgaggggcgg ccgggccaga acggcttgtg ta 92
<210> 230
   <211> 97
   <212> DNA
   <213> Homo sapiens
<400> 230
   gatgtcgtct gggagcagtg cgggcccctg cattgccaag gccttatagg cacgggctgg 60
   gcgggggtgg gcagtccgcc agccagcggc attctgc 97
<210> 231
   <211> 109
   <212> DNA
   <213> Homo sapiens
<400> 231
   gcggggctcc ccctaccggc cagacccggg gagaggcgcg cggaggctgc gaaggttcca 60
   gaagggcggg gagggggcgc cgcgcgctga ccctccctgg gcaccgctg 109
<210> 232
   <211> 97
   <212> DNA
   <213> Homo sapiens
<400> 232
   gccttcgctg ccgccacggg cccgtcttct tcctccttcg gctcccaggg taaggcgcgg 60
   ggcgcggggt tggatgcagg cgccctgccc gctgcgc 97
<210> 233
   <211> 113
   <212> DNA
   <213> Homo sapiens
<400> 233
   agaattcagg ccggcctcta tcgcttccca gaacgattgc accactgccg ctgccgccgg 60
   cctgacactg cctcagcctc agtgctggca gctttgggag aagaaccctg cgc 113
<210> 234
   <211> 82
   <212> DNA
   <213> Homo sapiens
<400> 234
   gccggatgaa gcattcgggc gttcccactg cggaagggcg gggatggctg tgacgcaggc 60
   gtgcccgccg tcgcctgcag gg 82
<210> 235
   <211> 96
   <212> DNA
   <213> Homo sapiens
<400> 235
   ccactccgcc cccagggagc tcccgggtca tcctctcatc cgggctgccc cgcggccccc 60
   aaggagcccc acccccggga ccaaatggcc cgcaag 96
<210> 236
   <211> 109
   <212> DNA
   <213> Homo sapiens
<400> 236
   ccccgtgtgg gaagagctcg tctgggtgca gcgccgcggc ccgcaacatt agcaacggca 60
   gcagcagtag cactggtaac gacgacagca ccggccgccc ggccacccg 109
<210> 237
   <211> 97
   <212> DNA
   <213> Homo sapiens
<400> 237
   ccttggtcac caggtagccc ggctcggcgg cccgcccggg gcatcagctc ggtgcagggc 60
   gcggagccgt tctgcagcgg gaacagcacg aagagtg 97
<210> 238
   <211> 114
   <212> DNA
   <213> Homo sapiens
<400> 238
   tcactctgcg agcggccccg cgacacgagg cagccgctcc cgtcctccga cgcggccatg 60
   ggcccggccg gcgcgggggc aagttagagc gagccgcgtg gaatcagagc atcc 114
<210> 239
   <211> 75
   <212> DNA
   <213> Homo sapiens
<400> 239
   gacctgggcg cctctggctc tccgcggtcc cgagttctcg acaaactttc tgcgccgact 60
   gcggcatgag aagcc 75
<210> 240
   <211> 113
   <212> DNA
   <213> Homo sapiens
<400> 240
   gggcgctgag gcagaagaac gtgcacgagg tgaaggacca caaattcatc gcgcgcttct 60
   tcaagcagcc caccttctgc agccactgca ccgacttcat ctggtaggtg ccg        113
<210> 241
   <211> 102
   <212> DNA
   <213> Homo sapiens
<400> 241
   ccaggccgga ggccccgccc ccttcctcct ggctcctccc ctcctccgtg ggccggccgc 60
   caacgacgcc agagccggaa atgacgacaa cggtgagggt tc                   102
<210> 242
   <211> 103
   <212> DNA
   <213> Homo sapiens
<400> 242
   gatgctccgg ccgaggtccc gccgcagccc tcccccagcc cccaggtcgc ggcgccctca 60
   cccgggaccc ctccggacct ggcgcatcca tctgcagccg ccc                  103
<210> 243
   <211> 126
   <212> DNA
   <213> Homo sapiens
<400> 243
   tgcagcctcc ggagtcagtg ccgcgcgccc gccgccccgc gccttcctgc tcgccgcacc 60
   tccgggagcc ggggcgcacc cagcccgcag cgccgcctcc ccgcccgcgc cgcctccgac 120
   cgcagg                                                            126
<210> 244
   <211> 96
   <212> DNA
   <213> Homo sapiens
<400> 244
   tcctgctcgc cgcacctccg ggagccgggg cgcacccagc ccgcagcgcc gcctccccgc 60
   ccgcgccgcc tccgaccgca ggccgagggc cgccac                           96
<210> 245
   <211> 80
   <212> DNA
   <213> Homo sapiens
<400> 245
   agtacagagt ggggagcgca gcgacgaaga atgaacaggg cctcgtgagg tcccaaacac 60
   ccgtttcggt ggcaggaaag 80
<210> 246
   <211> 79
   <212> DNA
   <213> Homo sapiens
<400> 246
   ctgcgccagc cgcttgcgct cgtccttagc ccacaccatg aaagcgttca tcggccgccg 60
   gatacgggac tcgcccttg 79
<210> 247
   <211> 79
   <212> DNA
   <213> Homo sapiens
<400> 247
   gccagccgag tccggaggca tcgggaggtc gagagccgcc gggaccccag ctctgcgttc 60
   actgccccgt ccggagctg 79
<210> 248
   <211> 95
   <212> DNA
   <213> Homo sapiens
<400> 248
   gcgccggagg ccaaggttgc cccgcacggc ccggcgggcg agcgagctcg ggctgcagca 60
   gccccgccgg cggcgcgcac ggcaactttg gagag 95
<210> 249
   <211> 99
   <212> DNA
   <213> Homo sapiens
<400> 249
   cgggtcagag cactgtccgg tggtgcccag gaggagtagg agcaggagca gaagcagaag 60
   cggggtccgg agctgcgcgc ctacgcggga cctgtgtcc 99
<210> 250
   <211> 114
   <212> DNA
   <213> Homo sapiens
<400> 250
   gacctcaccg gggaaacacc gcggacagtc gggccagcag cgcccggagc tcactccagg 60
   tctccaaact tgcagcactt cccagagcgc gcgcgctcgg agcgggacct gctt 114
<210> 251
   <211> 117
   <212> DNA
   <213> Homo sapiens
<400> 251
   ttaccccgca ggctgaggcc agggcgtggc ggctgctggg atcccggagc ttctcagtag 60
   caggggctgc gggaggaagt gaagccggga ggggctgccg gcgctgacag cagagga    117
<210> 252
   <211> 108
   <212> DNA
   <213> Homo sapiens
<400> 252
   gctgcatctt cgcggagcgc ccggcagaaa tagcctaggg aagacgaaaa acagctagcg 60
   gagccgccca ggctgcagct ataaagcgcc ggccagacgc actgtgag              108
<210> 253
   <211> 80
   <212> DNA
   <213> Homo sapiens
<400> 253
   gcccaggctg tggcctaggc cgtcggttcc cggccatgcc tagctcctct gaggtcgccc 60
   ttagtgagga cacgaggtgc                                             80
<210> 254
   <211> 97
   <212> DNA
   <213> Homo sapiens
<400> 254
   ggggccccca ggcacccggc ccgcacgcaa accctcagcc cggggccccc tgacccccgc 60
   gttcacccct cagcccggcc gccgggcact gcgcatc                          97
<210> 255
   <211> 95
   <212> DNA
   <213> Homo sapiens
<400> 255
   ccggccttca gtcccctcgg ccgcggggtg ggagctgggg gctgggccgg cagccgggac 60
   cccgggcgtc ctgtcccgtt tctgcgcagc aactg                            95
<210> 256
   <211> 116
   <212> DNA
   <213> Homo sapiens
<400> 256
   ccaccgagaa gcgccggcct cggggctgtc tacagcggcc cgggagaggc tgtggtggcc 60
   ccgagcgcga gtgtgtaggt gacaggacag cggccaggcc cgcccctccc ctcggt     116
<210> 257
   <211> 23
   <212> DNA
   <213> Homo sapiens
<400> 257
   tcggagtttt atagggtaac gaa 23
<210> 258
   <211> 21
   <212> DNA
   <213> Homo sapiens
<400> 258
   ttggagattt cgatagagcg t 21
<210> 259
   <211> 23
   <212> DNA
   <213> Homo sapiens
<400> 259
   ttaaataaaa ggtttacgag cgg 23
<210> 260
   <211> 23
   <212> DNA
   <213> Homo sapiens
<400> 260
   cgtaagtaat gtttcgagcg agt 23
<210> 261
   <211> 24
   <212> DNA
   <213> Homo sapiens
<400> 261
   gttttcgtaa gtaatgtttc gagc 24
<210> 262
   <211> 21
   <212> DNA
   <213> Homo sapiens
<400> 262
   ttcggggtat tgtttacgaa g 21
<210> 263
   <211> 22
   <212> DNA
   <213> Homo sapiens
<400> 263
   gtcgtaatag gttcggttcg tt 22
<210> 264
   <211> 19
   <212> DNA
   <213> Homo sapiens
<400> 264
   ctcttactaa ccgcacgcc 19
<210> 265
   <211> 21
   <212> DNA
   <213> Homo sapiens
<400> 265
   aaactaccga ctacacctcc g 21
<210> 266
   <211> 22
   <212> DNA
   <213> Homo sapiens
<400> 266
   taaatatccg acgacaaacg aa 22
<210> 267
   <211> 21
   <212> DNA
   <213> Homo sapiens
<400> 267
   tacttccacg tctacctccc g 21
<210> 268
   <211> 21
   <212> DNA
   <213> Homo sapiens
<400> 268
   tacttccacg tctacctccc g 21
<210> 269
   <211> 23
   <212> DNA
   <213> Homo sapiens
<400> 269
   aataaaatct aaaaccgcac acg 23
<210> 270
   <211> 21
   <212> DNA
   <213> Homo sapiens
<400> 270
   aacacgtcct actatcctcg c 21
<210> 271
   <211> 87
   <212> DNA
   <213> Homo sapiens
<400> 271
   tcggagtttt atagggtaac gaagcgcggg tagcggttgc ggagtcgggc ggaggtgcgc 60
   ggggtcgggg cgtgcggtta gtaagag 87
<210> 272
   <211> 111
   <212> DNA
   <213> Homo sapiens
<400> 272
   ttggagattt cgatagagcg tcggtttttt gattgttcgc gaagcgagac gcggggcgtc 60
   gggtttagcg tagtgagcgg cgaggcgcgg cggaggtgta gtcggtagtt t 111
<210> 273
   <211> 103
   <212> DNA
   <213> Homo sapiens
<400> 273
   ttaaataaaa ggtttacgag cggcggtttc gacgtgggga gtaagtaggt ttttttggtt 60
   tttgtagtag gcggatttag gttcgtttgt cgtcggatat tta 103
<210> 274
   <211> 95
   <212> DNA
   <213> Homo sapiens
<400> 274
   cgtaagtaat gtttcgagcg agtagatttt aggataataa ataaattaaa attatatttt 60
   aaattattgg agatcgggag gtagacgtgg aagta 95
<210> 275
   <211> 100
   <212> DNA
   <213> Homo sapiens
<400> 275
   gttttcgtaa gtaatgtttc gagcgagtag attttaggat aataaataaa ttaaaattat 60
   attttaaatt attggagatc gggaggtaga cgtggaagta 100
<210> 276
   <211> 113
   <212> DNA
   <213> Homo sapiens
<400> 276
   ttcggggtat tgtttacgaa gatcgtttat atgttgttgt atttttttat ttatttgtta 60
   cgtgatcgcg tttgtttatt ataggtttaa cgtgtgcggt tttagatttt att 113
<210> 277
   <211> 120
   <212> DNA
   <213> Homo sapiens
<400> 277
   gtcgtaatag gttcggttcg ttatagtagg ttttgaaggc gggtttttag cgttcgagta 60
   tcgcgaggag ggtgtcgtag tggttagtcg cgttcgtcgg cgaggatagt aggacgtgtt 120
<210> 278
   <211> 87
   <212> DNA
   <213> Homo sapiens
<400> 278
   tcggagtccc acagggcaac gaagcgcggg tagcggctgc ggagccgggc ggaggtgcgc 60
   ggggccgggg cgtgcggcca gcaagag                                     87
<210> 279
   <211> 111
   <212> DNA
   <213> Homo sapiens
<400> 279
   ctggagacct cgacagagcg ccggccccct gactgcccgc gaagcgagac gcggggcgcc 60
   gggtctagcg cagtgagcgg cgaggcgcgg cggaggtgca gccggcagcc c          111
<210> 280
   <211> 103
   <212> DNA
   <213> Homo sapiens
<400> 280
   tcaaacaaaa ggctcacgag cggcggcccc gacgtgggga gcaagcaggc ttctctggcc 60
   cctgcagcag gcggacccag gcccgcctgc cgccggacat tca                   103
<210> 281
   <211> 95
   <212> DNA
   <213> Homo sapiens
<400> 281
   cgtaagcaat gccccgagcg agtagatttc aggacaacaa acaaatcaaa attacacttc 60
   aaattattgg agatcgggag gcagacgtgg aagca                            95
<210> 282
   <211> 100
   <212> DNA
   <213> Homo sapiens
<400> 282
   gtcttcgtaa gcaatgcccc gagcgagtag atttcaggac aacaaacaaa tcaaaattac 60
   acttcaaatt attggagatc gggaggcaga cgtggaagca                       100
<210> 283
   <211> 113
   <212> DNA
   <213> Homo sapiens
<400> 283
   ctcggggcat tgcttacgaa gaccgtttat atgttgctgc atccctctac ctatctgtta 60
   cgtgaccgcg cttgtccatc acaggcccaa cgtgtgcggc tccagattcc act 113
<210> 284
   <211> 120
   <212> DNA
   <213> Homo sapiens
<400> 284
   gccgcaacag gttcggtccg ctacagcagg ctctgaaggc gggtttctag cgcccgagta 60
   tcgcgaggag ggtgccgcag tggccagccg cgtccgccgg cgaggacagc aggacgtgct 120
<210> 285
   <211> 19
   <212> DNA
   <213> Homo sapiens
<400> 285
   gtagtggcga gatgacgga 19
<210> 286
   <211> 23
   <212> DNA
   <213> Homo sapiens
<400> 286
   agtgtttaga gagttcgtcg gtt 23
<210> 287
   <211> 21
   <212> DNA
   <213> Homo sapiens
<400> 287
   gtcgggtgtt atatggtgcg t 21
<210> 288
   <211> 22
   <212> DNA
   <213> Homo sapiens
<400> 288
   ttttgagagg tcgttatcgt gt 22
<210> 289
   <211> 19
   <212> DNA
   <213> Homo sapiens
<400> 289
   cgttaggtag ggaggaggc 19
<210> 290
   <211> 22
   <212> DNA
   <213> Homo sapiens
<400> 290
   atttgggtag aaaagttcgt tc 22
<210> 291
   <211> 22
   <212> DNA
   <213> Homo sapiens
<400> 291
   aacgatttgg ggttagagtt tc 22
<210> 292
   <211> 19
   <212> DNA
   <213> Homo sapiens
<400> 292
   ttttcgttat ggacgcgga 19
<210> 293
   <211> 25
   <212> DNA
   <213> Homo sapiens
<400> 293
   cgttcgtata gagttcggta atgtc 25
<210> 294
   <211> 21
   <212> DNA
   <213> Homo sapiens
<400> 294
   cgtttttggt tttgttttcg t 21
<210> 295
   <211> 21
   <212> DNA
   <213> Homo sapiens
<400> 295
   tttagtaggt ttggtcgagg c 21
<210> 296
   <211> 21
   <212> DNA
   <213> Homo sapiens
<400> 296
   tataggtaag ggggcggttt c 21
<210> 297
   <211> 18
   <212> DNA
   <213> Homo sapiens
<400> 297
   gcgttgaagt cggggttc 18
<210> 298
   <211> 19
   <212> DNA
   <213> Homo sapiens
<400> 298
   ttagaggggc gtagggtgc 19
<210> 299
   <211> 21
   <212> DNA
   <213> Homo sapiens
<400> 299
   cgttgtagga ttttgggggt c 21
<210> 300
   <211> 19
   <212> DNA
   <213> Homo sapiens
<400> 300
   aaccgaaacc aaacaaacg 19
<210> 301
   <211> 22
   <212> DNA
   <213> Homo sapiens
<400> 301
   cgtaacgaat aaactacgcg at 22
<210> 302
   <211> 21
   <212> DNA
   <213> Homo sapiens
<400> 302
   aaaaacaatc ctcgtccgaa a 21
<210> 303
   <211> 22
   <212> DNA
   <213> Homo sapiens
<400> 303
   ttactcgaac tattccccga tt 22
<210> 304
   <211> 24
   <212> DNA
   <213> Homo sapiens
<400> 304
   aacgataaaa taaaaacaac gacc 24
<210> 305
   <211> 21
   <212> DNA
   <213> Homo sapiens
<400> 305
   ctcaataatt ttcccgacga c 21
<210> 306
   <211> 22
   <212> DNA
   <213> Homo sapiens
<400> 306
   taaccaaact accgctttac ga 22
<210> 307
   <211> 20
   <212> DNA
   <213> Homo sapiens
<400> 307
   ataaccttac cgaccccgaa 20
<210> 308
   <211> 22
   <212> DNA
   <213> Homo sapiens
<400> 308
   cctataacta aacgcgacgc ac 22
<210> 309
   <211> 19
   <212> DNA
   <213> Homo sapiens
<400> 309
   aacctaattc ccgcccgtt 19
<210> 310
   <211> 19
   <212> DNA
   <213> Homo sapiens
<400> 310
   acgctaaccc caaaatccg 19
<210> 311
   <211> 20
   <212> DNA
   <213> Homo sapiens
<400> 311
   gcgactctaa aaattccgct 20
<210> 312
   <211> 24
   <212> DNA
   <213> Homo sapiens
<400> 312
   cccgtacttc gctaacttta aacg 24
<210> 313
   <211> 21
   <212> DNA
   <213> Homo sapiens
<400> 313
   gccaaactaa aatcccaacg a 21
<210> 314
   <211> 19
   <212> DNA
   <213> Homo sapiens
<400> 314
   acccttctcc cgctcgata 19
<210> 315
   <211> 92
   <212> DNA
   <213> Homo sapiens
<400> 315
   gtagtggcga gatgacggat atttagcgag tttaatgggc gtcgaacgcg tttaggtttg      60
   gtggatttgt tagcgtttgt ttggtttcgg tt                                    92
<210> 316
   <211> 94
   <212> DNA
   <213> Homo sapiens
<400> 316
   agtgtttaga gagttcgtcg gttttatcgt ttttttaaag gagaattcgg tttatcgttc      60
   gtcgcggcgg cgatcgcgta gtttattcgt tacg                                  94
<210> 317
   <211> 116
   <212> DNA
   <213> Homo sapiens
<400> 317
   gtcgggtgtt atatggtgcg tgataatttg tttttgattt gggtttattt gaagagcgta      60
   gaattttaat aaataaatag ttttttggga tttgttttcg gacgaggatt gttttt         116
<210> 318
   <211> 107
   <212> DNA
   <213> Homo sapiens
<400> 318
   ttttgagagg tcgttatcgt gttatgggcg tgcgtaattg tttttacggt aataatatgt     60
   taggataacg cgatattttt tttgaaatcg gggaatagtt cgagtaa                  107
<210> 319
   <211> 117
   <212> DNA
   <213> Homo sapiens
<400> 319
   cgttaggtag ggaggaggcg gggaggggtt ggttttagaa gtgcgtgttt gaagcggtta     60
   atgtgtgtaa attagtaagg aggaggggtg cggggtcgtt gtttttattt tatcgtt       117
<210> 320
   <211> 87
   <212> DNA
   <213> Homo sapiens
<400> 320
   atttgggtag aaaagttcgt tcgtgacgtt attaagtttc ggaagttttt tggcgtcggc     60
   gtaagggtcg tcgggaaaat tattgag                                          87
<210> 321
   <211> 109
   <212> DNA
   <213> Homo sapiens
<400> 321
   aacgatttgg ggttagagtt tcgggagttg gcggagcgcg gagttcgtat cgtttttaga     60
   ggtaggacgt agtttttttt tttgaattcg taaagcggta gtttggtta               109
<210> 322
   <211> 88
   <212> DNA
   <213> Homo sapiens
<400> 322
   ttttcgttat ggacgcggac gattttcggg tttttaaggg ttttttgcgg aagtttttgg     60
   agtatttttt cggggtcggt aaggttat                                         88
<210> 323
   <211> 108
   <212> DNA
   <213> Homo sapiens
<400> 323
   cgttcgtata gagttcggta atgtcgaggt ttttttaacg ggtcggtttg cgaggagtaa      60
   aaaaggggtt tagaggaggg tagcgcgtgc gtcgcgttta gttatagg                  108
<210> 324
   <211> 119
   <212> DNA
   <213> Homo sapiens
<400> 324
   cgtttttggt tttgttttcg tcgcggagcg gaatttttta agtcgcggtt tgaggaggaa      60
   ggaaaagggg gcggttcggg agagtcgttg cgaaattagt aacgggcggg aattaggtt     119
<210> 325
   <211> 120
   <212> DNA
   <213> Homo sapiens
<400> 325
   tttagtaggt ttggtcgagg cgggattttc gtcgttttag ttttcgtttt cgttttcgcg      60
   ttttttcgtc gcgcggagtt ttggttggtt tgttttttaa tcggattttg gggttagcgt    120
<210> 326
   <211> 109
   <212> DNA
   <213> Homo sapiens
<400> 326
   tataggtaag ggggcggttt cgtttcgcgt tttggaacga ttttacggtt tcgtttatat      60
   tttcgttttt ggttttattt tcgtcgtaga gcggaatttt tagagtcgc                109
<210> 327
   <211> 75
   <212> DNA
   <213> Homo sapiens
<400> 327
   gcgttgaagt cggggttcgt tttgtggttt cgttcggttc gcgtttgtta gcgtttaaag      60
   ttagcgaagt acggg                                                      75
<210> 328
   <211> 113
   <212> DNA
   <213> Homo sapiens
<400> 328
   ttagaggggc gtagggtgcg cgggggtcgt tggtttttcg ggtatttttt ttttgcggtt      60
   ttttcgtttt ttttcggagt tggtgtttga ggtcgttggg attttagttt ggc            113
<210> 329
   <211> 84
   <212> DNA
   <213> Homo sapiens
<400> 329
   cgttgtagga ttttgggggt cggacggtgg gatacggtta attttcgggg agatgttgtg      60
   gtttttatcg agcgggagaa gggt                                              84
<210> 330
   <211> 92
   <212> DNA
   <213> Homo sapiens
<400> 330
   gcagtggcga gatgacggac acccagcgag tccaatgggc gtcgaacgcg tctaggcttg      60
   gtggacttgt cagcgcctgc ctggcttcgg tc                                     92
<210> 331
   <211> 94
   <212> DNA
   <213> Homo sapiens
<400> 331
   agtgcccaga gagtccgccg gtcccaccgc cccttcaaag gagaacccgg cccaccgccc      60
   gccgcggcgg cgaccgcgca gcccactcgt cacg                                   94
<210> 332
   <211> 116
   <212> DNA
   <213> Homo sapiens
<400> 332 u
<210> 333
   <211> 107
   <212> DNA
   <213> Homo sapiens
<400> 333
   ttttgagagg ccgccaccgt gttatgggcg tgcgcaactg cctctacggc aataatatgt      60
   caggacaacg cgatatcccc cctgaaatcg gggaacagcc cgagcaa                    107
<210> 334
   <211> 117
   <212> DNA
   <213> Homo sapiens
<400> 334
   cgccaggcag ggaggaggcg gggaggggct ggccccagaa gtgcgtgtct gaagcggcca      60
   atgtgtgcaa atcagcaagg aggaggggtg cggggccgct gcccccacct caccgcc         117
<210> 335
   <211> 87
   <212> DNA
   <213> Homo sapiens
<400> 335
   atttgggtag aaaagctcgc tcgtgacgtc accaagctcc ggaagtctcc tggcgtcggc      60
   gcaagggccg ccgggaaaac cattgag                                          87
<210> 336
   <211> 109
   <212> DNA
   <213> Homo sapiens
<400> 336
   aacgacctgg ggctagagcc ccgggagctg gcggagcgcg gagtccgcat cgtctccaga      60
   ggtaggacgc agcttttctc tctgaatccg caaagcggca gcttggtca                 109
<210> 337 _
   <211> 88
   <212> DNA
   <213> Homo sapiens
<400> 337
   tcctcgccat ggacgcggac gactcccggg cccccaaggg ctccttgcgg aagttcctgg      60
   agcacctctc cggggccggc aaggccat                                         88
<210> 338
   <211> 108
   <212> DNA
   <213> Homo sapiens
<400> 338
   cgctcgcaca gagcccggca atgccgaggc cctcccaacg ggtcggtctg cgaggagcaa      60
   aaaaggggtt cagaggaggg cagcgcgtgc gtcgcgctca gctatagg                  108
<210> 339
   <211> 120
   <212> DNA
   <213> Homo sapiens
<400> 339
   cccagcaggc ctggccgagg cgggaccttc gtcgctccag cccccgtccc cgcccccgcg      60
   cctccccgcc gcgcggagct ctggttggct tgctttccaa ccggactttg gggctagcgt     120
<210> 340
   <211> 120
   <212> DNA
   <213> Homo sapiens
<400> 340
   cccagcaggc ctggccgagg cgggaccttc gtcgctccag cccccgtccc cgcccccgcg      60
   cctccccgcc gcgcggagct ctggttggct tgctttccaa ccggactttg gggctagcgt     120
<210> 341
   <211> 109
   <212> DNA
   <213> Homo sapiens
<400> 341
   cacaggtaag ggggcggccc cgccccgcgc cctggaacga cctcacggcc ccgcccacat 60
   ccccgcccct ggccccacct ccgccgcaga gcggaaccct cagagtcgc 109
<210> 342
   <211> 75
   <212> DNA
   <213> Homo sapiens
<400> 342
   gcgctgaagt cggggcccgc cctgtggccc cgcccggccc gcgcttgcta gcgcccaaag 60
   ccagcgaagc acggg 75
<210> 343
   <211> 113
   <212> DNA
   <213> Homo sapiens
<400> 343
   tcagaggggc gcagggtgcg cgggggccgt tggccctccg ggcacttccc ctttgcggtc 60
   tccccgccct ccttcggagc tggtgcctga ggtcgctggg acctcagcct ggc 113
<210> 344
   <211> 84
   <212> DNA
   <213> Homo sapiens
<400> 344
   cgctgcagga ccctgggggc cggacggtgg gatacggcca atctccgggg agatgctgtg 60
   gctcttaccg agcgggagaa gggt 84
<210> 345
   <211> 27
   <212> DNA
   <213> Homo sapiens
<400> 345
   ttatatgtcg gttacgtgcg tttatat 27
<210> 346
   <211> 23
   <212> DNA
   <213> Homo sapiens
<400> 346
   agcgtagaga taggttggta acg 23
<210> 347
   <211> 21
   <212> DNA
   <213> Homo sapiens
<400> 347
   tacgcgtagg ttttaagtcg c 21
<210> 348
   <211> 25
   <212> DNA
   <213> Homo sapiens
<400> 348
   aaagttagta ggagtaagag gacgc 25
<210> 349
   <211> 20
   <212> DNA
   <213> Homo sapiens
<400> 349
   ggggttatag tttggcgttc 20
<210> 350
   <211> 20
   <212> DNA
   <213> Homo sapiens
<400> 350
   ttcggggtgt agcggtcgtc 20
<210> 351
   <211> 19
   <212> DNA
   <213> Homo sapiens
<400> 351
   gtttcggggt tcgtttttc 19
<210> 352
   <211> 19
   <212> DNA
   <213> Homo sapiens
<400> 352
   agttttcgga gaagacggc 19
<210> 353
   <211> 32
   <212> DNA
   <213> Homo sapiens
<400> 353
   cgttaggtta ttttttaaat agagtcggta gc 32
<210> 354
   <211> 20
   <212> DNA
   <213> Homo sapiens
<400> 354
   gggattataa gtcgcgtcgc 20
<210> 355
   <211> 22
   <212> DNA
   <213> Homo sapiens
<400> 355
   cgtgaataaa tagttgaggg gc 22
<210> 356
   <211> 27
   <212> DNA
   <213> Homo sapiens
<400> 356
   gcggggtttt ttttatcggt tagattc 27
<210> 357
   <211> 25
   <212> DNA
   <213> Homo sapiens
<400> 357
   gcggttttta aggagtttta ttttc 25
<210> 358
   <211> 22
   <212> DNA
   <213> Homo sapiens
<400> 358
   aacgatttgg ggttagagtt tc 22
<210> 359
   <211> 25
   <212> DNA
   <213> Homo sapiens
<400> 359
   cgttcgtata gagttcggta atgtc 25
<210> 360
   <211> 23
   <212> DNA
   <213> Homo sapiens
<400> 360
   tcgtcgtttt agttttcgtt ttc 23
<210> 361
   <211> 18
   <212> DNA
   <213> Homo sapiens
<400> 361
   gcgttgaagt cggggttc 18
<210> 362
   <211> 21
   <212> DNA
   <213> Homo sapiens
<400> 362
   aggtaggttt tagttttcgg c 21
<210> 363
   <211> 26
   <212> DNA
   <213> Homo sapiens
<400> 363
   tgtagttttc ggagttagtg tcgcgc 26
<210> 364
   <211> 19
   <212> DNA
   <213> Homo sapiens
<400> 364
   gagatgtttc gagggttgc 19
<210> 365
   <211> 22
   <212> DNA
   <213> Homo sapiens
<400> 365
   gttagtcgag ttcggaggta tc 22
<210> 366
   <211> 19
   <212> DNA
   <213> Homo sapiens
<400> 366
   gaaccaaaac gctccccat 19
<210> 367
   <211> 22
   <212> DNA
   <213> Homo sapiens
<400> 367
   aaaacgataa cccttaaacc ga 22
<210> 368
   <211> 22
   <212> DNA
   <213> Homo sapiens
<400> 368
   tcccgaacta aacgaaaccc cg 22
<210> 369
   <211> 19
   <212> DNA
   <213> Homo sapiens
<400> 369
   aatacgacgc caaaaaccg 19
<210> 370
   <211> 21
   <212> DNA
   <213> Homo sapiens
<400> 370
   gctctaaaaa ccacacccgt c 21
<210> 371
   <211> 22
   <212> DNA
   <213> Homo sapiens
<400> 371
   gccccaatac taaatcacga cg 22
<210> 372
   <211> 20
   <212> DNA
   <213> Homo sapiens
<400> 372
   cgactcgctc tatctcgcac 20
<210> 373
   <211> 26
   <212> DNA
   <213> Homo sapiens
<400> 373
   acgactaaac tactataacc ctacga 26
<210> 374
   <211> 27
   <212> DNA
   <213> Homo sapiens
<400> 374
   cgactatacg aactaacgaa taaaccg 27
<210> 375
   <211> 20
   <212> DNA
   <213> Homo sapiens
<400> 375
   cgaacgcaaa accgaaatcg 20
<210> 376
   <211> 20
   <212> DNA
   <213> Homo sapiens
<400> 376
   gacaatcccg aaaaacgaac 20
<210> 377
   <211> 27
   <212> DNA
   <213> Homo sapiens
<400> 377
   caacgatacc caaaaaaaat caacgcg 27
<210> 378
   <211> 21
   <212> DNA
   <213> Homo sapiens
<400> 378
   ctacgaaatt ccctttacgc t 21
<210> 379
   <211> 22
   <212> DNA
   <213> Homo sapiens
<400> 379
   taaccaaact accgctttac ga 22
<210> 380
   <211> 22
   <212> DNA
   <213> Homo sapiens
<400> 380
   cctataacta aacgcgacgc ac 22
<210> 381
   <211> 19
   <212> DNA
   <213> Homo sapiens
<400> 381
   acgctaaccc caaaatccg 19
<210> 382
   <211> 24
   <212> DNA
   <213> Homo sapiens
<400> 382
   cccgtacttc gctaacttta aacg 24
<210> 383
   <211> 20
   <212> DNA
   <213> Homo sapiens
<400> 383
   gacctcaaac accaactccg 20
<210> 384
   <211> 25
   <212> DNA
   <213> Homo sapiens
<400> 384
   cctacgatcg aaaacgacgc gaacg 25
<210> 385
   <211> 20
   <212> DNA
   <213> Homo sapiens
<400> 385
   ccgcaatatc actaaaccga 20
<210> 386
   <211> 24
   <212> DNA
   <213> Homo sapiens
<400> 386
   caactccgaa cgaaacaata aacg 24
<210> 387
   <211> 38
   <212> DNA
   <213> Homo sapiens
<400> 387
   cgtctgcccc gtcgaaaacc cgccgattaa cgcagacg 38
<210> 388
   <211> 36
   <212> DNA
   <213> Homo sapiens
<400> 388
   cgacatgcgc ggaggggtta gcgtggttgc atgtcg 36
<210> 389
   <211> 37
   <212> DNA
   <213> Homo sapiens
<400> 389
   cgacatgcct acgaccgcgt cgcccattag catgtcg 37
<210> 390
   <211> 32
   <212> DNA
   <213> Homo sapiens
<400> 390
   cgacatgcgg gcggtcgggg tcgcgcatgt cg 32
<210> 391
   <211> 42
   <212> DNA
   <213> Homo sapiens
<400> 391
   cgacatgcgg ttcgggttat ttgcgaagga gtcggcatgt cg 42
<210> 392
   <211> 41
   <212> DNA
   <213> Homo sapiens
<400> 392
   cgtctgcttg gggtcggcgg gagttcgcgg gattgcagac g 41
<210> 393
   <211> 35
   <212> DNA
   <213> Homo sapiens
<400> 393
   cgacatgcac gcgcgcaccc caacccagca tgtcg 35
<210> 394
   <211> 38
   <212> DNA
   <213> Homo sapiens
<400> 394
   cgacatgcac cacgaccacg cgaatcgaac gcatgtcg 38
<210> 395
   <211> 37
   <212> DNA
   <213> Homo sapiens
<400> 395
   cgacatgcac gccgaccgcg atctaactcg catgtcg 37
<210> 396
   <211> 35
   <212> DNA
   <213> Homo sapiens
<400> 396
   cgacacgata tggcgttgag gcggttatcg tgtcg 35
<210> 397
   <211> 38
   <212> DNA
   <213> Homo sapiens
<400> 397
   cgtctgccac aaaccgttct aacccgaccg cgcagacg 38
<210> 398
   <211> 32
   <212> DNA
   <213> Homo sapiens
<400> 398
   cgacatgccg acgccccctc cccggcatgt cg 32
<210> 399
   <211> 39
   <212> DNA
   <213> Homo sapiens
<400> 399
   cgacatgggt tcgtaaggtt tggggtagcg gccatgtcg 39
<210> 400
   <211> 32
   <212> DNA
   <213> Homo sapiens
<400> 400
   cgacatgcgc gctccgccaa ctccgcatgt cg 32
<210> 401
   <211> 38
   <212> DNA
   <213> Homo sapiens
<400> 401
   cgacatgcgg ggtttagagg agggtagcgc gcatgtcg 38
<210> 402
   <211> 27
   <212> DNA
   <213> Homo sapiens
<400> 402
   actccgcgtt ttttcgtcgc gcggagt 27
<210> 403
   <211> 42
   <212> DNA
   <213> Homo sapiens
<400> 403
   cgtctgcgtg gtttcgttcg gttcgcgttt gttaggcaga cg 42
<210> 404
   <211> 36
   <212> DNA
   <213> Homo sapiens
<400> 404
   cgacatgcgg gtgcgcgggg gtcgttgggc atgtcg 36
<210> 405
   <211> 47
   <212> DNA
   <213> Homo sapiens
<400> 405
   cgacatgctc gggagtcggg gcgtatttag ttcgtagcgg catgtcg 47
<210> 406
   <211> 43
   <212> DNA
   <213> Homo sapiens
<400> 406
   cgacatgcgt tcgtgttttg gtttgtcgcg gtttggcatg tcg 43
<210> 407
   <211> 36
   <212> DNA
   <213> Homo sapiens
<400> 407
   cgacatgccc gacgactctc gacctcccgc atgtcg 36
<210> 408
   <211> 74
   <212> DNA
   <213> Homo sapiens
<400> 408
   ttatatgtcg gttacgtgcg tttatattta gttaatcggc gggttttcga cgggaatggg 60
   gagcgttttg gttc 74
<210> 409
   <211> 111
   <212> DNA
   <213> Homo sapiens
<400> 409
   agcgtagaga taggttggta acggttttag ggaggcgcgg aggggttagc gtggttggtt 60
   taaaaggata tagggattga ggggtaagat cggtttaagg gttatcgttt t 111
<210> 410
   <211> 113
   <212> DNA
   <213> Homo sapiens
<400> 410
   tacgcgtagg ttttaagtcg cggttaatgg gcgacgcggt cgtagattcg ttcggtttcg 60
   ttttgttttg tgagtttttt cggtcgggtt gcggggtttc gtttagttcg gga 113
<210> 411
   <211> 117
   <212> DNA
   <213> Homo sapiens
<400> 411
   aaagttagta ggagtaagag gacgcgtagg agggtttcgg tcgcggttat ttttgggcgg 60
   tcggggtcgc ggtttcggga gcggtgcggg cgcgggttcg gtttttggcg tcgtatt 117
<210> 412
   <211> 97
   <212> DNA
   <213> Homo sapiens
<400> 412
   ggggttatag tttggcgttc ggattttggt tcgggttatt tgcgaaggag tcggttttgg      60
   ttaaggtgtt tttttggacg ggtgtggttt ttagagc                               97
<210> 413
   <211> 91
   <212> DNA
   <213> Homo sapiens
<400> 413
   ttcggggtgt agcggtcgtc ggggttgggg tcggcgggag ttcgcgggat tttttagaag      60
   agcggtcggc gtcgtgattt agtattgggg c                                     91
<210> 414
   <211> 96
   <212> DNA
   <213> Homo sapiens
<400> 414
   gtttcggggt tcgtttttcg gtaggttcgg ggagaggtgg ggtgataatg ggttggggtg      60
   cgcgcgtgtt ttataggtgc gagatagagc gagtcg                                96
<210> 415
   <211> 101
   <212> DNA
   <213> Homo sapiens
<400> 415
   agttttcgga gaagacggcg tttttaacgt tcgattcgcg tggtcgtggt agcgttacgc      60
   gagtttttta ggcgatcgta gggttatagt agtttagtcg t                         101
<210> 416
   <211> 117
   <212> DNA
   <213> Homo sapiens
<400> 416
   cgttaggtta ttttttaaat agagtcggta gcgcgtttcg ttcggtattt ttcgaagagt      60
   tagatcgcgg tcggcgttag cgttatcgtt cggtttattc gttagttcgt atagtcg       117
<210> 417
   <211> 91
   <212> DNA
   <213> Homo sapiens
<400> 417
   gggattataa gtcgcgtcgc gttgtcgttg gttttttagt aattttcgat atggcgttga      60
   ggcggttatc gcgatttcgg ttttgcgttc g                                     91
<210> 418
   <211> 124
   <212> DNA
   <213> Homo sapiens
<400> 418
   cgtgaataaa tagttgaggg gcggtcgggt tagaacggtt tgtgtaattt tgtaaacgtg      60
   ttagaaagtt taaaattttt tttttttttt ttattttaga tattgttcgt ttttcgggat     120
   tgtc                                                                  124
<210> 419
   <211> 109
   <212> DNA
   <213> Homo sapiens
<400> 419
   gcggggtttt ttttatcggt tagattcggg gagaggcgcg cggaggttgc gaaggtttta      60
   gaagggcggg gagggggcgt cgcgcgttga ttttttttgg gtatcgttg                 109
<210> 420
   <211> 99
   <212> DNA
   <213> Homo sapiens
<400> 420
   gcggttttta aggagtttta ttttcgggat taaatggttc gtaaggtttg gggtagcggc      60
   gttgtaggag atgagtttag cgtaaaggga atttcgtag                             99
<210> 421
   <211> 109
   <212> DNA
   <213> Homo sapiens
<400> 421
   aacgatttgg ggttagagtt tcgggagttg gcggagcgcg gagttcgtat cgtttttaga      60
   ggtaggacgt agtttttttt tttgaattcg taaagcggta gtttggtta                 109
<210> 422
   <211> 108
   <212> DNA
   <213> Homo sapiens
<400> 422
   cgttcgtata gagttcggta atgtcgaggt ttttttaacg ggtcggtttg cgaggagtaa      60
   aaaaggggtt tagaggaggg tagcgcgtgc gtcgcgttta gttatagg                  108
<210> 423
   <211> 92
   <212> DNA
   <213> Homo sapiens
<400> 423
   tcgtcgtttt agttttcgtt ttcgttttcg cgttttttcg tcgcgcggag ttttggttgg 60
   tttgtttttt aatcggattt tggggttagc gt 92
<210> 424
   <211> 75
   <212> DNA
   <213> Homo sapiens
<400> 424
   gcgttgaagt cggggttcgt tttgtggttt cgttcggttc gcgtttgtta gcgtttaaag 60
   ttagcgaagt acggg 75
<210> 425
   <211> 133
   <212> DNA
   <213> Homo sapiens
<400> 425
   aggtaggttt tagttttcgg cgcggggagg cggcgcgttt tagaggggcg tagggtgcgc 60
   gggggtcgtt ggtttttcgg gtattttttt tttgcggttt tttcgttttt tttcggagtt 120
   ggtgtttgag gtc 133
<210> 426
   <211> 126
   <212> DNA
   <213> Homo sapiens
<400> 426
   tgtagttttc ggagttagtg tcgcgcgttc gtcgtttcgc gtttttttgt tcgtcgtatt 60
   ttcgggagtc ggggcgtatt tagttcgtag cgtcgttttt tcgttcgcgt cgttttcgat 120
   cgtagg 126
<210> 427
   <211> 117
   <212> DNA
   <213> Homo sapiens
<400> 427
   gagatgtttc gagggttgcg cgggtttttc ggttcgaagt cgtcgttcgt gttttggttt 60
   gtcgcggttt ggtttatagc gtatttaggg tttttagtcg gtttagtgat attgcgg 117
<210> 428
   <211> 79
   <212> DNA
   <213> Homo sapiens
<400> 428
   gttagtcgag ttcggaggta tcgggaggtc gagagtcgtc gggattttag ttttgcgttt 60
   attgtttcgt tcggagttg 79
<210> 429
   <211> 74
   <212> DNA
   <213> Homo sapiens
<400> 429
   ccacatgtcg gtcacgtgcg cccacaccca gccaatcggc gggctcccga cgggaatggg      60
   gagcgccctg gtcc                                                        74
<210> 430
   <211> 111
   <212> DNA
   <213> Homo sapiens
<400> 430
   agcgcagaga caggctggca acggcttcag ggaggcgcgg aggggtcagc gtggctggct      60
   taaaaggata cagggactga ggggcaagac cggctcaagg gtcaccgctt c              111
<210> 431
   <211> 113
   <212> DNA
   <213> Homo sapiens
<400> 431
   cacgcgcagg ccccaagtcg cggccaatgg gcgacgcggc cgcagatccg cccggccccg      60
   ccctgccctg tgagttcctc cggccgggct gcggggctcc gctcagtccg gga            113
<210> 432
   <211> 117
   <212> DNA
   <213> Homo sapiens
<400> 432
   aaagccagca ggagcaagag gacgcgcagg agggcttcgg tcgcggtcat ctctgggcgg      60
   ccggggtcgc ggtcccggga gcggtgcggg cgcgggtccg gctcctggcg ccgcact        117
<210> 433
   <211> 97
   <212> DNA
   <213> Homo sapiens
<400> 433
   ggggtcacag cctggcgctc ggaccctggc ccgggtcatc tgcgaaggag ccggctttgg      60
   ccaaggtgcc ttcctggacg ggtgtggttc ccagagc                               97
<210> 434
   <211> 91
   <212> DNA
   <213> Homo sapiens
<400> 434
   cccggggtgc agcggccgcc ggggctgggg ccggcgggag tccgcgggac cctccagaag 60
   agcggccggc gccgtgactc agcactgggg c 91
<210> 435
   <211> 96
   <212> DNA
   <213> Homo sapiens
<400> 435
   gctccggggc tcgctctccg gcaggcccgg ggagaggtgg ggtgacaatg ggttggggtg 60
   cgcgcgtgcc tcataggtgc gagacagagc gagccg 96
<210> 436
   <211> 101
   <212> DNA
   <213> Homo sapiens
<400> 436
   agcccccgga gaagacggcg cccccaacgc ccgacccgcg tggccgtggc agcgccacgc 60
   gagccctcta ggcgaccgca gggccacagc agctcagccg c 101
<210> 437
   <211> 117
   <212> DNA
   <213> Homo sapiens
<400> 437
   cgtcaggcca ctccttaaat agagccggca gcgcgctccg ctcggcattt cccgaagagc 60
   cagatcgcgg ccggcgccag cgccaccgtc cggtccaccc gccagcccgc acagccg 117
<210> 438
   <211> 91
   <212> DNA
   <213> Homo sapiens
<400> 438
   gggactacaa gccgcgccgc gctgccgctg gcccctcagc aaccctcgac atggcgctga 60
   ggcggccacc gcgactccgg ctctgcgctc g 91
<210> 439
   <211> 124
   <212> DNA
   <213> Homo sapiens
<400> 439
   cgtgaacaaa tagctgaggg gcggccgggc cagaacggct tgtgtaactt tgcaaacgtg 60
   ccagaaagtt taaaatctct cctccttcct tcactccaga cactgcccgc tctccgggac 120
   tgcc 124
<210> 440
   <211> 109
   <212> DNA
   <213> Homo sapiens
<400> 440
   gcggggctcc ccctaccggc cagacccggg gagaggcgcg cggaggctgc gaaggttcca      60
   gaagggcggg gagggggcgc cgcgcgctga ccctccctgg gcaccgctg                 109
<210> 441
   <211> 99
   <212> DNA
   <213> Homo sapiens
<400> 441
   gcggccccca aggagcccca cccccgggac caaatggccc gcaaggtttg gggcagcggc      60
   gttgcaggag atgagctcag cgcaaaggga accccgcag                             99
<210> 442
   <211> 109
   <212> DNA
   <213> Homo sapiens
<400> 442
   aacgacctgg ggctagagcc ccgggagctg gcggagcgcg gagtccgcat cgtctccaga      60
   ggtaggacgc agcttttctc tctgaatccg caaagcggca gcttggtca                 109
<210> 443
   <211> 108
   <212> DNA
   <213> Homo sapiens
<400> 443
   cgctcgcaca gagcccggca atgccgaggc cctcccaacg ggtcggtctg cgaggagcaa      60
   aaaaggggtt cagaggaggg cagcgcgtgc gtcgcgctca gctatagg                  108
<210> 444
   <211> 92
   <212> DNA
   <213> Homo sapiens
<400> 444
   tcgtcgctcc agcccccgtc cccgcccccg cgcctccccg ccgcgcggag ctctggttgg      60
   cttgctttcc aaccggactt tggggctagc gt                                    92
<210> 445
   <211> 75
   <212> DNA
   <213> Homo sapiens
<400> 445
   gcgctgaagt cggggcccgc cctgtggccc cgcccggccc gcgcttgcta gcgcccaaag      60
   ccagcgaagc acggg                                                       75
<210> 446
   <211> 119
   <212> DNA
   <213> Homo sapiens
<400> 446
   aggcaggtcc cagtccccgg cgcggggagg cggcgcgctt cagaggggcg cagggtgcgc      60
   gggggccgtt ggccctccgg gcacttcccc tttgcggtct ccccgccctc cttcggagc      119
<210> 447
   <211> 126
   <212> DNA
   <213> Homo sapiens
<400> 447
   tgcagcctcc ggagtcagtg ccgcgcgccc gccgccccgc gccttcctgc tcgccgcacc      60
   tccgggagcc ggggcgcacc cagcccgcag cgccgcctcc ccgcccgcgc cgcctccgac     120
   cgcagg                                                                126
<210> 448
   <211> 117
   <212> DNA
   <213> Homo sapiens
<400> 448
   gagatgcccc gagggctgcg cgggtctccc ggcccgaagc cgccgcccgt gttctggcct      60
   gtcgcggtct ggtctacagc gtacccaggg cccccagccg gcctagtgac actgcgg        117
<210> 449
   <211> 79
   <212> DNA
   <213> Homo sapiens
<400> 449
   gccagccgag tccggaggca tcgggaggtc gagagccgcc gggaccccag ctctgcgttc      60
   actgccccgt ccggagctg                                                   79